# EUROPEAN PATENT APPLICATION

(11) **EP 4 534 105 A1**
(43) Date of publication of application: **09.04.2025**
(21) Application number: 23815257.3
(22) Date of filing: 31.05.2023
(51) Int. Cl.: A61K 47/68, A61K 45/06, A61K 31/4745, C07K 5/117, A61P 35/00

(54) **ANTI-CLDN18.2 ANTIBODY, AND ANTIBODY-DRUG CONJUGATE AND USE THEREOF**

(30) Priority: 01.06.2022 CN 202210618735
(71) Applicant: Bio-Thera Solutions, Ltd., Guangzhou, Guangdong 510530 (CN)
(72) Inventor: XIAO, Cuizhen, Guangzhou, Guangdong 510530 (CN); QIN, Chao, Guangzhou, Guangdong 510530 (CN); MAI, Siqi, Guangzhou, Guangdong 510530 (CN); YU, Jin-Chen, Guangzhou, Guangdong 510530 (CN); LI, Shengfeng, Guangzhou, Guangdong 510530 (CN)
(74) Representative: Engelhard, Markus
(86) International application number: PCT/CN2023/097470
(87) International publication number: WO 2023/232080

(57) **Abstract**

The present invention belongs to the field of biomedicine. Disclosed in the present invention are an anti-CLDN18.2 antibody, and an antibody-drug conjugate and use thereof. In some embodiments, the antibody-drug conjugate is a compound represented by Formula I or a stereoisomer or a pharmaceutically acceptable salt or solvate thereof. The anti-CLDN18.2 antibody and the antibody-drug conjugate can be used for treating diseases associated with CLDN18.2 expression.

## Description

### TECHNICAL FIELD

The present invention relates to an anti-CLDN 18.2 antibody, an antibody-drug conjugate, and use thereof.

### BACKGROUND

The targeted therapies for cancers, immunodeficiency, infectious diseases, etc., are currently main concerns in precision medicine. Over the years, there have been numerous reports of using cell surface receptor-binding molecules as drug delivery vehicles by forming conjugates with cytotoxin molecules for targeted delivery of cytotoxin molecules to attack various pathogenic cells (Allen, T.M. and Cullis, P.R., 2004 Science, 303(5665), 1818-22; Hu, Q.Y., et al. (2016), Chem Soc Rev 45(6): 1691-1719).

The antibody-drug conjugate consists of three moieties: an antibody, a cytotoxin molecule, and a linker in between for linking the two (Thomas, A., et al. (2016), Lancet Oncol 17(6):e254-e262). The three components each possess unique functionality. The antibody specifically binds to tumor cells; the cytotoxin molecule has sufficient activity and a broad killing spectrum of tumor cells; the unique functionality of the linker keeps the conjugate stable in the blood circulation and allows the release of the cytotoxin molecule upon reaching the tumor cells (Chari, R.V. (2008), Acc Chem Res 41(1):98-107). Desirable clinical outcomes can be achieved only when the three components are properly combined (Singh, S.K., et al. (2015), Pharm Res 32(11):3541-3571; Hamilton, G.S. (2015), Biologicals 43(5):318-332).

Tight junction molecule claudin-18 isoform 2 (Claudin-18.2 or CLDN18.2) is one of the targets currently in development for treating tumors (Sahin U., et al., Claudin-18 splice variant 2 is a pan-cancer target suitable for therapeutic antibody development, Clin Cancer Res., 2008, 14(23):7624-34).

### SUMMARY

The present invention provides an antibody or antigen-binding unit specifically binding to CLDN18.2. In one or more embodiments, the antibody or antigen-binding unit of the present invention is capable of recognizing and binding to human CLDN18.2.

In one or more embodiments, the present invention provides an anti-CLDN18.2 antibody or antigen-binding unit comprising one or more amino acid sequences of (a)-(f):
(a) a VH CDR1 comprising the amino acid sequence set forth in any one of SEQ ID NOs: 1-6;
(b) a VH CDR2 comprising the amino acid sequence set forth in any one of SEQ ID NOs: 7-13;
(c) a VH CDR3 comprising the amino acid sequence set forth in any one of SEQ ID NOs: 14-21;
(d) a VL CDR1 comprising the amino acid sequence set forth in any one of SEQ ID NOs: 22-29;
(e) a VL CDR2 comprising the amino acid sequence set forth in any one of SEQ ID NOs: 30-37; and
(f) a VL CDR3 comprising the amino acid sequence set forth in any one of SEQ ID NOs: 38-45.

In one or more embodiments, the antibody or antigen-binding unit comprises a VH CDR1 set forth in any one of SEQ ID NOs: 1-6, a VH CDR2 set forth in any one of SEQ ID NOs: 7-13, and a VH CDR3 set forth in any one of SEQ ID NOs: 14-21.

In one or more embodiments, the antibody or antigen-binding unit comprises a VL CDR1 set forth in any one of SEQ ID NOs: 22-29, a VL CDR2 set forth in any one of SEQ ID NOs: 30-37, and a VL CDR3 set forth in any one of SEQ ID NOs: 38-45.

In one or more embodiments, the antibody or antigen-binding unit comprises a VH CDR1 set forth in any one of SEQ ID NOs: 1-6, a VH CDR2 set forth in any one of SEQ ID NOs: 7-13, a VH CDR3 set forth in any one of SEQ ID NOs: 14-21, a VL CDR1 set forth in any one of SEQ ID NOs: 22-29, a VL CDR2 set forth in any one of SEQ ID NOs: 30-37, and a VL CDR3 set forth in any one of SEQ ID NOs: 38-45.

In one or more embodiments, the antibody or antigen-binding unit comprises a VH CDR1 set forth in SEQ ID NO: 2, a VH CDR2 set forth in SEQ ID NO: 8, a VH CDR3 set forth in SEQ ID NO: 15, a VL CDR1 set forth in SEQ ID NO: 26, a VL CDR2 set forth in SEQ ID NO: 34, and a VL CDR3 set forth in SEQ ID NO: 42.

In one or more embodiments, the antibody or antigen-binding unit comprises a VH CDR1 set forth in SEQ ID NO: 3, a VH CDR2 set forth in SEQ ID NO: 9, a VH CDR3 set forth in SEQ ID NO: 16, a VL CDR1 set forth in SEQ ID NO: 25, a VL CDR2 set forth in SEQ ID NO: 33, and a VL CDR3 set forth in SEQ ID NO: 41.

In one or more embodiments, the antibody or antigen-binding unit comprises a VH CDR1 set forth in SEQ ID NO: 6, a VH CDR2 set forth in SEQ ID NO: 13, a VH CDR3 set forth in SEQ ID NO: 20, a VL CDR1 set forth in SEQ ID NO: 28, a VL CDR2 set forth in SEQ ID NO: 36, and a VL CDR3 set forth in SEQ ID NO: 44.

In one or more embodiments, the antibody or antigen-binding unit comprises a VH CDR1 set forth in SEQ ID NO: 5, a VH CDR2 set forth in SEQ ID NO: 12, a VH CDR3 set forth in SEQ ID NO: 19, a VL CDR1 set forth in SEQ ID NO: 29, a VL CDR2 set forth in SEQ ID NO: 37, and a VL CDR3 set forth in SEQ ID NO: 45.

In one or more embodiments, the antibody or antigen-binding unit comprises a VH CDR1 set forth in SEQ ID NO: 4, a VH CDR2 set forth in SEQ ID NO: 11, a VH CDR3 set forth in SEQ ID NO: 18, a VL CDR1 set forth in SEQ ID NO: 27, a VL CDR2 set forth in SEQ ID NO: 35, and a VL CDR3 set forth in SEQ ID NO: 43.

In one or more embodiments, the antibody or antigen-binding unit comprises a VH CDR1 set forth in SEQ ID NO: 3, a VH CDR2 set forth in SEQ ID NO: 9, a VH CDR3 set forth in SEQ ID NO: 16, a VL CDR1 set forth in SEQ ID NO: 26, a VL CDR2 set forth in SEQ ID NO: 34, and a VL CDR3 set forth in SEQ ID NO: 42.

In one or more embodiments, the antibody or antigen-binding unit comprises a VH CDR1 set forth in SEQ ID NO: 3, a VH CDR2 set forth in SEQ ID NO: 9, a VH CDR3 set forth in SEQ ID NO: 16, a VL CDR1 set forth in SEQ ID NO: 24, a VL CDR2 set forth in SEQ ID NO: 32, and a VL CDR3 set forth in SEQ ID NO: 40.

In one or more embodiments, the antibody or antigen-binding unit comprises a VH CDR1 set forth in SEQ ID NO: 1, a VH CDR2 set forth in SEQ ID NO: 7, a VH CDR3 set forth in SEQ ID NO: 14, a VL CDR1 set forth in SEQ ID NO: 22, a VL CDR2 set forth in SEQ ID NO: 30, and a VL CDR3 set forth in SEQ ID NO: 38.

In one or more embodiments, the antibody or antigen-binding unit comprises a VH CDR1 set forth in SEQ ID NO: 1, a VH CDR2 set forth in SEQ ID NO: 7, a VH CDR3 set forth in SEQ ID NO: 14, a VL CDR1 set forth in SEQ ID NO: 23, a VL CDR2 set forth in SEQ ID NO: 31, and a VL CDR3 set forth in SEQ ID NO: 39.

In one or more embodiments, the antibody or antigen-binding unit comprises a VH CDR1 set forth in SEQ ID NO: 2, a VH CDR2 set forth in SEQ ID NO: 10, a VH CDR3 set forth in SEQ ID NO: 17, a VL CDR1 set forth in SEQ ID NO: 27, a VL CDR2 set forth in SEQ ID NO: 35, and a VL CDR3 set forth in SEQ ID NO: 43.

In one or more embodiments, the antibody or antigen-binding unit comprises a VH CDR1 set forth in SEQ ID NO: 2, a VH CDR2 set forth in SEQ ID NO: 8, a VH CDR3 set forth in SEQ ID NO: 21, a VL CDR1 set forth in SEQ ID NO: 26, a VL CDR2 set forth in SEQ ID NO: 34, and a VL CDR3 set forth in SEQ ID NO: 42.

In one or more embodiments, the antibody or antigen-binding unit is a murine antibody, a chimeric antibody, or a humanized antibody.

In one or more embodiments, the antibody or antigen-binding unit comprises a heavy chain variable region and/or a light chain variable region. In one or more embodiments, the heavy chain variable region comprises the amino acid sequence set forth in any one of SEQ ID NOs: 46-55, or an amino acid sequence having at least 80% or 90% identity to the amino acid sequence set forth in any one of SEQ ID NOs: 46-55, or an amino acid sequence having one or more conservative amino acid substitutions compared with the amino acid sequence set forth in any one of SEQ ID NOs: 46-55. In one or more embodiments, the light chain variable region comprises the amino acid sequence set forth in any one of SEQ ID NOs: 56-64, or an amino acid sequence having at least 80% or 90% identity to the amino acid sequence set forth in any one of SEQ ID NOs: 56-64, or an amino acid sequence having one or more conservative amino acid substitutions compared with the amino acid sequence set forth in any one of SEQ ID NOs: 56-64. In one or more embodiments, the heavy chain variable region comprises the amino acid sequence set forth in any one of SEQ ID NOs: 46-55, and the light chain variable region comprises the amino acid sequence set forth in any one of SEQ ID NOs: 56-64.

In one or more embodiments, the antibody or antigen-binding unit comprises a heavy chain variable region and a light chain variable region.

In one or more embodiments, the heavy chain variable region comprises the amino acid sequence set forth in SEQ ID NO: 47, and the light chain variable region comprises the amino acid sequence set forth in SEQ ID NO: 60.

In one or more embodiments, the heavy chain variable region comprises the amino acid sequence set forth in SEQ ID NO: 48, and the light chain variable region comprises the amino acid sequence set forth in SEQ ID NO: 59.

In one or more embodiments, the heavy chain variable region comprises the amino acid sequence set forth in SEQ ID NO: 52, and the light chain variable region comprises the amino acid sequence set forth in SEQ ID NO: 62.

In one or more embodiments, the heavy chain variable region comprises the amino acid sequence set forth in SEQ ID NO: 51, and the light chain variable region comprises the amino acid sequence set forth in SEQ ID NO: 63.

In one or more embodiments, the heavy chain variable region comprises the amino acid sequence set forth in SEQ ID NO: 50, and the light chain variable region comprises the amino acid sequence set forth in SEQ ID NO: 61.

In one or more embodiments, the heavy chain variable region comprises the amino acid sequence set forth in SEQ ID NO: 48, and the light chain variable region comprises the amino acid sequence set forth in SEQ ID NO: 60.

In one or more embodiments, the heavy chain variable region comprises the amino acid sequence set forth in SEQ ID NO: 48, and the light chain variable region comprises the amino acid sequence set forth in SEQ ID NO: 58.

In one or more embodiments, the heavy chain variable region comprises the amino acid sequence set forth in SEQ ID NO: 46, and the light chain variable region comprises the amino acid sequence set forth in SEQ ID NO: 56.

In one or more embodiments, the heavy chain variable region comprises the amino acid sequence set forth in SEQ ID NO: 46, and the light chain variable region comprises the amino acid sequence set forth in SEQ ID NO: 57.

In one or more embodiments, the heavy chain variable region comprises the amino acid sequence set forth in SEQ ID NO: 49, and the light chain variable region comprises the amino acid sequence set forth in SEQ ID NO: 61.

In one or more embodiments, the heavy chain variable region comprises the amino acid sequence set forth in SEQ ID NO: 53, and the light chain variable region comprises the amino acid sequence set forth in SEQ ID NO: 64.

In one or more embodiments, the heavy chain variable region comprises the amino acid sequence set forth in SEQ ID NO: 54, and the light chain variable region comprises the amino acid sequence set forth in SEQ ID NO: 64.

In one or more embodiments, the heavy chain variable region comprises the amino acid sequence set forth in SEQ ID NO: 55, and the light chain variable region comprises the amino acid sequence set forth in SEQ ID NO: 64.

In one or more embodiments, the antibody or antigen-binding unit further comprises a heavy chain constant region, a light chain constant region, an Fc region, or a combination thereof. In some embodiments, the light chain is of kappa (κ) or lambda (λ) type. In one or more embodiments, the light chain constant region is a κ or λ chain constant region. In one or more embodiments, the antibody or antigen-binding unit is of one of the isotypes IgG, IgM, IgA, IgE, and IgD. In one or more embodiments, the isotype is IgG1, IgG2, IgG3, or IgG4. In one or more embodiments, the antibody is an IgG1 antibody.

In one or more embodiments, the Fc is a variant Fc region. In one or more embodiments, the variant Fc region has one or more amino acid modifications, such as substitutions, deletions, or insertions, relative to a parent Fc region. In one or more embodiments, the amino acid modification of the Fc region alters the effector function activity relative to the activity of the parent Fc region. In one or more embodiments, the variant Fc region may have altered (i.e., increased or decreased) antibody-dependent cellular cytotoxicity (ADCC), complement-dependent cytotoxicity (CDC), phagocytosis, opsonization, or cell binding capacity. In one or more embodiments, the amino acid modification of the Fc region may alter the affinity of the variant Fc region for an FcγR (Fcy receptor) relative to the parent Fc region. In one or more embodiments, the Fc region is derived from IgG1 or IgG4.

In one or more embodiments, the antibody or antigen-binding unit is an isolated antibody or antigen-binding unit. In one or more embodiments, the antibody or antigen-binding unit is an scFV, a Fab, a F(ab)₂, or IgG1. In one or more embodiments, the antibody or antigen-binding unit is a monoclonal antibody.

In one or more embodiments, the heavy chain constant region comprises one or more of the following amino acid mutations: N297A, L234A, L235A, and P329G (EU numbering). In one or more embodiments, the heavy chain constant region comprises the following amino acid mutation: N297A (EU numbering). In one or more embodiments, the heavy chain constant region comprises the following amino acid mutations: L234A, L235A, and P329G (EU numbering).

In one or more embodiments, the heavy chain constant region comprises the amino acid sequence set forth in any one of SEQ ID NOs: 65-67, or an amino acid sequence having at least 80% or 90% identity to the amino acid sequence set forth in any one of SEQ ID NOs: 65-67, or an amino acid sequence having one or more conservative amino acid substitutions compared with the amino acid sequence set forth in any one of SEQ ID NOs: 65-67.

In one or more embodiments, the light chain constant region comprises the amino acid sequence set forth in SEQ ID NO: 68, or an amino acid sequence having at least 80% or at least 90% identity to the amino acid sequence set forth in SEQ ID NO: 68, or an amino acid sequence having one or more conservative amino acid substitutions compared with the amino acid sequence set forth in SEQ ID NO: 68.

In one or more embodiments, the heavy chain constant region comprises the amino acid sequence set forth in any one of SEQ ID NOs: 65-67, and the light chain constant region comprises the amino acid sequence set forth in SEQ ID NO: 68. In one or more embodiments, the heavy chain constant region comprises the amino acid sequence set forth in SEQ ID NO: 65, and the light chain constant region comprises the amino acid sequence set forth in SEQ ID NO: 68. In one or more embodiments, the heavy chain constant region comprises the amino acid sequence set forth in SEQ ID NO: 66, and the light chain constant region comprises the amino acid sequence set forth in SEQ ID NO: 68. In one or more embodiments, the heavy chain constant region comprises the amino acid sequence set forth in SEQ ID NO: 67, and the light chain constant region comprises the amino acid sequence set forth in SEQ ID NO: 68.

In one or more embodiments, the heavy chain of the antibody comprises the amino acid sequence set forth in any one of SEQ ID NOs: 69-71, or an amino acid sequence having at least 80% or 90% identity to the amino acid sequence set forth in any one of SEQ ID NOs: 69-71, or an amino acid sequence having one or more conservative amino acid substitutions compared with the amino acid sequence set forth in any one of SEQ ID NOs: 69-71. In one or more embodiments, the light chain of the antibody comprises the amino acid sequence set forth in SEQ ID NO: 72, or an amino acid sequence having at least 80% or 90% identity to the amino acid sequence set forth in SEQ ID NO: 72, or an amino acid sequence having one or more conservative amino acid substitutions compared with the amino acid sequence set forth in SEQ ID NO: 72.

In one or more embodiments, the heavy chain of the antibody comprises the amino acid sequence set forth in any one of SEQ ID NOs: 69-71, and the light chain of the antibody comprises the amino acid sequence set forth in SEQ ID NO: 72. In one or more embodiments, the heavy chain of the antibody comprises the amino acid sequence set forth in SEQ ID NO: 69, and the light chain of the antibody comprises the amino acid sequence set forth in SEQ ID NO: 72. In one or more embodiments, the heavy chain of the antibody comprises the amino acid sequence set forth in SEQ ID NO: 70, and the light chain of the antibody comprises the amino acid sequence set forth in SEQ ID NO: 72. In one or more embodiments, the heavy chain of the antibody comprises the amino acid sequence set forth in SEQ ID NO: 71, and the light chain of the antibody comprises the amino acid sequence set forth in SEQ ID NO: 72.

In one or more embodiments, the antibody described herein comprises two heavy chains with identical sequences and two light chains with identical sequences.

In one or more embodiments, the present invention further provides a polynucleotide encoding the antibody or antigen-binding unit described herein, or a portion thereof. In one or more embodiments, the polynucleotide is an isolated polynucleotide.

In one or more embodiments, the present invention further provides an expression vector comprising the polynucleotide encoding the antibody or antigen-binding unit described herein.

In one or more embodiments, the present invention further provides a cell comprising the polynucleotide encoding the antibody or antigen-binding unit described herein.

In one or more embodiments, the vector, the cell, or the microorganism is an isolated vector, cell, or microorganism. In one or more embodiments, the cell is a CHO cell, a HEK cell (e.g., a HEK293F cell), a BHK cell, a Cos1 cell, a Cos7 cell, a CV1 cell, or a murine L cell. In one or more embodiments, the cell is a CHO cell.

The present invention further provides a method for preparing the antibody, comprising: culturing a host cell comprising a nucleic acid encoding the antibody in a culture medium. In some embodiments, the method further comprises purifying the antibody. The purification may be conducted by conventional methods, for example, by conducting centrifugation on a cell suspension and collecting the supernatant, and conducting centrifugation again to further remove impurities. Protein A affinity column, ion exchange column, and other methods may be used for purifying antibody protein.

In one or more embodiments, the present invention further provides a diagnostic method and use. In one or more embodiments, the present invention provides a method for detecting CLDN18.2 expression in a sample, comprising: contacting the sample with the antibody or antigen-binding unit to allow the antibody or antigen-binding unit to bind to CLDN18.2, and detecting the binding, i.e., the amount of CLDN18.2 in the sample. In one or more embodiments, the present invention provides use of the antibody or antigen-binding unit in preparing a kit for the diagnosis or prognosis of a cancer or a tumor. In some embodiments, the present invention provides a diagnostic or prognostic kit comprising the antibody or antigen-binding unit.

In one or more embodiments, the present invention further provides an antibody-drug conjugate (ADC) or a pharmaceutically acceptable salt or solvate thereof, comprising the antibody or antigen-binding unit described herein conjugated to a drug via a linker.

In one or more embodiments, the linker is a cleavable linker.

In one or more embodiments, the drug is an anti-cancer drug, a cytotoxic drug, a cell differentiation factor, a stem cell trophic factor, a steroid drug, a drug for treating autoimmune diseases, an anti-inflammatory drug or a drug for treating infectious diseases.

In one or more embodiments, the drug is an anti-cancer drug.

In one or more embodiments, the drug is a tubulin inhibitor, a DNA damaging agent or a DNA topoisomerase inhibitor.

In one or more embodiments, the tubulin inhibitor is selected from dolastatin, auristatins and maytansinoids.

In one or more embodiments, the drug is an auristatin, such as monomethyl auristatin E (MMAE), monomethyl auristatin F (MMAF) or auristatin F (AF).

In one or more embodiments, the drug is a DNA damaging agent, e.g., a calicheamicin, a duocarmycin, or an anthramycin derivative PBD (pyrrolobenzodiazepine).

In one or more embodiments, the drug is a DNA topoisomerase inhibitor or a salt thereof, e.g., irinotecan, irinotecan hydrochloride, camptothecin, 9-aminocamptothecin, 9-nitrocamptothecin, 10-hydroxycamptothecin, 9-chloro-10-hydroxycamptothecin, a camptothecin derivative SN-38, 22-hydroxyacuminatine, topotecan, lurtotecan, belotecan, exatecan, an exatecan derivative, homosilatecan, 6,8-dibromo-2-methyl-3-[2-(D-xylopyranosylamino)phenyl]-4(3*H*)-quinazolinone, 2-cyano-3-(3,4-dihydroxyphenyl)-*N*-(phenylmethyl)-(2*E*)-2-propenamide, 2-cyano-3-(3,4-dihydroxyphenyl)-*N*-(3-hydroxyphenylpropyl)-(*E*)-2-propenamide, 12-β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5,7(6*H*)-dione, *N-*[2-(dimethylamino)ethyl]-4-acridinecarboxamide dihydrochloride or *N*-[2-(dimethylamino)ethyl]-4-acridinecarboxamide.

In one or more embodiments, the DNA topoisomerase inhibitor is camptothecin, 10-hydroxycamptothecin, topotecan, belotecan, irinotecan, 22-hydroxyacuminatine or exatecan.

In one or more embodiments, the drug has an amino group or an amino group substituted with one alkyl group, and links to the linker via an amide bond.

In one or more embodiments, the drug is 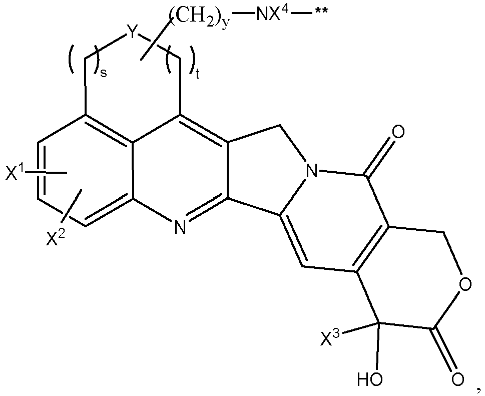 or a stereoisomer thereof, wherein X¹ and X² are each independently:
H,
hydroxy,
C1-C6 alkyl,
C1-C6 alkyl substituted with one or more hydroxy, halogen, nitro or cyano groups,
C2-C6 alkenyl,
C2-C6 alkynyl,
C1-C6 alkoxy,
C1-C6 aminoalkoxy,
halogen,
nitro,
cyano,
thiol,
alkylthio,
amino, amino substituted with an amino-protecting group, C1-C6 aminoalkyl optionally substituted at the amino moiety with an amino-protecting group or C1-C6 alkyl,
C1-C6 aminoalkylamino optionally substituted at the amino moiety with an amino-protecting group or C1-C6 alkyl,
C1-C6 alkyl linking to a heterocyclic ring, wherein the heterocyclic ring is optionally substituted with one or more C1-C6 alkyl, C1-C6 alkoxy, amino, halogen, nitro or cyano groups,
C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or a protecting group,
amino-substituted heterocyclyl optionally substituted at a nitrogen atom of the heterocyclyl moiety or at the amino moiety with a protecting group or one or more C1-C6 alkyl groups,
heterocyclylamino optionally substituted at a nitrogen atom of the heterocyclyl moiety or at the amino moiety with a protecting group or C1-C6 alkyl,
carbamoyl optionally substituted with a carbamoyl-protecting group or C1-C6 alkyl,
morpholin-1-yl, or
piperidin-1-yl;
X³ is C1-C6 alkyl;
X⁴ is H, -(CH₂)_{q}-CH₃, -(CHRⁿ)_{q}-CH₃, C3-C8 carbocyclyl, -O-(CH₂)_{q}-CH₃, arylene-CH₃, -(CH₂)_{q}-arylene-CH₃, -arylene-(CH₂)_{q}-CH₃, -(CH₂)_{q}-(C3-C8 carbocyclyl)-CH₃, -(C3-C8 carbocyclyl)-(CH₂)_{q}-CH₃, C3-C8 heterocyclyl, -(CH₂)_{q}-(C3-C8 heterocyclyl)-CH₃, -(C3-C8 heterocyclyl)-(CH₂)_{q}-CH₃, - (CH₂)_{q}C(O)NRⁿ(CH₂)_{q}-CH₃, -(CH₂CH₂O)_{q}-CH₃, -(CH₂CH₂O)_{q}-CH₂-CH₃, - (CH₂)_{q}C(O)NRⁿ(CH₂CH₂O)_{q}-CH₃, -(CH₂)_{q}C(O)NRⁿ(CH₂CH₂O)_{q}-CH₂-CH₃, - (CH₂CH₂O)_{q}C(O)NRⁿ(CH₂CH₂O)_{q}-CH₃, -(CH₂CH₂O)_{q}C(O)NRⁿ(CH₂CH₂O)_{q}-CH₂-CH₃ or - (CH₂CH₂O)_{q}C(O)NRⁿ(CH₂)_{q}-CH₃; wherein each Rⁿ is independently H, C1-C6 alkyl, C3-C8 carbocyclyl, phenyl or benzyl, and each q is independently 1, 2, 3, 4, 5, 6, 7, 8, 9 or 10;
** links to a linker;
y is 0, 1 or 2;
Y is O, S or CR¹R², wherein R¹ and R² are each independently H or C1-C6 alkyl;
s and t are each independently 0, 1 or 2, but not both 0.

In one or more embodiments, X⁴ is H or C1-C6 alkyl.

In one or more embodiments, the heterocyclyl is azetidine, niverazine, morpholine, pyrrolidine, piperidine, imidazole, thiazole, oxazole or pyridine.

In one or more embodiments, the amino-protecting group is formyl, acetyl, trityl, *t*-butoxycarbonyl, benzyl, or *p-*methoxybenzyloxycarbonyl.

In one or more embodiments, the drug is 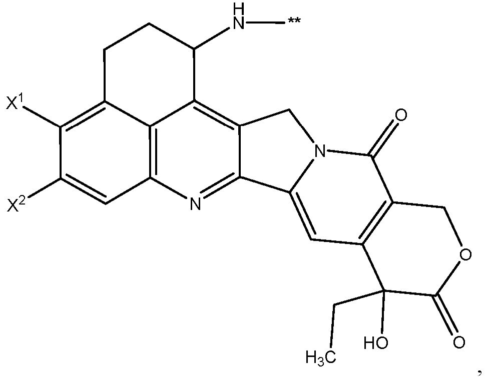 or a stereoisomer thereof, wherein X¹ and X² are each independently C1-C6 alkyl, halogen or -OH; ** links to a linker.

In one or more embodiments, the drug is 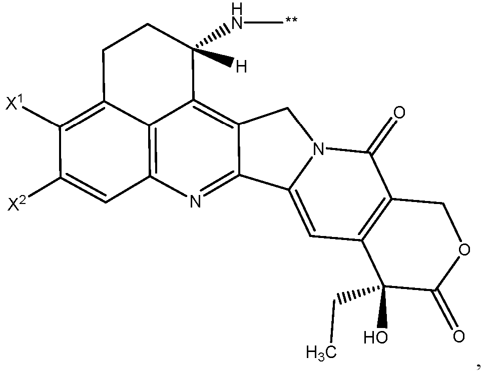 or a stereoisomer thereof, wherein X¹ and X² are each independently C1-C6 alkyl, halogen or -OH; ** links to a linker.

In one or more embodiments, X¹ and X² are each -CH₃.

In one or more embodiments, X¹ and X² are each independently F, Cl, Br or I.

In one or more embodiments, X¹ and X² are each F.

In one or more embodiments, X¹ and X² are each independently -CH₃, F, or -OH.

In one or more embodiments, X¹ and X² are each independently F or -CH₃.

In one or more embodiments, X¹ is -CH₃, and X² is F.

In one or more embodiments, the antibody-drug conjugate of the present invention has a structure shown as Formula I or a stereoisomer thereof, or a pharmaceutically acceptable salt or solvate thereof: 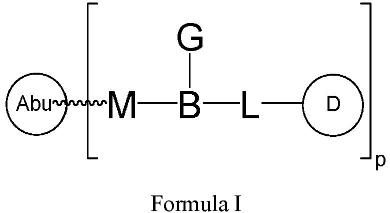 wherein
Abu is an antibody or antigen-binding unit, wherein the antibody or antigen-binding unit specifically binds to CLDN18.2; D is a drug;
M is 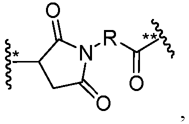 wherein * links to Abu, ** links to B, and R is selected from: -(CH₂)ᵣ-, - (CHR^{m})ᵣ-, C3-C8 carbocyclyl, -O-(CH₂)ᵣ-, arylene, -(CH₂)ᵣ-arylene-, -arylene-(CH₂)ᵣ-, -(CH₂)ᵣ-(C3-C8 carbocyclyl)-, -(C3-C8 carbocyclyl)-(CH₂)ᵣ-, C3-C8 heterocyclyl, -(CH₂)ᵣ-(C3-C8 heterocyclyl)-, -(C3-C8 heterocyclyl)-(CH₂)ᵣ-, -(CH₂)ᵣC(O)NR^{m}(CH₂)ᵣ-, -(CH₂CH₂O)ᵣ-, -(CH₂CH₂O)ᵣ-CH₂-, - (CH₂)ᵣC(O)NR^{m}(CH₂CH₂O)ᵣ-, -(CH₂)ᵣC(O)NR^{m}(CH₂CH₂O)ᵣ-CH₂-, - (CH₂CH₂O)ᵣC(O)NR^{m}(CH₂CH₂O)ᵣ-, -(CH₂CH₂O)ᵣC(O)NR^{m}(CH₂CH₂O)ᵣ-CH₂- and - (CH₂CH₂O)ᵣC(O)NR^{m}(CH₂)ᵣ-; wherein each R^{m} is independently H, C1-C6 alkyl, C3-C8 carbocyclyl, phenyl or benzyl, and each r is independently 1, 2, 3, 4, 5, 6, 7, 8, 9 or 10;
B is 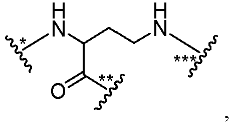 e.g., 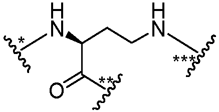 ; wherein * links to M, ** links to L, and *** links to G;
L is -(AA)ᵢ-(FF)_{f}-, wherein AA is an amino acid or polypeptide, and i is 1, 2, 3, 4, 5, 6, 7, 8, 9, 10, 11, 12, 13, 14, 15, 16, 17, 18, 19 or 20; each FF is independently 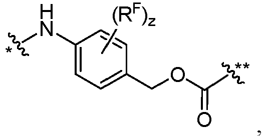 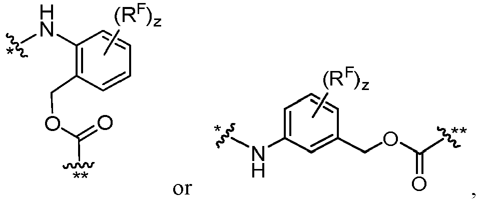 wherein each R^{F} is independently C1-C6 alkyl, C1-C6 alkoxy, -NO₂ or halogen; z is 0, 1, 2, 3 or 4; f is 1, 2, 3, 4, 5, 6, 7, 8, 9 or 10; wherein * links to AA, and ** links to D;
G is 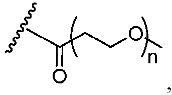 wherein n is an integer from 1 to 24, e.g., 1, 2, 3, 4, 5, 6, 7, 8, 9, 10, 11, 12, 13, 14, 15, 16, 17, 18, 19, 20, 21, 22, 23 or 24;
p is an integer from 1 to 10, e.g., 1, 2, 3, 4, 5, 6, 7, 8, 9 or 10.

In one or more embodiments, the drug is an anti-cancer drug, a cytotoxic drug, a cell differentiation factor, a stem cell trophic factor, a steroid drug, a drug for treating autoimmune diseases, an anti-inflammatory drug or a drug for treating infectious diseases.

In one or more embodiments, the drug is an anti-cancer drug.

In one or more embodiments, the drug is a tubulin inhibitor, a DNA damaging agent, or a DNA topoisomerase inhibitor.

In one or more embodiments, the tubulin inhibitor is selected from dolastatin, auristatins and maytansinoids.

In one or more embodiments, the drug is an auristatin, e.g., MMAE, MMAF, or AF.

In one or more embodiments, the drug is a DNA damaging agent, e.g., a calicheamicin, a duocarmycin, or an anthramycin derivative PBD (pyrrolobenzodiazepine).

In one or more embodiments, the drug is a DNA topoisomerase inhibitor or a salt thereof, e.g., irinotecan, irinotecan hydrochloride, camptothecin, 9-aminocamptothecin, 9-nitrocamptothecin, 10-hydroxycamptothecin, 9-chloro-10-hydroxycamptothecin, a camptothecin derivative SN-38, 22-hydroxyacuminatine, topotecan, lurtotecan, belotecan, exatecan, an exatecan derivative, homosilatecan, 6,8-dibromo-2-methyl-3-[2-(D-xylopyranosylamino)phenyl]-4(3*H*)-quinazolinone, 2-cyano-3-(3,4-dihydroxyphenyl)-*N*-(phenylmethyl)-(2*E*)-2-propenamide, 2-cyano-3-(3,4-dihydroxyphenyl)-*N*-(3-hydroxyphenylpropyl)-(*E*)-2-propenamide, 12-β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5,7(6*H*)-dione, *N-*[2-(dimethylamino)ethyl]-4-acridinecarboxamide dihydrochloride or *N*-[2-(dimethylamino)ethyl]-4-acridinecarboxamide.

In one or more embodiments, the DNA topoisomerase inhibitor is camptothecin, 10-hydroxycamptothecin, topotecan, belotecan, irinotecan, 22-hydroxyacuminatine or exatecan.

In one or more embodiments, the drug is a tubulysin, a taxane drug derivative, a leptomycine derivative, CC-1065 or an analog thereof, an amatoxin, a spliceosome inhibitor, a benzodiazepine (PBD) dimer, adriamycin, methotrexate, vincristine, vinblastine, daunorubicin, mitomycin C, melphalan or a chlorambucil derivative.

In one or more embodiments, the drug has an amino group or an amino group substituted with one alkyl group, and links to FF via an amide bond.

In one or more embodiments, the drug is 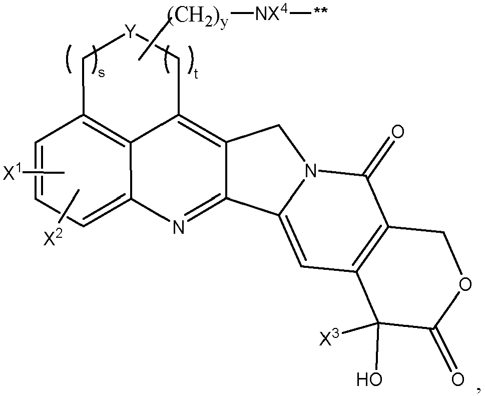 wherein
X¹ and X² are each independently:
H,
hydroxy,
C1-C6 alkyl,
C1-C6 alkyl substituted with one or more hydroxy, halogen, nitro or cyano groups,
C2-C6 alkenyl,
C2-C6 alkynyl,
C1-C6 alkoxy,
C1-C6 aminoalkoxy,
halogen,
nitro,
cyano,
thiol,
alkylthio,
amino, amino substituted with an amino-protecting group, C1-C6 aminoalkyl optionally substituted at the amino moiety with an amino-protecting group or C1-C6 alkyl,
C1-C6 aminoalkylamino optionally substituted at the amino moiety with an amino-protecting group or C1-C6 alkyl,
C1-C6 alkyl linking to a heterocyclic ring, wherein the heterocyclic ring is optionally substituted with one or more C1-C6 alkyl, C1-C6 alkoxy, amino, halogen, nitro or cyano groups,
C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or protecting group,
amino-substituted heterocyclyl optionally substituted at a nitrogen atom of the heterocyclyl moiety or at the amino moiety with a protecting group or one or more C1-C6 alkyl groups,
heterocyclylamino optionally substituted at a nitrogen atom of the heterocyclyl moiety or at the amino moiety with a protecting group or C1-C6 alkyl,
carbamoyl optionally substituted with a carbamoyl-protecting group or C1-C6 alkyl,
morpholin-1-yl, or
piperidin-1-yl;
X³ is C1-C6 alkyl;
X⁴ is H, -(CH₂)_{q}-CH₃, -(CHRⁿ)_{q}-CH₃, C3-C8 carbocyclyl, -O-(CH₂)_{q}-CH₃, arylene-CH₃, -(CH₂)_{q}-arylene-CH₃, -arylene-(CH₂)_{q}-CH₃, -(CH₂)_{q}-(C3-C8 carbocyclyl)-CH₃, -(C3-C8 carbocyclyl)-(CH₂)_{q}-CH₃, C3-C8 heterocyclyl, -(CH₂)_{q}-(C3-C8 heterocyclyl)-CH₃, -(C3-C8 heterocyclyl)-(CH₂)_{q}-CH₃, - (CH₂)_{q}C(O)NRⁿ(CH₂)_{q}-CH₃, -(CH₂CH₂O)_{q}-CH₃, -(CH₂CH₂O)_{q}-CH₂-CH₃, - (CH₂)_{q}C(O)NRⁿ(CH₂CH₂O)_{q}-CH₃, -(CH₂)_{q}C(O)NRⁿ(CH₂CH₂O)_{q}-CH₂-CH₃, - (CH₂CH₂O)_{q}C(O)NRⁿ(CH₂CH₂O)_{q}-CH₃, -(CH₂CH₂O)_{q}C(O)NRⁿ(CH₂CH₂O)_{q}-CH₂-CH₃ or - (CH₂CH₂O)_{q}C(O)NRⁿ(CH₂)_{q}-CH₃; wherein each Rⁿ is independently H, C1-C6 alkyl, C3-C8 carbocyclyl, phenyl or benzyl, and each q is independently 1, 2, 3, 4, 5, 6, 7, 8, 9 or 10;
** links to L;
y is 0, 1 or 2;
Y is O, S or CR¹R², wherein R¹ and R² are each independently H or C1-C6 alkyl;
s and t are each independently 0, 1 or 2, but not both 0.

In one or more embodiments, X⁴ is H or C1-C6 alkyl.

In one or more embodiments, the heterocyclyl is azetidine, niverazine, morpholine, pyrrolidine, piperidine, imidazole, thiazole, oxazole or pyridine.

In one or more embodiments, the amino-protecting group is formyl, acetyl, trityl, t-butoxycarbonyl, benzyl, or p-methoxybenzyloxycarbonyl.

In one or more embodiments, the drug is 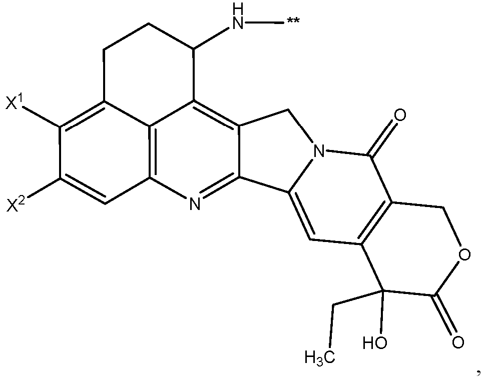 wherein X¹ and X² are each independently C1-C6 alkyl, halogen, or -OH; ** links to L.

In one or more embodiments, the drug is 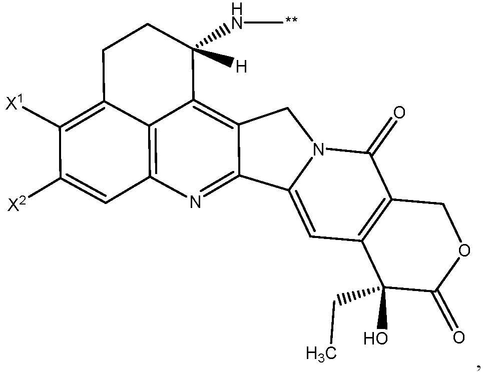 wherein X¹ and X² are each independently C1-C6 alkyl, halogen, or -OH; ** links to L.

In one or more embodiments, X¹ and X² are each -CH₃.

In one or more embodiments, X¹ and X² are each independently F, Cl, Br or I.

In one or more embodiments, X¹ and X² are each F.

In one or more embodiments, X¹ and X² are each independently -CH₃, F, or -OH.

In one or more embodiments, X¹ and X² are each independently F or -CH₃.

In one or more embodiments, X¹ is -CH₃, and X² is F.

In one or more embodiments, R is -(CH₂)ᵣ-.

In one or more embodiments, R is -(CH₂)ᵣ-, wherein r is 1 or 5.

In one or more embodiments, each AA is independently selected from the following amino acid or peptide sequences: Val-Cit, Val-Lys, Phe-Lys, Lys-Lys, Ala-Lys, Phe-Cit, Leu-Cit, Ile-Cit, Trp, Cit, Phe-Ala, Phe-Phe-Lys, D-Phe-Phe-Lys, Gly-Phe-Lys, Leu-Ala-Leu, Ile-Ala-Leu, Val-Ala-Val, Ala-Leu-Ala-Leu, β-Ala-Leu-Ala-Leu, and Gly-Phe-Leu-Gly.

In one or more embodiments, i is 1.

In one or more embodiments, AA is Val-Cit, and i is 1.

In one or more embodiments, each FF is independently 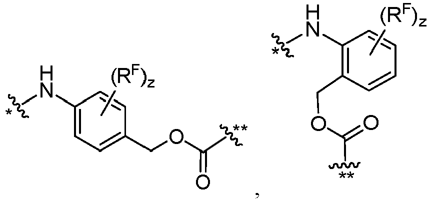 or 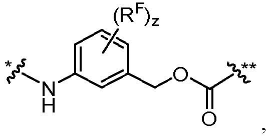 wherein each R^{F} is independently C1-C6 alkyl, C1-C6 alkoxy, -NO₂ or halogen; wherein * links to AA, and ** links to D.

In one or more embodiments, halogen is F, and z is 0, 1, 2, 3 or 4.

In one or more embodiments, R^{F} is -CH₃, F, -NO₂ or -OCH₃.

In one or more embodiments, z is 0.

In one or more embodiments, z is 1 or 2.

In one or more embodiments, each FF is independently 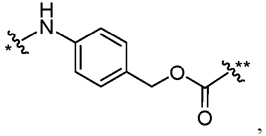 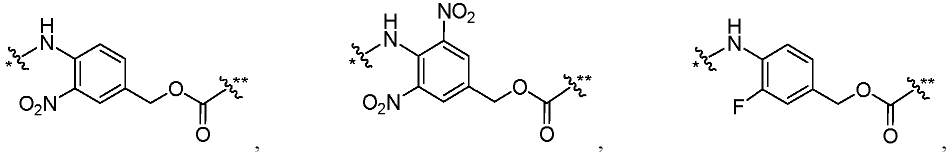 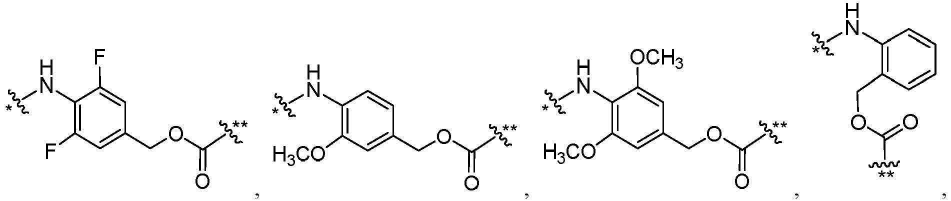 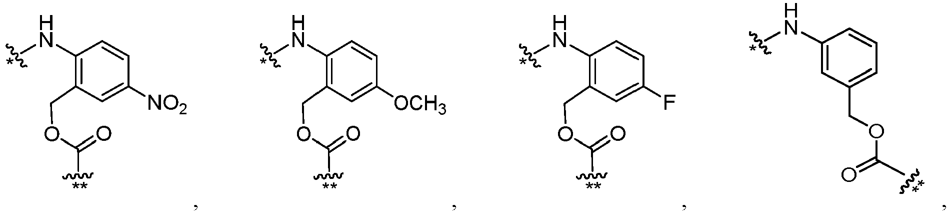 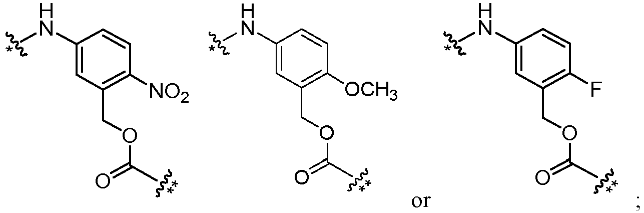 wherein * links to AA, and ** links to D.

In one or more embodiments, f is 1.

In one or more embodiments, FF is 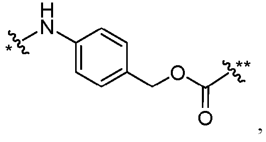 and f is 1; wherein * links to AA, and ** links to D.

In one or more embodiments, L is 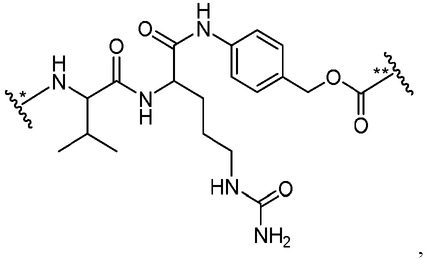 wherein * links to B, and ** links to D.

In one or more embodiments, L is 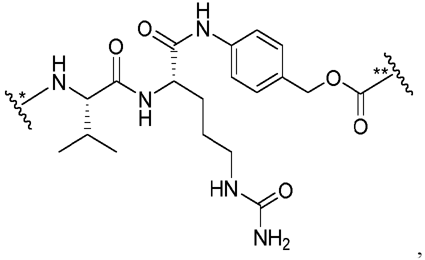 wherein * links to B, and ** links to D.

In one or more embodiments, G is 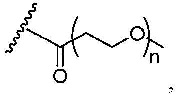 and n is 4-12.

In one or more embodiments, n is 4-8.

In one or more embodiments, n is 4.

In one or more embodiments, n is 8.

In one or more embodiments, p is 2-8.

In one or more embodiments, p is 4-8.

In one or more embodiments, p is 6-8.

In one or more embodiments, p is 7-8.

In one or more embodiments, Abu is the antibody or antigen-binding unit provided by the present invention.

In one or more embodiments, the antibody-drug conjugate has a structure shown as Formula I-1 or Formula I-2, or a stereoisomer thereof, or a pharmaceutically acceptable salt or solvate thereof: 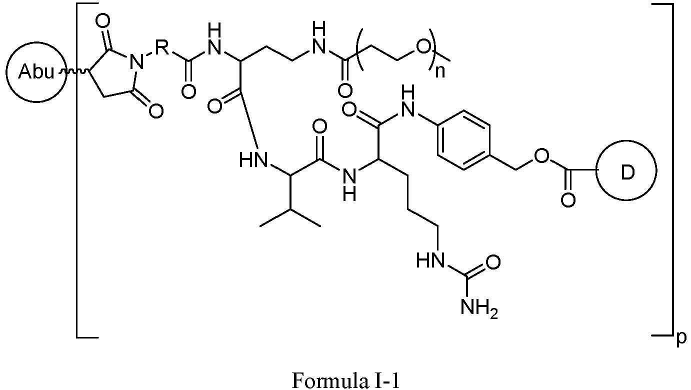 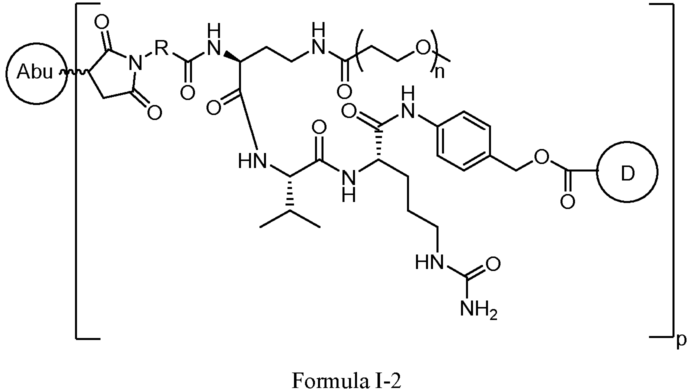 wherein
Abu is the antibody or antigen-binding unit provided by the present invention;
R is selected from: -(CH₂)ᵣ-, -(CHR^{m})ᵣ-, C3-C8 carbocyclyl, -O-(CH₂)ᵣ-, arylene, -(CH₂)ᵣ-arylene-, - arylene-(CH₂)ᵣ-, -(CH₂)ᵣ-(C3-C8 carbocyclyl)-, -(C3-C8 carbocyclyl)-(CH₂)ᵣ-, C3-C8 heterocyclyl, - (CH₂)ᵣ-(C3-C8 heterocyclyl)-, -(C3-C8 heterocyclyl)-(CH₂)ᵣ-, -(CH₂)ᵣC(O)NR^{m}(CH₂)ᵣ-, -(CH₂CH₂O)ᵣ-, -(CH₂CH₂O)ᵣ-CH₂-, -(CH₂)ᵣC(O)NR^{m}(CH₂CH₂O)ᵣ-, -(CH₂)ᵣC(O)NR^{m}(CH₂CH₂O)ᵣ-CH₂-, - (CH₂CH₂O)ᵣC(O)NR^{m}(CH₂CH₂O)ᵣ-, -(CH₂CH₂O)ᵣC(O)NR^{m}(CH₂CH₂O)ᵣ-CH₂- and - (CH₂CH₂O)ᵣC(O)NR^{m}(CH₂)ᵣ-; wherein each R^{m} is independently H, C1-C6 alkyl, C3-C8 carbocyclyl, phenyl or benzyl, and each r is independently 1, 2, 3, 4, 5, 6, 7, 8, 9 or 10;
D is a drug;
n is an integer from 1 to 24, e.g., 1, 2, 3, 4, 5, 6, 7, 8, 9, 10, 11, 12, 13, 14, 15, 16, 17, 18, 19, 20, 21, 22, 23 or 24;
p is 1-10, e.g., 1, 2, 3, 4, 5, 6, 7, 8, 9 or 10.

In one or more embodiments, R is -(CH₂)ᵣ-.

In one or more embodiments, , R is -(CH₂)ᵣ-, r is 1 or 5.

In one or more embodiments, n is 4-12.

In one or more embodiments, n is 4-8.

In one or more embodiments, n is 4.

In one or more embodiments, n is 8.

In one or more embodiments, p is 2-8.

In one or more embodiments, p is 4-8.

In one or more embodiments, p is 6-8.

In one or more embodiments, p is 7-8.

In one or more embodiments, the antibody-drug conjugate has a structure shown as Formula I-3 or Formula I-4, or a stereoisomer thereof, or a pharmaceutically acceptable salt or solvate thereof: 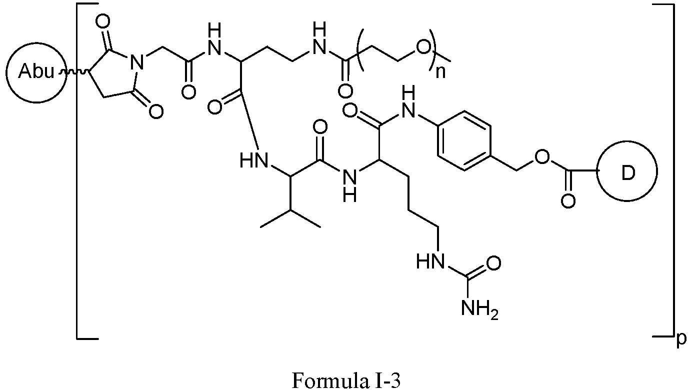 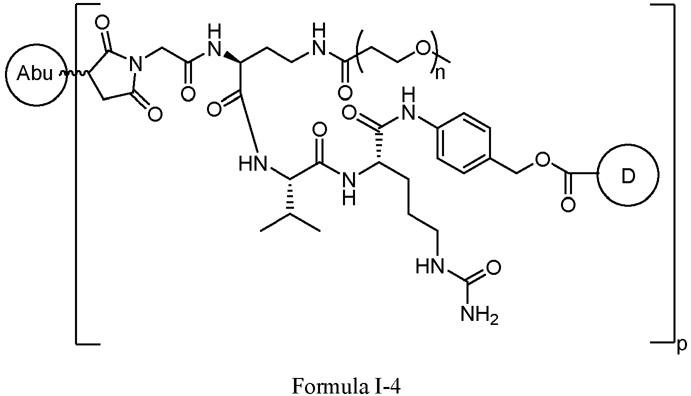 wherein
Abu is the antibody or antigen-binding unit provided by the present invention;
D is a drug;
n is an integer from 1 to 24, e.g., 1, 2, 3, 4, 5, 6, 7, 8, 9, 10, 11, 12, 13, 14, 15, 16, 17, 18, 19, 20, 21, 22, 23 or 24;
p is 1-10, e.g., 1, 2, 3, 4, 5, 6, 7, 8, 9 or 10.

In one or more embodiments, n is 4-12.

In one or more embodiments, n is 4-8.

In one or more embodiments, n is 4.

In one or more embodiments, n is 8.

In one or more embodiments, p is 2-8.

In one or more embodiments, p is 4-8.

In one or more embodiments, p is 6-8.

In one or more embodiments, p is 7-8.

In one or more embodiments, the antibody-drug conjugate has a structure shown as Formula I-5, I-6, I-7, I-8, I-9, I-10, or I-11, or a stereoisomer thereof, or a pharmaceutically acceptable salt or solvate thereof: 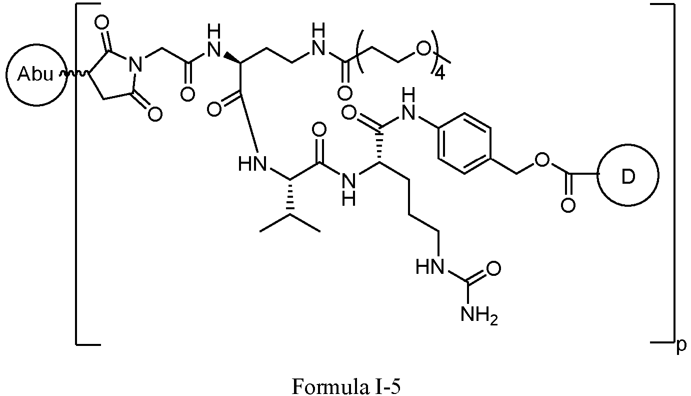 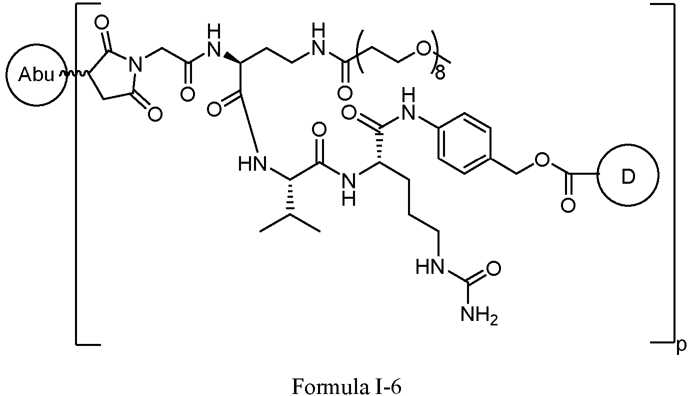 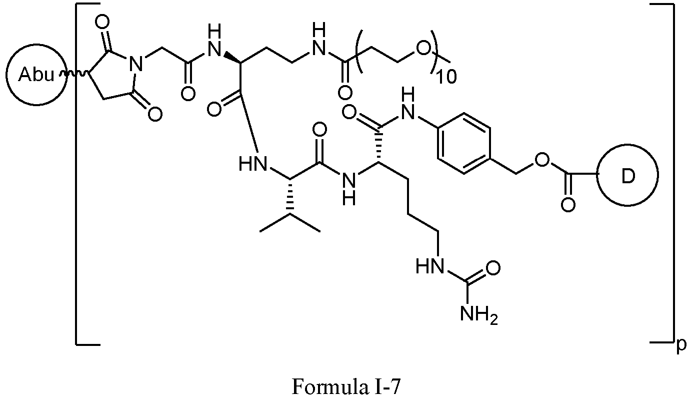 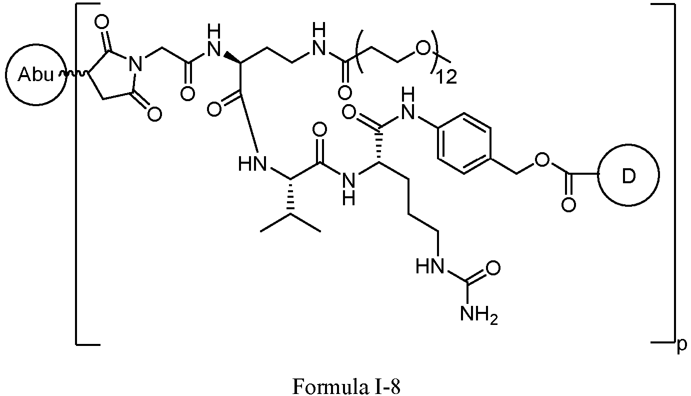 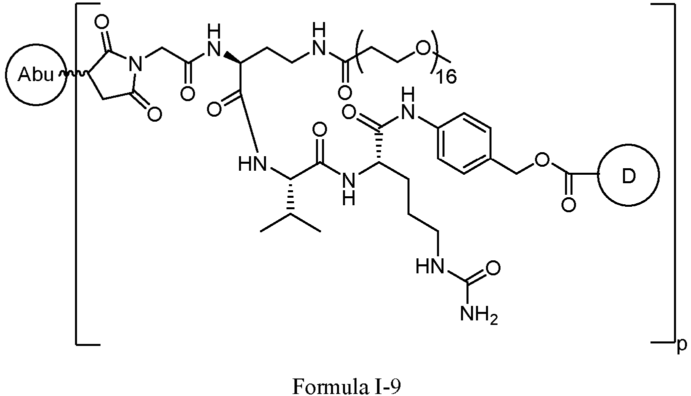 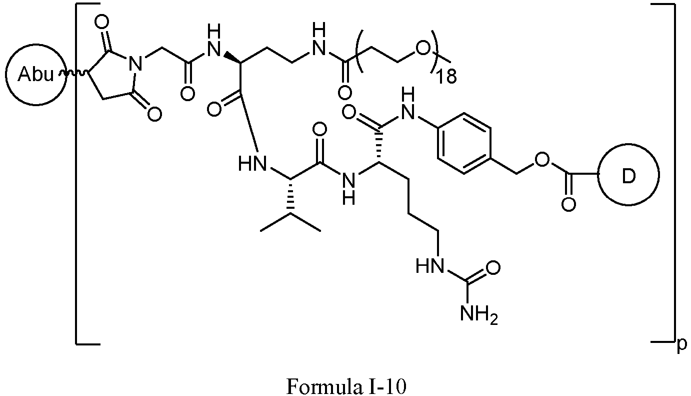 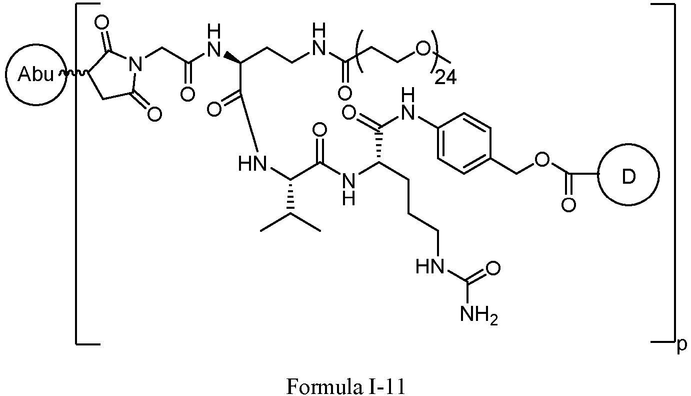 wherein
Abu is the antibody or antigen-binding unit provided by the present invention;
D is a drug;
p is 1-10, e.g., 1, 2, 3, 4, 5, 6, 7, 8, 9 or 10.

In one or more embodiments of Formula I-1, I-2, I-3, I-4, I-5, I-6, I-7, I-8, I-9, I-10, or I-11, p is 2-8.

In one or more embodiments of Formula I-1, I-2, I-3, I-4, I-5, I-6, I-7, I-8, I-9, I-10, or I-11, p is 4-8.

In one or more embodiments of Formula I-1, I-2, I-3, I-4, I-5, I-6, I-7, I-8, I-9, I-10, or I-11, p is 6-8.

In one or more embodiments of Formula I-1, I-2, I-3, I-4, I-5, I-6, I-7, I-8, I-9, I-10, or I-11, p is 7-8.

In one or more embodiments of Formula I-1, I-2, I-3, I-4, I-5, I-6, I-7, I-8, I-9, I-10, or I-11, the drug is an anti-cancer drug, a cytotoxic drug, a cell differentiation factor, a stem cell trophic factor, a steroid drug, a drug for treating autoimmune diseases, an anti-inflammatory drug or a drug for treating infectious diseases.

In one or more embodiments of Formula I-1, I-2, I-3, I-4, I-5, I-6, I-7, I-8, I-9, I-10, or I-11, the drug is a tubulin inhibitor, a DNA damaging agent, or a DNA topoisomerase inhibitor.

In one or more embodiments of Formula I-1, I-2, I-3, I-4, I-5, I-6, I-7, I-8, I-9, I-10, or I-11, the tubulin inhibitor is selected from dolastatin, auristatins and maytansinoids.

In one or more embodiments of Formula I-1, I-2, I-3, I-4, I-5, I-6, I-7, I-8, I-9, I-10, or I-11, the drug is an auristatin, e.g., MMAE, MMAF, or AF.

In one or more embodiments of Formula I-1, I-2, I-3, I-4, I-5, I-6, I-7, I-8, I-9, I-10, or I-11, the drug is DNA damaging agent, e.g., a calicheamicin, a duocarmycin, or an anthramycin derivative PBD (pyrrolobenzodiazepine).

In one or more embodiments of Formula I-1, I-2, I-3, I-4, I-5, I-6, I-7, I-8, I-9, I-10, or I-11, the drug is a DNA topoisomerase inhibitor or a salt thereof, e.g., irinotecan, irinotecan hydrochloride, camptothecin, 9-aminocamptothecin, 9-nitrocamptothecin, 10-hydroxycamptothecin, 9-chloro-10-hydroxycamptothecin, a camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3*H*)-quinazolinone, 2-cyano-3-(3,4-dihydroxyphenyl)-*N*-(phenylmethyl)-(2*E*)-2-propenamide, 2-cyano-3-(3,4-dihydroxyphenyl)-*N*-(3-hydroxyphenylpropyl)-(*E*)-2-propenamide, 12-β-D-glucopyranosyl-12,13-dihydro-2,10-dihydroxy-6-[[2-hydroxy-1-(hydroxymethyl)ethyl]amino]-5*H-*indolo[2,3-*a*]pyrrolo[3,4-*c*]carbazole-5,7(6*H*)-dione, *N*-[2-(dimethylamino)ethyl]-4-acridinecarboxamide dihydrochloride, or *N*-[2-(dimethylamino)ethyl]-4-acridinecarboxamide.

In one or more embodiments of Formula I-1, I-2, I-3, I-4, I-5, I-6, I-7, I-8, I-9, I-10, or I-11, the DNA topoisomerase inhibitor is camptothecin, 10-hydroxycamptothecin, topotecan, belotecan, irinotecan, 22-hydroxyacuminatine or exatecan.

In one or more embodiments, the drug is a tubulysin, a taxane drug derivative, a leptomycine derivative, CC-1065 or an analog thereof, an amatoxin, a spliceosome inhibitor, a benzodiazepine (PBD) dimer, adriamycin, methotrexate, vincristine, vinblastine, daunorubicin, mitomycin C, melphalan or a chlorambucil derivative.

In one or more embodiments of Formula I-1, I-2, I-3, I-4, I-5, I-6, I-7, I-8, I-9, I-10, or I-11, the drug is 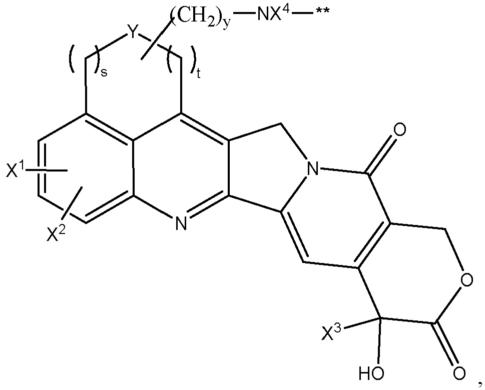 wherein
X¹ and X² are each independently:
H,
hydroxy,
C1-C6 alkyl,
C1-C6 alkyl substituted with one or more hydroxy, halogen, nitro or cyano groups,
C2-C6 alkenyl,
C2-C6 alkynyl,
C1-C6 alkoxy,
C1-C6 aminoalkoxy,
halogen,
nitro,
cyano,
thiol,
alkylthio,
amino, amino substituted with an amino-protecting group, C1-C6 aminoalkyl optionally substituted at the amino moiety with an amino-protecting group or C1-C6 alkyl,
C1-C6 aminoalkylamino optionally substituted at the amino moiety with an amino-protecting group or C1-C6 alkyl,
C1-C6 alkyl linking to a heterocyclic ring, wherein the heterocyclic ring is optionally substituted with one or more C1-C6 alkyl, C1-C6 alkoxy, amino, halogen, nitro or cyano groups,
C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or protecting group,
amino-substituted heterocyclyl optionally substituted at a nitrogen atom of the heterocyclyl moiety or at the amino moiety with a protecting group or one or more C1-C6 alkyl groups,
heterocyclylamino optionally substituted at a nitrogen atom of the heterocyclyl moiety or at the amino moiety with a protecting group or C1-C6 alkyl,
carbamoyl optionally substituted with a carbamoyl-protecting group or C1-C6 alkyl,
morpholin-1-yl, or
piperidin-1-yl;
X³ is C1-C6 alkyl;
X⁴ is H, -(CH₂)_{q}-CH₃, -(CHRⁿ)_{q}-CH₃, C3-C8 carbocyclyl, -O-(CH₂)_{q}-CH₃, arylene-CH₃, -(CH₂)_{q}-arylene-CH₃, -arylene-(CH₂)_{q}-CH₃, -(CH₂)_{q}-(C3-C8 carbocyclyl)-CH₃, -(C3-C8 carbocyclyl)-(CH₂)_{q}-CH₃, C3-C8 heterocyclyl, -(CH₂)_{q}-(C3-C8 heterocyclyl)-CH₃, -(C3-C8 heterocyclyl)-(CH₂)_{q}-CH₃, - (CH₂)_{q}C(O)NRⁿ(CH₂)_{q}-CH₃, -(CH₂CH₂O)_{q}-CH₃, -(CH₂CH₂O)_{q}-CH₂-CH₃, - (CH₂)_{q}C(O)NRⁿ(CH₂CH₂O)_{q}-CH₃, -(CH₂)_{q}C(O)NRⁿ(CH₂CH₂O)_{q}-CH₂-CH₃, - (CH₂CH₂O)_{q}C(O)NRⁿ(CH₂CH₂O)_{q}-CH₃, -(CH₂CH₂O)_{q}C(O)NRⁿ(CH₂CH₂O)_{q}-CH₂-CH₃ or - (CH₂CH₂O)_{q}C(O)NRⁿ(CH₂)_{q}-CH₃; wherein each Rⁿ is independently H, C1-C6 alkyl, C3-C8 carbocyclyl, phenyl or benzyl, and each q is independently 1, 2, 3, 4, 5, 6, 7, 8, 9 or 10;
** links to other parts of the antibody-drug conjugate;
y is 0, 1 or 2;
Y is O, S or CR¹R², wherein R¹ and R² are each independently H or C1-C6 alkyl;
s and t are each independently 0, 1 or 2, but not both 0.

In one or more embodiments, X⁴ is H or C1-C6 alkyl.

In one or more embodiments, the heterocyclyl is azetidine, niverazine, morpholine, pyrrolidine, piperidine, imidazole, thiazole, oxazole or pyridine.

In one or more embodiments, the amino-protecting group is formyl, acetyl, trityl, *t*-butoxycarbonyl, benzyl, or *p*-methoxybenzyloxycarbonyl.

In one or more embodiments of Formula I-1, I-2, I-3, I-4, I-5, I-6, I-7, I-8, I-9, I-10, or I-11, the drug is 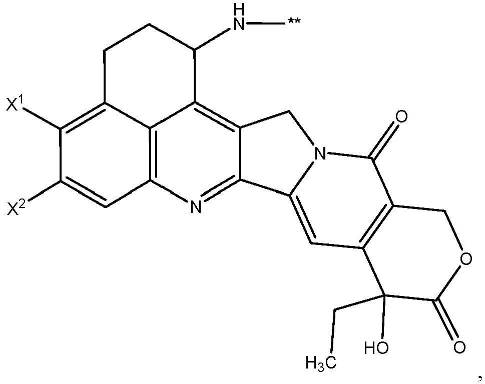 wherein X¹ and X² are each independently C1-C6 alkyl, halogen or -OH; ** links to other parts of the antibody-drug conjugate.

In one or more embodiments of Formula I-1, I-2, I-3, I-4, I-5, I-6, I-7, I-8, I-9, I-10, or I-11, the drug is 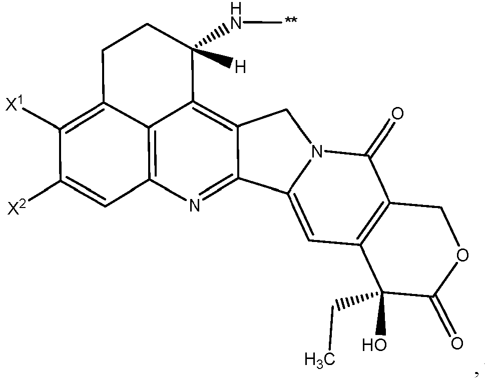 wherein X¹ and X² are each independently C1-C6 alkyl, halogen or -OH; ** links to other parts of the antibody-drug conjugate.

In one or more embodiments, C1-C6 alkyl is -CH₃

In one or more embodiments, halogen is F.

In one or more embodiments, X¹ and X² are each independently -CH₃, F or -OH.

In one or more embodiments, X¹ and X² are each independently -CH₃.

In one or more embodiments, X¹ and X² are each independently F, Cl, Br, or I.

In one or more embodiments, X¹ and X² are each F.

In one or more embodiments, X¹ and X² are each independently F or -CH₃.

In one or more embodiments, X¹ is -CH₃ and X² is F.

In one or more embodiments, the antibody-drug conjugate has a structure shown as Formula I-12, I-13, I-14, I-15, I-16, I-17, I-18, I-19, I-20, I-21, I-22, I-23, I-24, or I-25, or a stereoisomer thereof, or a pharmaceutically acceptable salt or solvate thereof 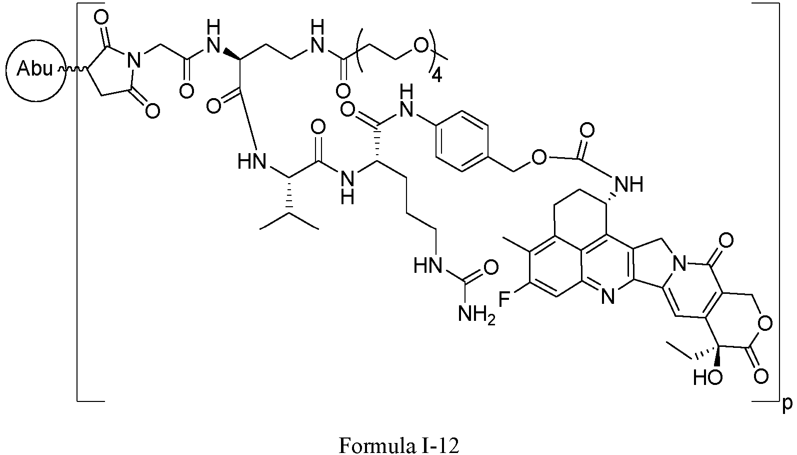 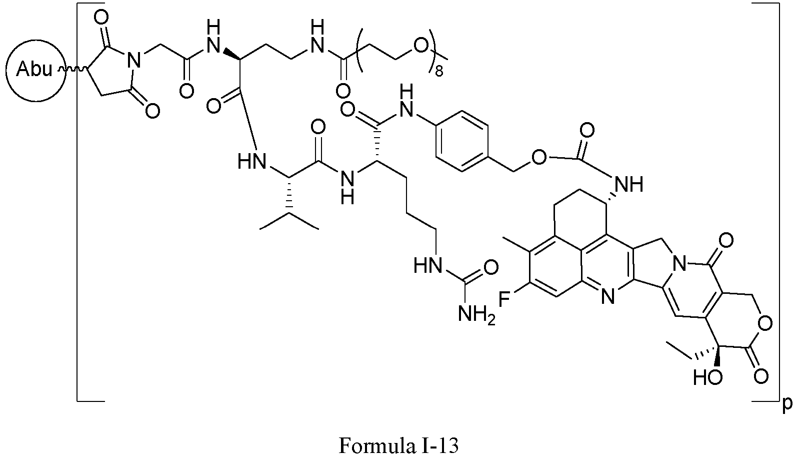 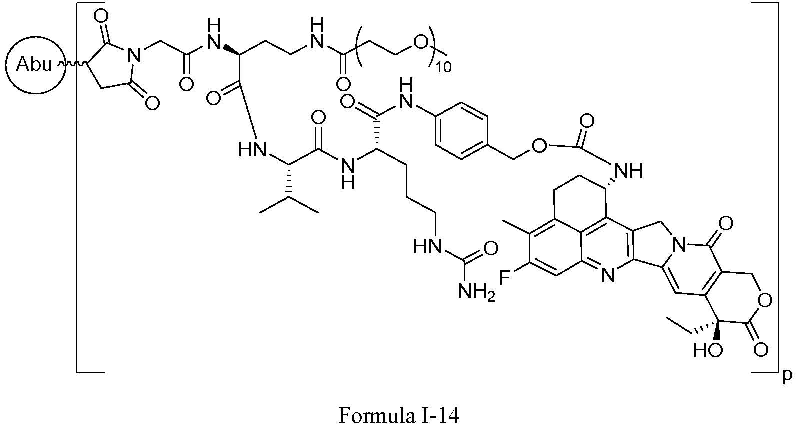 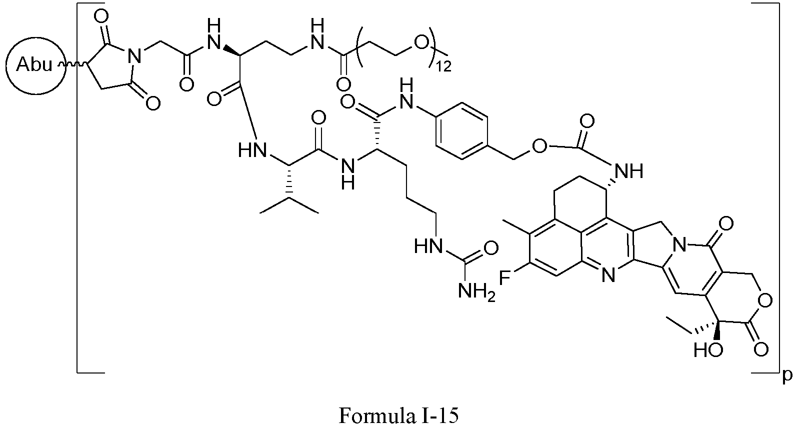 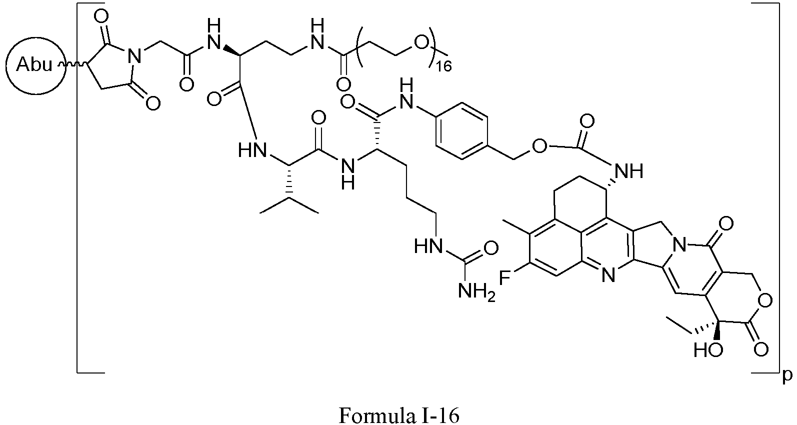 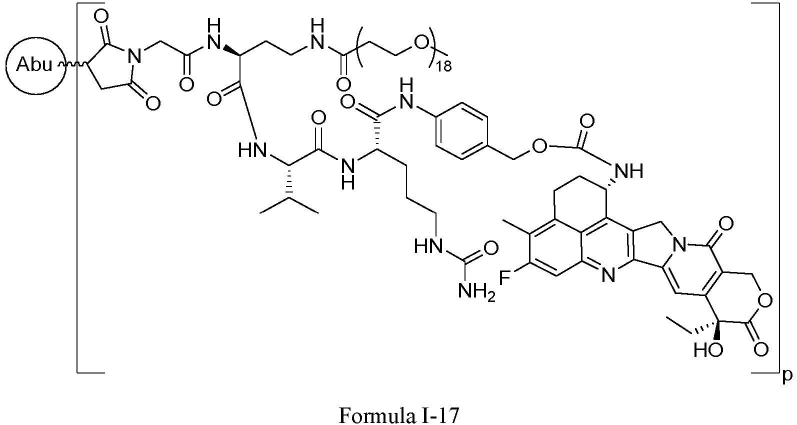 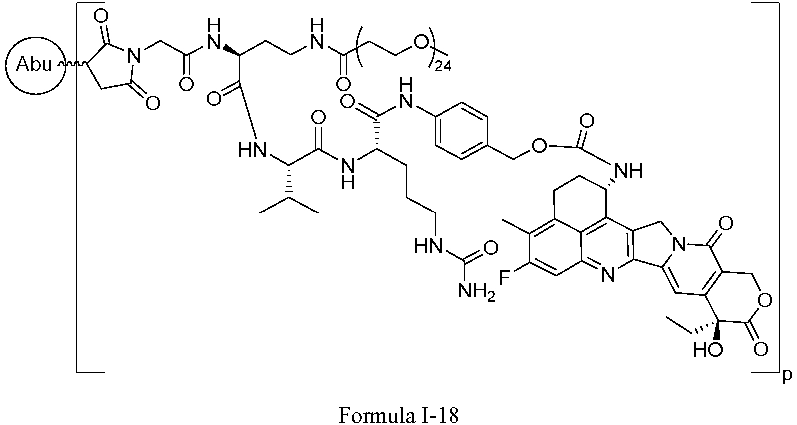 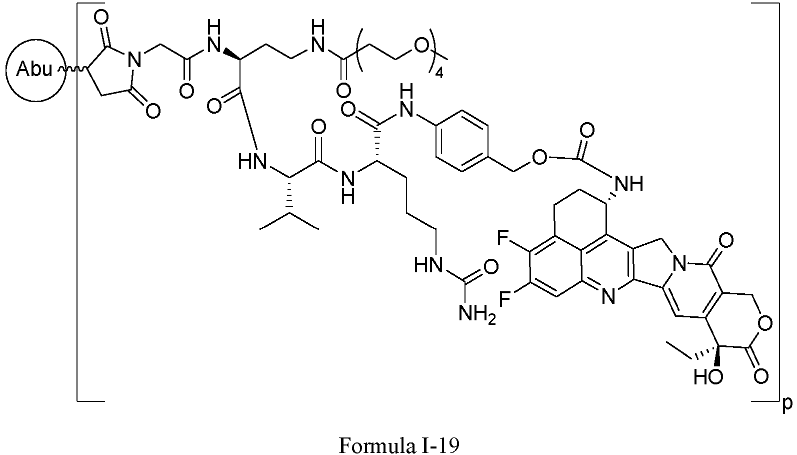 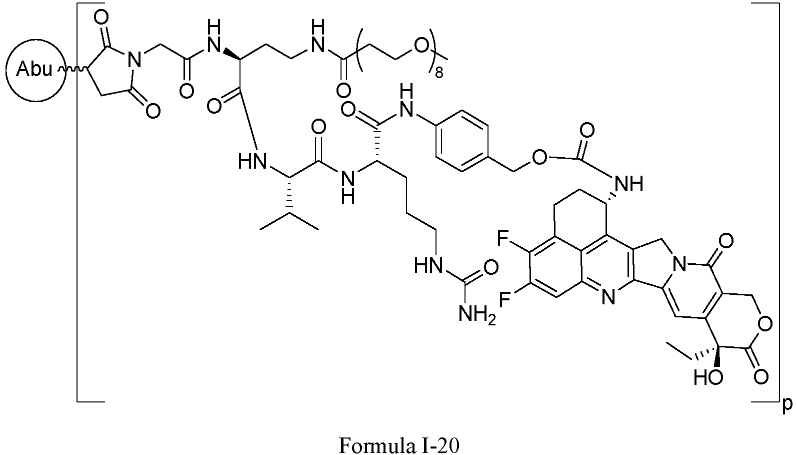 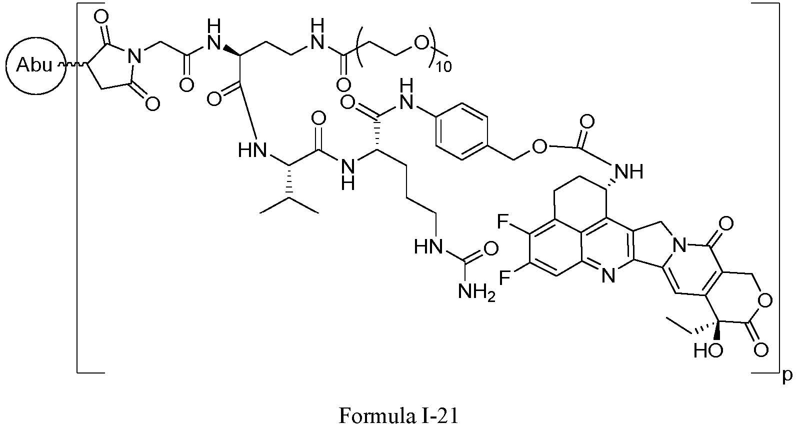 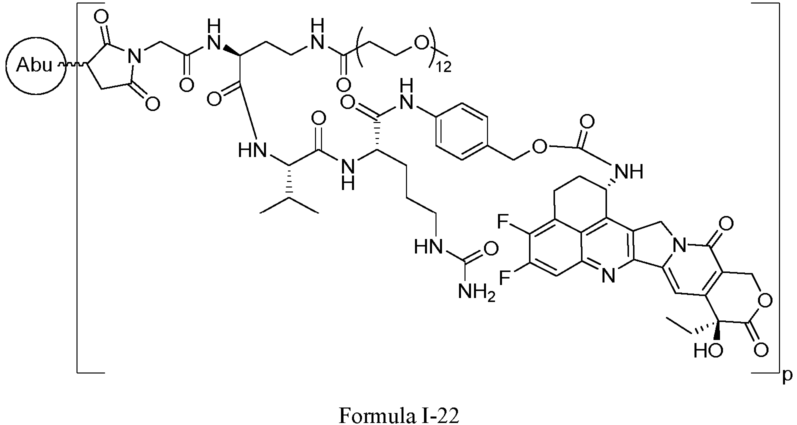 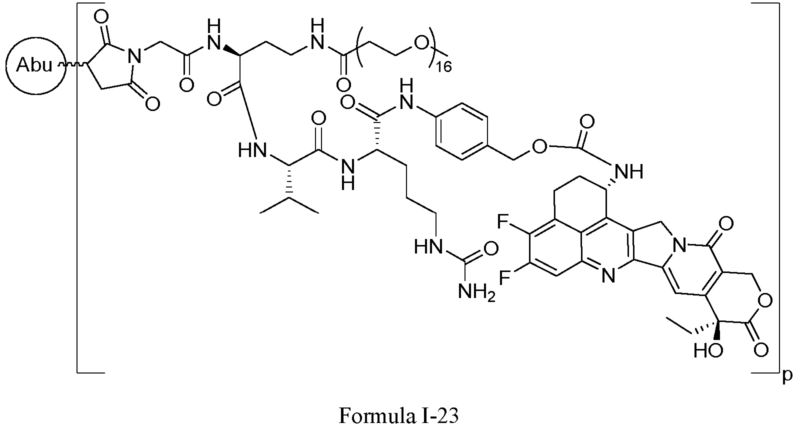 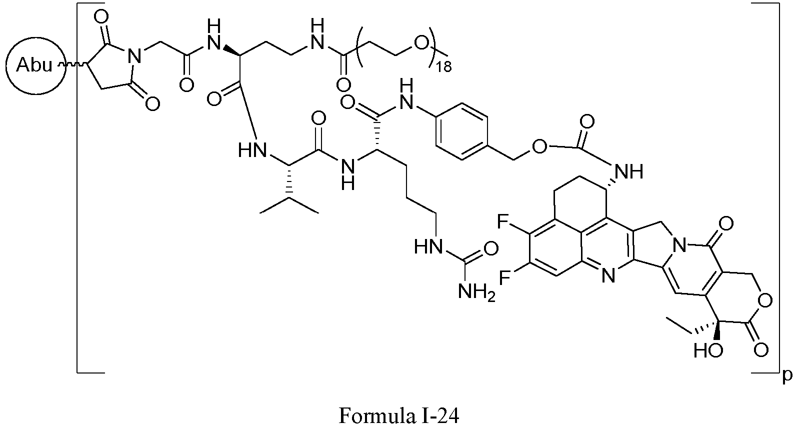 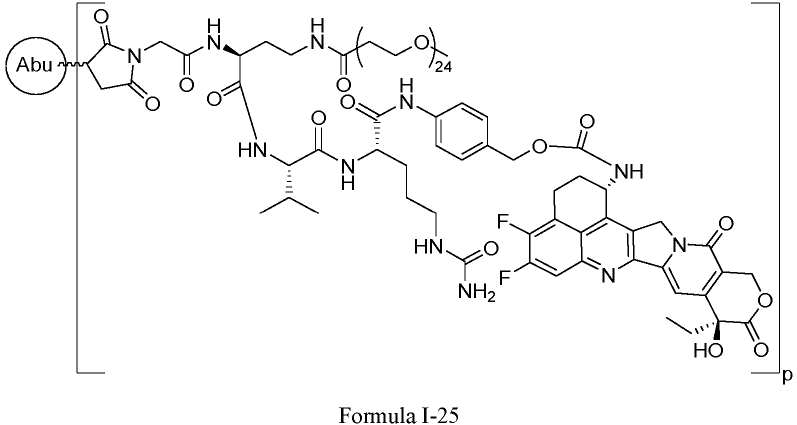 wherein
Abu is an antibody or antigen-binding unit ;
p is 1-10, e.g., 1, 2, 3, 4, 5, 6, 7, 8, 9 or 10.

In one or more embodiments, p is 2-8.

In one or more embodiments, p is 4-8.

In one or more embodiments, p is 6-8.

In one or more embodiments, p is 7-8.

In one or more embodiments, the present invention further provides an antibody or antigen-binding unit or an antibody-drug conjugate for use as a drug.

In one or more embodiments, the antibody or antigen-binding unit or the antibody-drug conjugate of the present invention is used in combination with one or more additional therapies. Suitable additional therapies include existing drugs for specific applications (such as cancer) and/or surgical therapies. For example, the antibody or antigen-binding unit or the antibody-drug conjugate is used in combination with one or more additional chemotherapeutic or anti-tumor agents. Alternatively, the additional chemotherapeutic agent is radiotherapy. In one or more embodiments, the chemotherapeutic agent is an apoptosis inducer.

In one or more embodiments, the antibody or antigen-binding unit or the antibody-drug conjugate and the additional agent are formulated into a single therapeutic composition, and the antibody or antigen-binding unit or the antibody-drug conjugate and the additional agent are administered simultaneously. Alternatively, the antibody or antigen-binding unit or the antibody-drug conjugate and the additional agent are separated from each other, for example, are each formulated into a separate therapeutic composition, and the antibody or antigen-binding unit or the antibody-drug conjugate and the additional agent are administered simultaneously, or the antibody or antigen-binding unit or the antibody-drug conjugate and the additional agent are administered at different time points during the treatment regimen. For example, the antibody or antigen-binding unit or the antibody-drug conjugate is administered prior to the administration of the additional agent or subsequent to the administration of the additional agent, or the antibody or antigen-binding unit or the antibody-drug conjugate and the additional agent are administered alternately. As used herein, the antibody or antigen-binding unit or the antibody-drug conjugate and the additional agent are administered in a single dose or multiple doses.

In one or more embodiments, the present invention further provides a pharmaceutical composition comprising the antibody or antigen-binding unit or the antibody-drug conjugate described herein, and a pharmaceutically acceptable carrier, an excipient and/or an adjuvant. In one or more embodiments, the pharmaceutical composition further comprises an additional anti-cancer drug. The pharmaceutical composition described herein may be administered by any convenient route, e.g., by infusion or bolus injection, by absorption through epithelial or cutaneous mucosa (e.g., oral mucosa or rectal and intestinal mucosa), and may be co-administered with other biologically active agents. Therefore, the pharmaceutical composition may be administered intravenously, orally, rectally, parenterally, intracistemally, intravaginally, intraperitoneally, topically (e.g. through powder, ointment, drops or transdermal patch), buccally or by oral or nasal spray.

In one or more embodiments, the present invention further provides use of at least one of the antibody or antigen-binding unit, the biomaterial (selected from the polynucleotide, the expression vector, and the cell), the antibody-drug conjugate, and the pharmaceutical composition described herein in preparing a medicament for treating and/or preventing a disease. In one or more embodiments, the disease is a disease associated with CLDN18.2 expression. In one or more embodiments, the disease is a disease associated with CLDN18.2 overexpression. In one or more embodiments, the disease is a cancer or tumor expressing CLDN18.2. In one or more embodiments, the disease is a cancer or tumor overexpressing CLDN18.2.

In one or more embodiments, the present invention further provides use of the antibody or antigen-binding unit, the biomaterial (selected from the polynucleotide, the expression vector, and the cell), the antibody-drug conjugate, and/or the pharmaceutical composition comprising same described herein in treating and/or preventing a disease. In one or more embodiments, the disease is a disease associated with CLDN18.2 expression. In one or more embodiments, the disease is a cancer or tumor. In one or more embodiments, the disease is a disease associated with CLDN18.2 overexpression. In one or more embodiments, the disease is a cancer or tumor expressing CLDN18.2. In one or more embodiments, the disease is a cancer or tumor overexpressing CLDN18.2.

In one or more embodiments, the present invention further provides a method for treating and/or preventing a disease, comprising: administering to a patient in need an effective amount of the antibody or antigen-binding unit, the biomaterial (selected from the polynucleotide, the expression vector, and the cell), the antibody-drug conjugate, and/or the pharmaceutical composition comprising same described herein. In one or more embodiments, the disease is a cancer or tumor. In one or more embodiments, the disease is a disease associated with CLDN18.2 expression. In one or more embodiments, the disease is a disease associated with CLDN18.2 overexpression. In one or more embodiments, the disease is a cancer or tumor expressing CLDN18.2. In one or more embodiments, the disease is a cancer or tumor overexpressing CLDN18.2. In one or more embodiments, the effective amount refers to an amount of an active compound or drug that results in a biological or pharmacological response in tissues, systems, animals, individuals, and humans which is being sought by researchers, vets, doctors, or other clinical physicians, including the treatment of a disease. In one or more embodiments, the effective amount is about 0.1-100 mg/kg.

Examples of the cancer include, but are not limited to, a solid tumor, a hematological cancer, and a metastatic lesion. In some embodiments, the cancer or tumor includes, but is not limited to, bladder cancer, ovarian cancer (e.g., ovarian adenocarcinoma and ovarian teratocarcinoma), lung cancer (e.g., small cell lung cancer and non-small cell lung cancer), adenocarcinoma, gastric cancer, breast cancer, liver cancer, pancreatic cancer, skin cancer (e.g., basal cell carcinoma and squamous cell carcinoma), malignant melanoma, head and neck cancer, sarcoma (e.g., synovial sarcoma and carcinosarcoma), bile duct cancer, renal cancer (clear cell renal cell carcinoma and papillary renal cell carcinoma), colon cancer, small intestine cancer, testicular embryonal carcinoma, placental choriocarcinoma, cervical cancer, testicular cancer, uterine cancer, esophageal cancer, gallbladder cancer cells, and the like.

In one or more embodiments, the antibody or the antigen-binding unit or the antibody-drug conjugate may be formulated into a pharmaceutical composition and administered to a patient in a form suitable for the chosen route of administration, e.g., parenteral, intravenous (iv), intramuscular, topical or subcutaneous administration.

In one or more embodiments, the patient receives treatment for one treatment cycle. In one or more embodiments, the patient receives treatment for multiple (e.g., 2, 3, 4, 5, 6, 7, 8, 9, 10, 11 or 12) treatment cycles. In one or more embodiments, the patient receives treatment until conditions are alleviated and no treatment is required.

In one or more embodiments, the antibody or antigen-binding unit or the antibody-drug conjugate or the pharmaceutical composition comprising the same is administered by injection. In one or more embodiments, the antibody or the antigen-binding unit or the antibody-drug conjugate or the pharmaceutical composition comprising the same is administered by subcutaneous (s.c.) injection, intraperitoneal (i.p.) injection, parenteral injection, intraarterial injection, intravenous (i.v.) injection, or the like. In one or more embodiments, the antibody or the antigen-binding unit or the antibody-drug conjugate or the pharmaceutical composition comprising the same is administered by infusion. In one or more embodiments, the antibody or the antigen-binding unit or the antibody-drug conjugate or the pharmaceutical composition comprising the same is administered by bolus injection. In one or more embodiments, the antibody or the antigen-binding unit or the antibody-drug conjugate or the pharmaceutical composition comprising the same is administered by intravenous injection. In one or more embodiments, the antibody or the antigen-binding unit or the antibody-drug conjugate or the pharmaceutical composition comprising the same is administered by intravenous infusion. The dose of the antibody or the antigen-binding unit or the antibody-drug conjugate will depend on the properties of the drug, the extent to which the internalization, transport and release of the drug are triggered at the cell surface, and the disease being treated and the conditions of the patient (e.g., age, sex and body weight).

In one or more embodiments, the antibody or the antigen-binding unit or the antibody-drug conjugate or the pharmaceutical composition comprising the same is administered by infusing intravenously (i.v.) (i.e., intravenous infusion). In one or more embodiments, the duration of the intravenous infusion is about 10 minutes, about 15 minutes, about 20 minutes, about 25 minutes, about 30 minutes, about 40 minutes, about 50 minutes, about 55 minutes, about 60 minutes, about 65 minutes, about 70 minutes, about 75 minutes, about 81 minutes, about 87 minutes, about 90 minutes, about 95 minutes, or a range between any two of these values (inclusive) or any value therein. In one or more embodiments, the duration of the intravenous infusion is less than or equal to 30 minutes. In one or more embodiments, the duration of the intravenous infusion is greater than or equal to 60 minutes. In one or more embodiments, the duration of the intravenous infusion is greater than or equal to 90 minutes.

In one or more embodiments, the present invention provides a pharmaceutical composition comprising the antibody or the antigen-binding unit or the antibody-drug conjugate and suitable for injection, such as a pharmaceutical composition for bolus injection or a pharmaceutical composition for infusion (dripping). The pharmaceutical composition suitable for injection includes a sterile aqueous solution (here water-soluble) or dispersion and sterile powders for the instant preparation of a sterile injectable solution or dispersion. For intravenous administration, a suitable carrier includes normal saline, bacteriostatic water or phosphate-buffered saline (PBS), ethanol, a solvent or dispersion medium for polyol (e.g., glycerol, propylene glycol and liquid polyethylene glycol), and a suitable mixture thereof. In one or more embodiments, the pharmaceutical composition further comprises a pharmaceutically acceptable carrier. In one or more embodiments, the pharmaceutically acceptable carrier may comprise an antibacterial and/or antifungal agent, e.g., p-hydroxylbenzoate, chlorobutanol, phenol, ascorbic acid and thimerosal. In one or more embodiments, the pharmaceutical composition comprises at least 0.1% of the antibody or the antigen-binding unit or the antibody-drug conjugate. The percentage of the antibody may vary and is between about 2% and 90% by weight of a given dosage form. The amount of the antibody or the antigen-binding unit or the antibody-drug conjugate in such a pharmaceutical composition may be an effective amount for administration.

In one or more embodiments, the present invention provides a method for preparing a pharmaceutical composition: mixing the antibody or the antigen-binding unit or the antibody-drug conjugate described herein with a pharmaceutically acceptable carrier (e.g., water for injection, normal saline, etc.). Methods for mixing the antibody or the antigen-binding unit or the antibody-drug conjugate described above with a pharmaceutically acceptable carrier are generally known in the art.

In one or more embodiments, the present invention provides a kit comprising the antibody or the antigen-binding unit, the antibody-drug conjugate or the pharmaceutical composition comprising the same described herein and instructions guiding administration to a patient.

One or more embodiments provide an article of manufacture comprising the antibody or the antigen-binding unit, the antibody-drug conjugate or the pharmaceutically acceptable salt or solvate thereof or the pharmaceutical composition described herein;
a container; and
a package insert, instructions or label indicating that the antibody or the antigen-binding fragment, the antibody-drug conjugate or the pharmaceutical composition comprising the same described herein is for use in treating and/or preventing a disease.

### BRIEF DESCRIPTION OF THE DRA WINGS

FIG. 1 shows the mean fluorescence intensity-dose curves for the binding of anti-CLDN18.2 chimeric antibodies to KATOIII cells.
FIG. 2 shows the results of the cell binding specificity assay of the anti-CLDN18.2 humanized antibodies, wherein H239H-2a+K-6a denotes antibody H239H-2a-K-6a, and H239H-2b+K-6a denotes antibody H239H-2b-K-6a.
FIG. 3 shows the mean fluorescence intensity-dose curve for the binding of anti-CLDN18.2 antibodies to KATOIII cells, wherein CLDN18.2 Ab denotes antibody H239H-2b-K-6a-1.
FIG. 4 shows the proliferation inhibition-dose curves of ADCs against CHO-CLDN18.2 cells.
FIG. 5 shows the viable cell counts in the ADC bystander effect assay.
FIG. 6 shows the growth curves (mean ± standard error) of tumor volumes in GA0006 xenograft model mice in various groups.
FIG. 7 shows the tumor weights (mean ± standard error) in GA0006 xenograft model mice in various groups.

### DETAILED DESCRIPTION

Unless otherwise defined, scientific and technical terms used in the present invention have the meanings that are commonly understood by those skilled in the art. Generally, the nomenclature and techniques used in cell culture, molecular biology and protein purification described herein are well known and commonly used in the art. Standard techniques are used for recombinant DNA, oligonucleotide synthesis and cell culture and transformation (e.g., electroporation and lipofection). Enzymatic reactions and purification techniques are performed according to the manufacturer's instructions, or methods commonly used in the art or described herein. The aforementioned techniques and methods are generally used as described in various comprehensive and specific documents that are well known in the art and that are cited and discussed in this specification. See, e.g., Sambrook et al., Molecular Cloning: A Laboratory Manual (the 2nd edition, Cold Spring Harbor Laboratory Press, Cold Spring Harbor, N.Y. (1989)).

### Definitions

It should be noted that the term "an" entity refers to one or more of the entities. For example, "an antibody" should be interpreted as one or more antibodies. As such, the terms "a" (or "an"), "one or more" and "at least one" can be used interchangeably herein.

As used herein, the term "contain" or "comprise" means that the antibodies, compositions, methods or the like comprise the recited elements, such as components or steps and do not exclude the others. "Consisting essentially of... " means that the antibodies, compositions, methods or the like exclude other elements that have a fundamental impact on the characteristics of the combination, but do not exclude elements that do not substantially affect the characteristics of the antibodies, compositions, methods or the like. "Consisting of..." means that elements not specifically listed are excluded.

The term "antibody" as used herein refers to immunoglobulin (Ig) molecules and immunologically active portions of immunoglobulin molecules, i.e., molecules that contain an antigen-binding site that specifically binds to (immunoreacts with) an antigen. Antibodies include, but are not limited to, monoclonal antibodies, chimeric antibodies, dAbs (domain antibodies), single-chain antibodies, Fab, Fab- and F(ab')₂ fragments, Fv and Fab expression libraries.

The term "antibody" includes a wide variety of polypeptides that can be biochemically distinguished.

The antibodies, antigen-binding units or derivatives disclosed herein include, but are not limited to, polyclonal antibodies, monoclonal antibodies, multispecific antibodies, fully human antibodies, humanized antibodies, primatized antibodies, chimeric antibodies, single-chain antibodies, epitope-binding fragments (e.g., Fab, Fab' and F(ab')₂), and single-chain Fv (scFv).

The term "monoclonal antibody" (mAb) refers to a population of antibody molecules that contain only one molecular species of the antibody molecules consisting of a unique light chain gene product and a unique heavy chain gene product. In particular, the complementarity determining regions (CDRs) of the monoclonal antibody are identical in all the molecules of the population. MAbs contain an antigen-binding site capable of immunoreacting with a particular epitope of an antigen.

The term "single-chain antibody" (scFv) refers to an antibody in which its heavy chain variable region (VH) and light chain variable region (VL) are linked by a linker of 15-20 amino acids. The linker may be enriched with glycine to improve flexibility, and enriched with serine or threonine to improve solubility, and may link the N terminus of VH and the C terminus of VL, or vice versa. Although the protein has the constant region removed and the linker introduced, it retains the specificity of the original immunoglobulin. ScFv molecules are generally known in the art and are described, for example, in U.S. patent No. 5,892,019.

Those skilled in the art will appreciate that the classes of heavy chains include gamma, mu, alpha, delta, or epsilon (γ, µ, α, δ, or ε), and some subclasses (e.g., γ1-γ4). The nature of this chain determines the "type" of the antibody as IgG, IgM, IgA, IgG or IgE. Immunoglobulin subclasses (isotypes), such as IgG1, IgG2, IgG3, IgG4, IgG5, etc., have been well characterized and the functional specificity imparted is also known. All types of immunoglobulins are within the scope of the present invention. In one or more embodiments, the type of immunoglobulin molecule is IgG. Two heavy chains and two light chains are connected in a "Y" configuration through disulfide bonds, wherein the light chain starts at the opening of "Y" configuration and extends through the variable region to surround the heavy chain.

Light chains can be classified into kappa (κ) or lambda (λ). Each heavy chain may bind to a κ or λ light chain. In general, when an immunoglobulin is produced by a hybridoma, a B cell or a genetically engineered host cell, the light and heavy chains are connected by covalent bonds, and the "tail" portions of the two heavy chains are connected by covalent disulfide bonds or non-covalent bonds. In the heavy chains, the amino acid sequence extends from the N terminus at the forked end of the Y configuration to the C terminus at the bottom of each chain. The immunoglobulin κ light chain variable region is V_{κ}; and the immunoglobulin λ light chain variable region is V_{λ}.

The light chain variable region (VL) and the heavy chain variable region (VH) of the antibody determine the antigen recognition and specificity. The light chain constant region (CL) and the heavy chain constant region (CH) impart important biological properties such as secretion, transplacental movement, Fc receptor binding, complement fixation, etc. By convention, the numbering of amino acids in the constant regions increases as they become further away from the antigen-binding site of the antibody or amino terminus. The N-terminal portion is the variable region, and the C-terminal portion is the constant region; the CH3 and CL domains actually comprise the carboxyl termini of the heavy chain and light chain, respectively.

In naturally occurring antibodies, the six "complementarity determining regions" or "CDRs" present in each antigen-binding domain are short, non-contiguous, antigen-specific binding amino acid sequences that form the antigen-binding domain, assuming that the antibody is present in its three-dimensional configuration in an aqueous environment. The remaining amino acids in the antigen-binding domain, referred to as the "framework" region ("FR"), exhibit little intermolecular variability. Most of the framework regions adopt a β-sheet conformation, with the CDRs forming a loop structure connected to, or in some cases forming part of the β-sheet structure. Thus, the framework regions position the CDRs in a correct orientation by interchain non-covalent interactions through forming a scaffold. The antigen-binding domain with the specifically positioned CDRs forms a surface complementary to an epitope on the antigen that facilitates non-covalent binding of the antibody to its antigenic epitope. Generally, in an antibody molecule, the heavy and light chains each have three CDRs, referred to as VH CDR1, VH CDR2, VH CDR3, VL CDR1, VL CDR2, and VL CDR3, respectively. In positional order, the heavy chain variable region typically comprises VH FR1, VH CDR1, VH FR2, VH CDR2, VH FR3, VH CDR3, and VH FR4, and the light chain variable region comprises VL FR1, VL CDR1, VL FR2, VL CDR2, VL FR3, VL CDR3, and VL FR4. For a given heavy or light chain variable region, amino acids in the CDRs and the framework regions may be identified by those of ordinary skill in the art according to known methods (see, Kabat, E., et al., U.S. Department of Health and Human Services, Sequences of Proteins of Immunological Interest, (1983) and Chothia and Lesk, J. Mol. Biol., 196: 901-917 (1987)).

The framework and CDR regions of a humanized antibody need not correspond precisely to the parental sequences, e.g., the donor antibody CDR or the consensus framework may be mutagenized by substitution, insertion and/or deletion of at least one amino acid residue so that the CDR or framework residue at that site does not correspond to either the donor antibody or the consensus framework. Usually, at least 80%, at least 85%, at least 90% or at least 95% of the humanized antibody residues will correspond to those of the parental FR and CDR sequences. As used herein, the term "consensus framework" refers to the framework region in the consensus immunoglobulin sequence. As used herein, the term "consensus immunoglobulin sequence" refers to the sequence formed from the most frequently occurring amino acids (or nucleotides) in a family of related immunoglobulin sequences (See e.g., Winnaker, From Genes to Clones (Verlagsgesellschaft, Weinheim, Germany 1987)). In a family of immunoglobulins, each position in the consensus sequence is occupied by the amino acid occurring most frequently at that position in the family. If two amino acids occur equally frequently, either can be included in the consensus sequence.

Where the terms used and/or accepted in the art have two or more definitions, the definitions of the terms used herein include all of these meanings unless explicitly indicated as opposite. One specific example is the use of the term "complementarity determining regions" ("CDRs") to describe non-contiguous antigen-binding sites found within the variable regions of heavy and light chain polypeptides. This specific region is described in Kabat et al., U.S. Dept. of Health and Human Services, Sequences of Proteins of Immunological Interest (1983) and Chothia et al., J. Mol. Biol. 196:901-917 (1987), which are incorporated herein by reference in their entirety.

Kabat et al. also define a numbering scheme applicable to the variable region sequence of any antibody. One of ordinary skills in the art can apply the "Kabat numbering" scheme to any variable region sequence without depending on other experimental data beyond the sequence itself. "Kabat numbering" refers to the numbering system proposed by Kabat et al., U.S. Dept. of Health and Human Services in Sequence of Proteins of Immunological Interest (1983). EU or Chothia numbering scheme can also be applicable to the antibody.

The antibodies disclosed herein may be derived from any animal, including fish, birds and mammals. Preferably, the antibody is derived from a human being, a mouse, a donkey, a rabbit, a goat, a camel, a llama, a horse, or a chicken source. In another embodiment, the variable region may be derived from a condricthoid source (e.g., from a shark).

The "heavy chain constant region" comprises at least one of a CH1 domain, a hinge (e.g., upper, middle, and/or lower hinge region) domain, a CH2 domain, a CH3 domain, or a variant or a fragment. The heavy chain constant regions of the antibody may be derived from different immunoglobulin molecules. For example, the heavy chain constant regions of the antibody may comprise a CH1 domain derived from an IgG1 molecule and a hinge region derived from an IgG3 molecule. In another embodiment, the heavy chain constant region may comprise a hinge region derived partially from an IgG1 molecule and partially from an IgG3 molecule. In another embodiment, a portion of the heavy chain may comprise a chimeric hinge region derived partially from an IgG1 molecule and partially from an IgG4 molecule.

"Light chain constant region" includes a part of amino acid sequence from the light chain of an antibody. Preferably, the light chain constant region comprises at least one of a constant κ domain or a constant λ domain. "Light chain-heavy chain pair" refers to a collection of light and heavy chains that can form dimers through disulfide bonds between the CL domain of the light chain and the CH1 domain of the heavy chain.

"Disulfide bond" refers to a covalent bond formed between two sulfur atoms. The thiol group of cysteine can form a disulfide bond or a bridge with a second thiol group. In most naturally occurring IgG molecules, the CH1 and CL regions are linked by a disulfide bond.

"Chimeric antibody" refers to any antibody in which the variable region of the antibody is obtained or derived from a first species, and the constant region thereof (which may be intact, partial or modified) is derived from a second species. In certain embodiments, the variable region is derived from a non-human source (e.g., mouse or primate) and the constant region is derived from a human source.

The term "epitope" as used herein includes any protein determining region that can specifically bind to an immunoglobulin or a fragment thereof or a T cell receptor. The epitope determinant generally consists of chemically active surface groups of molecules (such as amino acids or sugar side chains), and generally has particular three-dimensional structural properties and specific charge properties.

As used herein, the term "specific bind to" or "immune response" or "against" refers to a non-covalent interaction between an immunoglobulin molecule and one or more antigenic determinants of its target antigen. The strength or affinity of an immunological binding interaction can be expressed in terms of the equilibrium dissociation constant (KD) of the interaction, where a smaller KD represents a greater affinity. The immunological binding properties of the selected polypeptides may be quantified using methods well known in the art. One such method requires the measurement of the rates of antigen-binding site/antigen complex formation and dissociation, where those rates depend on the concentration of the complex partners, the affinity of the interaction and geometric parameters that affect the rates equally in both directions. Thus, both "association rate constant" (kₒₙ) and "dissociation rate constant" (k_{off}) can be determined by calculating the concentration and the actual association and dissociation rates (see Malmqvist, M.,Nature 361: 186-87 (1993)). The ratio k_{off}/kₒₙ is capable of eliminating all the parameters that are independent of affinity and is equal to the equilibrium dissociation constant KD (generally see Davies et al., (1990) Annual Rev Biochem 59:439-473). Specific binding can be measured by radioligand binding assays, surface plasmon resonance (SPR), flow cytometry binding assay or similar assays known to those skilled in the art.

The term "isolated" as used herein with respect to cells, nucleic acids, polypeptides and the like, e.g., "isolated" DNA, RNA or polypeptides, refers to molecules that are separated from one or more other components, e.g., DNA or RNA, in the natural environment of the cell. The term "isolated" as used herein also refers to nucleic acids or peptides that are substantially free of cellular materials, viral materials or cell media when produced by recombinant DNA techniques, or of chemical precursors or other chemicals when chemically synthesized. In addition, "isolated nucleic acid" is intended to include nucleic acid fragments that do not and will not occur in nature. The term "isolated" is also used herein to refer to cells or polypeptides that are separated from other cellular proteins or tissues. Isolated polypeptides are intended to include both purified and recombinant polypeptides. Isolated polypeptides and the like are usually prepared by at least one purification step. In one or more embodiments, the purity of the isolated nucleic acids, polypeptides and the like is at least about 50%, about 60%, about 70%, about 80%, about 90%, about 95%, about 99%, or a range between any two of these values (inclusive) or any value therein.

The term "encoding" as applied to a polynucleotide refers to a polynucleotide known to "encode" a polypeptide. When in its native state or manipulated by methods well known to those skilled in the art, the polynucleotide can be transcribed and/or translated to produce the polypeptide and/or a fragment thereof.

The term "recombinant", with regard to a polypeptide or polynucleotide, is intended to refer to a polypeptide or polynucleotide that does not occur in nature, and non-limiting examples can be combined to produce a polynucleotide or polypeptide that does not normally occur.

"Amino acid" refers to an organic compound containing both an amino group and a carboxyl group, such as an α-amino acid that can be encoded by a nucleic acid, either directly or in the form of a precursor. A single amino acid is encoded by a nucleic acid consisting of three nucleotides (so-called codon or base triplet). Each amino acid is encoded by at least one codon. The encoding of the same amino acid by different codons is known as "codon degeneracy". Amino acids include natural amino acids and non-natural amino acids.

As used herein, the twenty conventional amino acids and their abbreviations follow conventional usage. See Immunology-A Synthesis (second edition, Eds. E. S. Golub and D. R. Gren, Sinauer Associates, Sunderland, Mass. (1991)). Stereoisomers (e.g., D-amino acids) of the twenty conventional amino acids, unnatural amino acids (such as α- or α-disubstituted amino acids), N-alkyl amino acids, and other unconventional amino acids can also be suitable components for the polypeptides of the present disclosure. Examples of unconventional amino acids include: citrulline (Cit), 4-hydroxyproline, γ-carboxyglutamate, ε-N,N,N-trimethyllysine, ε-N-acetyllysine, O-phosphoserine, N-acetylserine, N-formylmethionine, 3-methylhistidine, 5-hydroxylysine, σ-N-methylarginine and other similar amino acids and imino acids (e.g., 4-hydroxyproline). In the polypeptide notation used herein, the left-hand direction is the amino-terminal direction and the right-hand direction is the carboxy-terminal direction, in accordance with standard usage and convention. The conventional (natural) amino acids include alanine (three-letter symbol: Ala, one-letter symbol: A), arginine (Arg, R), asparagine (Asn, N), aspartic acid (Asp, D), cysteine (Cys, C), glutamine (Gln, Q), glutamic acid (Glu, E), glycine (Gly, G), histidine (His, H), isoleucine (Ile, I), leucine (Leu, L), lysine (Lys, K), methionine (Met, M), phenylalanine (Phe, F), proline (Pro, P), serine (Ser, S), threonine (Thr, T), tryptophan (Trp, W), tyrosine (Tyr, Y), and valine (Val, V).

The term "polypeptide" is intended to encompass both the singular form "polypeptide" and the plural form "polypeptides", and refers to a molecule consisting of amino acid monomers linearly linked by amide bonds (also known as peptide bonds). The term "polypeptide" refers to any single chain or multiple chains of two or more amino acids and does not refer to a specific length of the product. Thus, included within the definition of "polypeptide" are peptides, dipeptides, tripeptides, oligopeptides, "proteins", "amino acid chains" or any other term used to refer to chains of two or more amino acids, and the term "polypeptide" may be used in place of, or interchangeably with, any of the above terms. The term "polypeptide" is also intended to refer to a product of post-expression polypeptide modification, including but not limited to glycosylation, acetylation, phosphorylation, amidation, derivatization by known protecting/blocking groups, proteolytic cleavage, or non-naturally occurring amino acid modification. The polypeptide may be derived from a natural biological source or produced by recombinant techniques, but it is not necessarily translated from a specified nucleic acid sequence. It may be produced in any manner, including chemical synthesis.

It will be appreciated by those skilled in the art that when an amino acid or polypeptide is used as a component of a molecule (e.g., an antibody or an ADC), the amino acid or polypeptide refers to the amino acid residue or polypeptide residue (whether or not explicitly indicated), i.e., the remainder after some of groups of the amino acid or polypeptide (e.g., one hydrogen atom of an amino group and/or hydroxy group of a carboxyl group) are lost due to the formation of a covalent bond (e.g., an amide bond) with other portions of the molecule when the amino acid or polypeptide binds to other portions of the molecule.

The term "substantially identical" when applied to polypeptides means that two peptide sequences, when optimally aligned, such as by the program GAP or BESTFIT using default GAP weights, share at least 80% sequence identity, preferably at least 90% sequence identity, more preferably at least 95% sequence identity, and most preferably at least 99% sequence identity.

A polynucleotide consists of a specific sequence of four bases: adenine (A), cytosine (C), guanine (G) and thymine (T)/uracil (U, instead of thymine when the polynucleotide is RNA). A "polynucleotide sequence" can be a letter representation of a polynucleotide molecule. The letter representation can be input into a database in a computer with a central processing unit and used for bioinformatics applications, such as functional genomics and homology searches.

The terms "polynucleotide" and "oligonucleotide" are used interchangeably to refer to a polymeric form of nucleotides of any length, whether deoxyribonucleotides or ribonucleotides or analogs thereof. The polynucleotide may have any three-dimensional structure and may perform any function, known or unknown. The following are non-limiting examples of polynucleotides: genes or gene fragments (such as probes, primers, EST or SAGE tags), exons, introns, messenger RNA (mRNA), transfer RNA, ribosomal RNA, ribozymes, cDNA, dsRNA, siRNA, miRNA, recombinant polynucleotides, branched polynucleotides, plasmids, vectors, DNA, RNA, and nucleic acid probes and primers. Polynucleotides can include modified nucleotides, such as methylated nucleotides and nucleotide analogs. If present, structural modifications to the nucleotide can be made before or after the assembly of the polynucleotide. The sequence of nucleotides can be interrupted by non-nucleotide components. The polynucleotide may be further modified after polymerization, such as by conjugation with a labeling component. This term also refers to double-stranded and single-stranded molecules. Unless otherwise stated or required, examples of any polynucleotide disclosed herein include a double-stranded form and each of the two complementary single-stranded forms known or predicted to constitute the double-stranded form.

A polynucleotide or a polynucleotide sequence (or a polypeptide or an antibody sequence) having a certain percentage (e.g., 90%, 95%, 98% or 99%) of "identity or sequence identity" to another sequence refers to that when the sequences are aligned, the percentage of bases (or amino acids) in the compared sequences are the same. This alignment and identity percentage or sequence identity can be determined using visual inspection or software programs known in the art, such as the software programs described in Ausubel et al. eds., (2007), Current Protocols in Molecular Biology. Preferably, the alignment is performed using default parameters. One alignment program is BLAST using default parameters, such as BLASTN and BLASTP, both using the following default parameters: Genetic code = standard; filter = none; strand = both; cutoff = 60; expect = 10; Matrix = BLOSUM62; Descriptions = 50 sequences; sort by = HIGH SCORE; Databases = non-redundant; GenBank + EMBL + DDBJ + PDB + GenBank CDS translations + SwissProtein + SPupdate + PIR. Biologically equivalent polynucleotides are polynucleotides having the above-specified percentage identity and encoding polypeptides having identical or similar biological activity.

Minor variations in the amino acid sequences of antibodies or immunoglobulin molecules are contemplated by the present disclosure, provided that the identity of the amino acid sequences is maintained to be at least 90%, such as at least 92%, 95%, 98% or 99%. In some embodiments, the variations are conservative amino acid substitutions. Conservative amino acid substitutions are those that occur within a family of amino acids that are related in their side chains. Genetically encoded amino acids are generally classified into the following categories: (1) acidic amino acids: aspartate, and glutamate; (2) basic amino acids: lysine, arginine, and histidine; (3) non-polar amino acids: alanine, valine, leucine, isoleucine, proline, phenylalanine, methionine, and tryptophan; and (4) uncharged polar amino acids: glycine, asparagine, glutamine, cysteine, serine, threonine, and tyrosine. Amino acids of the other families include (i) serine and threonine of the aliphatic-hydroxy family; (ii) asparagine and glutamine of the amide-containing family; (iii) alanine, valine, leucine, and isoleucine of the aliphatic family; and (iv) phenylalanine, tryptophan, and tyrosine of the aromatic family. In some embodiments, conservative amino acid substitution groups are valine-leucine-isoleucine, phenylalanine-tyrosine, lysine-arginine, alanine-valine, glutamic acid-aspartic acid, and asparagine-glutamine. For example, it is reasonable to expect that the single replacement of leucine with isoleucine or valine, aspartate with glutamate, threonine with serine, or the similar replacement of an amino acid with a structurally related amino acid, will not have a major effect on the binding or properties of the resulting molecule, particularly when the replacement does not involve an amino acid within a binding site. Whether an amino acid change results in a functional peptide can be readily determined by measuring the specific activity of the polypeptide derivative. The measurement is described in detail herein. Fragments or analogs of antibodies or immunoglobulin molecules can be readily prepared by those of ordinary skill in the art.

In some embodiments, the amino acid substitutions have the following effects: (1) reducing susceptibility to proteolysis, (2) reducing susceptibility to oxidation, (3) altering the binding affinity for formation of protein complexes, (4) altering binding affinity, and (5) conferring or improving other physicochemical or functional properties of such analogs. Analogs can include various mutant proteins with sequences different from those of the naturally occurring peptide sequences. For example, single or multiple amino acid substitutions (preferably conservative amino acid substitutions) may be made in the naturally occurring sequence (preferably in a portion of the polypeptide outside the domains that form intermolecular contacts). A conservative amino acid substitution should not significantly alter the structural characteristics of the parent sequence (e.g., an amino acid for the replacement should not tend to disrupt a helix that occurs in the parent sequence, or disrupt other types of secondary structures that characterize the parent sequence). Examples of secondary and tertiary structures of artificially recognized polypeptides are described in Proteins: Structures and Molecular Principles (Ed. Creighton, W. H. Freeman and Company, New York (1984)); Introduction to Protein Structure (Eds. C. Branden and J. Tooze, Garland Publishing, New York, N.Y. (1991)); and Thornton et al., Nature 354:105 (1991).

The number of amino acids of conservative amino acid substitutions of VL or VH may be about 1, about 2, about 3, about 4, about 5, about 6, about 8, about 9, about 10, about 11, about 13, about 14, about 15, or a range between any two of these values (inclusive) or any value therein. The number of amino acids of conservative amino acid substitutions of a heavy chain constant region, a light chain constant region, a heavy chain or a light chain may be about 1, about 2, about 3, about 4, about 5, about 6, about 8, about 9, about 10, about 11, about 13, about 14, about 15, about 18, about 19, about 22, about 24, about 25, about 29, about 31, about 35, about 38, about 41, about 45 conservative amino acid substitutions, or a range between any two of these values (inclusive) or any value therein.

The term "agent" as used herein means a chemical compound, a mixture of chemical compounds, a biological macromolecule, or an extract made from biomaterial.

As used herein, the term "label" or "labeled" refers to incorporation of a detectable marker, e.g., by incorporation of a radiolabeled amino acid or attachment to a polypeptide of biotinyl moieties that can be detected by marked avidin (e.g., streptavidin containing a fluorescent marker or having enzymatic activity that can be detected by optical or calorimetric methods). In certain instances, the label or labels may also be therapeutic. Various methods for labeling polypeptides and glycoproteins are known in the art and can be used. Examples of labels for polypeptides include, but are not limited to: radioisotopes or radionuclides (e.g., ³H, ¹⁴C, ¹⁵N, ³⁵S, ⁹⁰Y, ⁹⁹Tc, ¹¹¹In, ¹²⁵I and ¹³¹I), fluorescent labels (e.g., FITC, rhodamine and lanthanide phosphor), enzymatic labels (e.g., horseradish peroxidase, β-galactosidase, luciferase and alkaline phosphatase), chemiluminescent labels, biotinyl groups and predetermined polypeptide epitopes recognized by secondary reporter genes (e.g., leucine zipper pair sequences, secondary antibody-binding sites, metal binding domains and epitope tags). In some embodiments, the labels are connected by spacer arms of various lengths to reduce potential steric hindrance. The term "medicament" or "drug" refers to a compound or composition that is capable of inducing the desired therapeutic effect when properly administered to a patient.

"About" refers to a general error range for corresponding values as readily understood by those skilled in the relevant art. In some embodiments, "about" mentioned herein refers to the values described and ranges thereof of ±10%, ±5% or ±1%.

"EC₅₀", i.e., concentration for 50% of maximal effect, refers to a concentration that causes 50% of maximal effect.

"IC₅₀" represents 50% inhibitory concentration, i.e., a concentration of drug or inhibitor required to inhibit half of a given biological process.

"Treatment" refers to both therapeutic treatment and prophylactic or preventative measures, the purpose of which is to prevent, slow down, ameliorate, or halt undesirable physiological changes or disorders, such as the progression of a disease, including, but not limited to, alleviation of symptoms, diminishment of extent of disease, stabilized (i.e., not worsening) state of disease, delay or slowing of disease progression, amelioration, palliation, alleviation, or elimination (whether partial or total) of state of disease, prolongation of life expectancy as compared with that in the absence of treatment, and the like, as either detectable or undetectable. Patients in need of treatment include patients already with a condition or disorder, patients susceptible to a condition or disorder, or patients in need of prevention of the condition or disorder, or patients who may or are expected to benefit from administration of the antibody or the pharmaceutical composition disclosed herein for detection, diagnostic procedures, and/or treatment.

The term "cancer" means or is intended to describe the physiological state of a mammal, which is typically characterized by uncontrolled cell growth. Examples of cancers include, but are not limited to, carcinoma, lymphoma, blastoma, sarcoma or leukemia. More specific examples of such cancers include, but are not limited to, colorectal cancer, lung cancer, ovarian cancer, uterine cancer, endometrial cancer, salivary gland cancer, peritoneal cancer, fallopian tube cancer, pancreatic cancer, thyroid cancer, head and neck squamous cell carcinoma, nasopharyngeal cancer, laryngeal cancer, lung adenocarcinoma, lung squamous cell carcinoma, liver cancer, hepatocellular carcinoma, gastrointestinal cancer, glioblastoma, breast cancer, brain cancer, renal cancer, renal cell carcinoma, colon cancer, rectal cancer, prostate cancer, vulval cancer, testicular cancer, squamous cell carcinoma, small cell lung cancer, cervical cancer, bladder cancer, retinoblastoma, glioblastoma, mesothelioma, oral epithelioid cancer, choriocarcinoma and head and neck cancer.

The term "overexpression" or "overexpressed" interchangeably refers to a gene that is transcribed or translated at a detectably greater level, usually in certain cells, e.g., a cancer cell, in comparison to a normal cell. Overexpression may be overexpression of a protein and RNA (due to increased transcription, post transcriptional processing, translation, post translational processing, altered stability, and altered protein degradation), as well as local overexpression due to altered protein traffic patterns (increased nuclear localization), and augmented functional activity, e.g., increased enzyme hydrolysis of substrate. Overexpression may be by 5%, 10%, 20%, 30%, 50%, 60%, 70%, 80%, 90% or more in comparison to a normal cell or control cell. In some certain examples, the antibody or antigen-binding unit or the antibody-drug conjugate of the present invention is used for treating a solid tumor possibly expressing CLDN18.2.

As used herein, the term "tumor overexpressing CLDN18.2" refers to a tumor that overexpresses CLDN18.2 (including benign tumors and cancers). In some examples, CLDN18.2 expression in a tumor sample above the immunohistochemical background level (e.g., as determined by immunohistochemical staining) indicates that the tumor is a tumor overexpressing CLDN18.2. Methods for detecting the expression of CLDN18.2 in a tumor are known in the art, such as immunohistochemical assays. In some examples, the "CLDN18.2-negative cell" is a cell that lacks CLDN18.2 above the background level in a cell sample (e.g., as determined by immunohistochemical techniques).

As used herein, the term "administer" refers to the delivery of a substance (e.g., an anti-CLDN18.2 antibody or ADC) for therapeutic purposes (e.g., treating a disease associated with CLDN18.2). The mode of administration may be parenteral, enteral, and topical. Parenteral administration is typically performed by injection, including but not limited to, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, and intrasternal injection and infusion.

As used herein, the term "effective amount" or "therapeutically effective amount" refers to the amount of a drug, e.g., an antibody or ADC, that is sufficient to reduce or ameliorate the severity and/or duration of a disorder (e.g., cancer) or one or more symptoms thereof, prevent the progression of a disorder, cause regression of a disorder, prevent the recurrence, development, onset or progression of one or more symptoms associated with a disorder, detect a disorder, or enhance or improve the prophylactic or therapeutic effect of another therapy (e.g., a prophylactic or therapeutic agent). For example, the effective amount of an antibody may inhibit tumor growth (e.g., inhibit an increase in tumor volume), decrease tumor growth (e.g., decrease tumor volume), reduce the number of cancer cells, and/or relieve to some extent one or more of the symptoms associated with the cancer. For example, the effective amount may improve disease free survival (DFS), improve overall survival (OS), or decrease likelihood of recurrence.

The terms "patient" and "subject" are used interchangeably and refer to any mammal in need of diagnosis, prognosis or treatment, including, but not limited to, humans, dogs, cats, guinea pigs, rabbits, rats, mice, horses, cattle, and the like.

As used herein, the term "in need" means that the patient has been identified as in need of a particular method or treatment. In some embodiments, identification may be made by any diagnostic mode. The patient may be in need of any methods and treatments described herein.

The term "administration" as used herein refers to administering a substance for therapeutic purposes (e.g., treating a tumor).

The term "drug for treating a tumor" as used herein refers to an agent having the functional property of inhibiting in a human the development or progression of a tumor, particularly a malignant (cancerous) lesion, such as cancer, sarcoma, lymphoma or leukemia. Inhibition of metastasis is a property of antineoplastic drugs in many cases.

The term "pharmaceutically acceptable carrier" refers generally to any type of non-toxic solid, semi-solid, or liquid filler, diluent, encapsulating material, formulation additive, etc.

The term "carrier" refers to a diluent, an adjuvant, an excipient or a carrier that can be administered to a patient together with the active ingredient. Such pharmaceutical carriers can be sterile liquids, such as water and oils, including oils originating from petroleum, animal, plant or synthesis, such as peanut oil, soybean oil, mineral oil, sesame oil, etc. Water is a preferred carrier when the pharmaceutical composition is administered intravenously. A saline aqueous solution, a glucose aqueous solution, and a glycerol solution can also be used as a liquid carrier, especially for injection. Suitable pharmaceutical excipients include starch, gelatin, malt, rice, flour, chalk, silica gel, sodium stearate, glycerol monostearate, talc, skimmed milk powder, glycerol, propylene, glycol, water, ethanol, and the like. If desired, the composition may also comprise a small amount of a wetting agent, an emulsifier, or a pH buffering agent. Antibacterials such as benzyl alcohol or methylparaben, and antioxidants such as ascorbic acid or sodium bisulfite. Such compositions may be in the form of solutions, suspensions, emulsions, tablets, pills, capsules, pulvises, sustained-release formulations and the like. The composition may be formulated as a suppository using conventional binders and carriers such as triglycerides. Oral formulations may comprise standard carriers such as mannitol, lactose, starch, magnesium stearate, sodium saccharin, cellulose and magnesium carbonate of pharmaceutical grade. Examples of suitable pharmaceutical carriers are described in Remington's Pharmaceutical Sciences by E. W. Martin, which is hereby incorporated herein by reference. Such compositions will contain a clinically effective dose of an antibody or antigen-binding unit or ADC, preferably in purified form, together with an appropriate amount of a carrier, to provide a form suitable for administration to a patient. The formulation should be suitable for the administration mode. The formulation may be encapsulated in an ampoule bottle, a disposable syringe, or a multi-dose vial made of glass or plastic.

The term "antibody-drug conjugate" or "ADC" refers to a binding protein (e.g., an antibody or antigen-binding unit) that is linked to one or more chemical drugs, which may optionally be a therapeutic agent or a cytotoxic agent. In a preferred embodiment, the ADC comprises an antibody, a drug (e.g., a cytotoxic drug), and a linker capable of attaching or conjugating the drug to the antibody. Non-limiting examples of drugs that can be included in the ADC are a mitotic inhibitor, an anti-tumor antibiotic, an immunomodulator, a vector for gene therapy, an alkylating agent, an anti-angiogenic agent, an antimetabolite, a boron-containing agent, a chemoprotective agent, a hormone, an anti-hormonal agent, a corticosteroid, a photoactive therapeutic agent, an oligonucleotide, a radionuclide agent, a topoisomerase inhibitor, a kinase inhibitor (e.g., a TEC-family kinase inhibitor and a serine/threonine kinase inhibitor) and a radiosensitizer.

The terms "antibody-drug conjugate" and "ADC" are used interchangeably. The terms "anti-CLDN18.2 antibody-drug conjugate" and "anti-CLDN18.2 ADC" are used interchangeably to refer to an ADC comprising an antibody that specifically binds to CLDN18.2, wherein the antibody is conjugated to one or more drugs. In one or more embodiments, the anti-CLDN18.2 ADC comprises an antibody conjugated to exatecan. In one or more embodiments, the anti-CLDN18.2 antibody or ADC binds to CLDN18.2 (e.g., human CLDN18.2).

The term "drug to antibody ratio" or "DAR" refers to the number of drugs (e.g., exatecan) that are attached to the antibody in the ADC. The DAR of an ADC may be in the range of 1 to 10, but a higher load (e.g., 20) is also possible depending on the number of connection sites on the antibody. The term DAR may be used when referring to the number of drugs loaded onto a single antibody, or alternatively, when referring to an average or mean DAR for a set of ADCs. In some embodiments, the value is selected from 1, 2, 3, 4, 5, 6, 7, 8, 9, or 10. In considering the mean binding number of small molecule drugs, i.e., the mean drug binding number of an antibody, or the mean drug to antibody ratio, the value is selected from about 0 to about 10, or about 2 to about 8. In some embodiments, the drug to antibody ratio is about 3 to about 6. In other embodiments, the drug to antibody ratio is about 6 to about 8, or about 7 to about 8. DAR values may be denoted herein by p. The DAR value of ADC can be measured by ultraviolet-visible absorption spectroscopy (UV-Vis), high performance liquid chromatography-hydrophobic interaction chromatography (HPLC-HIC), reversed-phase high performance liquid chromatography (RP-HPLC), liquid chromatography-mass spectrometry (LC-MS), etc. These techniques are described in Ouyang, J. Methods Mol Biol, 2013, 1045: p. 275-83.

Various substituents are defined below. In some cases, the number of carbon atoms in a substituent (e.g., alkyl, alkenyl, alkynyl, alkoxy, aminoalkoxy, aminoalkyl, aminoalkylamino, alkylamino, heterocyclyl, heterocyclylamino and aryl) is indicated by the prefix "Cx-Cy" or "Cx-y", wherein x is the minimum number and y is the maximum number of carbon atoms. Thus, for example, "C1-C6 alkyl" refers to an alkyl containing 1 to 6 carbon atoms. If a substituent is described as "substituted with... ", a hydrogen atom on a carbon or nitrogen is substituted with a non-hydrogen group. For example, a substituted alkyl substituent is an alkyl substituent in which at least one hydrogen atom on the alkyl is substituted with a non-hydrogen group. To illustrate, monofluoroalkyl is an alkyl substituted with a fluoro group, and difluoroalkyl is an alkyl substituted with two fluoro groups. It should be recognized that if there is more than one substitution on a substituent, each substitution may be identical or different (unless otherwise stated). If a substituent is described as "optionally substituted with...", the substituent may be (1) unsubstituted or (2) substituted. Possible substituents include, but are not limited to, hydroxy, C1-C6 alkyl, C2-C6 alkenyl, C2-C6 alkynyl, C1-C6 alkoxy, C1-C6 aminoalkoxy, halogen, nitro, cyano, sulfhydryl, alkylthio, amino, C1-C6 aminoalkyl, C1-C6 aminoalkylamino, C1-C6 alkyl linking to a heterocycle, C1-C6 alkylamino linking to a heterocycle, heterocyclyl, amino-substituted heterocyclyl, heterocyclylamino, carbamoyl, morpholin-1-yl, piperidin-1-yl, -(CH₂)_{q}-CH₃, -(CHRⁿ)_{q}-CH₃, C3-C8 carbocyclyl, -O-(CH₂)_{q}-CH₃, arylene-CH₃, -(CH₂)_{q}-arylene-CH₃, -arylene-(CH₂)_{q}-CH₃, -(CH₂)_{q}-(C3-C8 carbocyclyl)-CH₃, -(C3-C8 carbocyclyl)-(CH₂)_{q}-CH₃, C3-C8 heterocyclyl, -(CH₂)_{q}-(C3-C8 heterocyclyl)-CH₃, -(C3-C8 heterocyclyl)-(CH₂)_{q}-CH₃, -(CH₂)_{q}C(O)NRⁿ(CH₂)_{q}-CH₃, -(CH₂CH₂O)_{q}-CH₃, -(CH₂CH₂O)_{q}-CH₂-CH₃, -(CH₂)_{q}C(O)NRⁿ(CH₂CH₂O)_{q}-CH₃, -(CH₂)_{q}C(O)NRⁿ(CH₂CH₂O)_{q}-CH₂-CH₃, -(CH₂CH₂O)_{q}C(O)NRⁿ(CH₂CH₂O)_{q}-CH₃, -(CH₂CH₂O)_{q}C(O)NRⁿ(CH₂CH₂O)_{q}-CH₂-CH₃ or - (CH₂CH₂O)_{q}C(O)NRⁿ(CH₂)_{q}-CH₃; wherein each Rⁿ is independently H, C1-C6 alkyl, C3-C8 carbocyclyl, phenyl or benzyl, and each q is independently 1, 2, 3, 4, 5, 6, 7, 8, 9 or 10.

The "alkyl" refers to a saturated aliphatic hydrocarbyl group, and this term includes linear and branched hydrocarbyl groups. For example, C1-C20 alkyl, such as C1-C6 alkyl. C1-C20 alkyl refers to an alkyl group containing 1 to 20 carbon atoms, e.g., an alkyl group containing 1 carbon atom, 2 carbon atoms, 3 carbon atoms, 4 carbon atoms, 5 carbon atoms, 6 carbon atoms, 7 carbon atoms, 8 carbon atoms, 9 carbon atoms, 10 carbon atoms, 11 carbon atoms, 12 carbon atoms, 13 carbon atoms, 14 carbon atoms, 15 carbon atoms, 16 carbon atoms, 17 carbon atoms, 18 carbon atoms, 19 carbon atoms, or 20 carbon atoms. Non-limiting examples of alkyl include methyl, ethyl, *n*-propyl, isopropyl, *n*-butyl, isobutyl, *sec-*butyl, *tert-*butyl, *n*-pentyl, neopentyl, *n*-hexyl, and the like. The alkyl may be unsubstituted or substituted with one or more substituents including, but not limited to, alkyl, alkoxy, cyano, hydroxy, carbonyl, carboxy, aryl, heteroaryl, amino, halogen, sulfonyl, sulfinyl, phosphonyl, and the like.

The term "alkenyl", by itself or as part of another substituent, refers to an unsaturated branched, linear or cyclic alkyl having at least one carbon-carbon double bond derived by the removal of one hydrogen atom from a single carbon atom of a parent olefin. Typical alkenyl includes, but is not limited to, ethenyl; propenyl (e.g., prop-1-en-1-yl, prop-1-en-2-yl, prop-2-en-1-yl, prop-2-en-2-yl, cycloprop-1-en-1-yl); cycloprop-2-en-1-yl; butenyl (e.g., but-1-en-1-yl, but-1-en-2-yl, 2-methyl-prop-1-en-1-yl, but-2-en-1-yl, but-2-en-2-yl, but-1,3-dien-1-yl, but-1,3-dien-2-yl, cyclobut-1-en-1-yl, cyclobut-1-en-3-yl, cyclobut-1,3-dien-1-yl, etc.), and the like.

The term "alkynyl", by itself or as part of another substituent, refers to an unsaturated branched, linear or cyclic alkyl having at least one carbon-carbon triple bond derived by the removal of one hydrogen atom from a single carbon atom of a parent alkyne. Typical alkynyl includes, but is not limited to, ethynyl; propynyl (e.g., prop-1-yn-1-yl, prop-2-yn-1-yl, etc.); butynyl (e.g., but-1-yn-1-yl, but-1-yn-3-yl, but-3-yn-1-yl, etc.), and the like.

The "carbocyclyl" refers to a stable non-aromatic monocyclic or polycyclic hydrocarbyl radical consisting of carbon and hydrogen atoms only, and may comprise a fused or bridged ring system, contains 3 to 15 carbon atoms, for example, 3 to 10 (e.g., 3, 4, 5, 6, 7, 8, 9 or 10) carbon atoms. It is saturated or unsaturated, and links to the remainder of the molecule through a single bond. Monocyclic radicals include, for example, cyclopropyl, cyclobutyl, cyclopentyl, cyclohexyl, cycloheptyl and cyclooctyl. Polycyclic radicals include, for example, adamantyl, norbornyl, and decahydronaphthyl. When specifically stated in the specification, the carbocyclyl may be optionally substituted with one or more substituents independently selected from the following: alkyl, halogen, haloalkyl, cyano, nitro, oxo, aryl, aralkyl, carbocyclyl, carbocyclylalkyl, heterocyclyl, heterocyclylalkyl, heteroaryl and heteroarylalkyl.

The "aryl" refers to an all-carbon monocyclic or all-carbon fused ring having a completely conjugated π-electron system, and typically has 5-14 carbon atoms, e.g., 6, 10, 12, or 14 carbon atoms. Aryl may be unsubstituted or substituted with one or more substituents including, but not limited to, alkyl, alkoxy, cyano, hydroxy, carboxy, aryl, aralkyl, amino, halogen, sulfonyl, sulfinyl, phosphonyl. Examples of unsubstituted aryl include, but are not limited to, phenyl, naphthyl, and anthracenyl.

The "heterocyclyl" refers to a stable 3 to 18 membered aromatic or nonaromatic ring group consisting of 2 to 8 (e.g., 2, 3, 4, 5, 6, 7 or 8) carbon atoms and 1 to 6 (1, 2, 3, 4, 5 or 6) heteroatoms selected from nitrogen, oxygen and sulfur. Unless otherwise specifically stated in the specification, heterocyclyl may be a monocyclic, bicyclic, tricyclic or tetracyclic ring system, and may include fused or bridged ring systems; and the nitrogen, carbon or sulfur atoms in heterocyclyl may be optionally oxidized; the nitrogen atoms are optionally quaternized; and heterocyclyl may be partially or fully saturated. Examples of such heterocyclyl include, but are not limited to, dioxolanyl, dioxinyl, thienyl[1,3]dithianyl, decahydroisoquinolinyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidinonyl, pyrrolidinyl, pyrazolidinyl, quinuclidinyl, thiazolidinyl, 1,2,4-thiadiazol-5(4*H*)-ylidene, tetrahydrofuranyl, trioxacyclohexyl, trithianyl, triazinanyl, tetrahydropyranyl, thiomorpholinyl, thiamorpholinyl, 1-oxo-thiomorpholinyl and 1,1-dioxo-thiomorpholinyl. When specifically stated in the specification, heterocyclyl may be optionally substituted with one or more substituents selected from: alkyl, alkenyl, halogen, haloalkyl, cyano, oxo, thioxo, nitro, aryl, aralkyl, cycloalkyl, cycloalkylalkyl, optionally substituted heterocyclyl, optionally substituted heterocyclylalkyl, optionally substituted heteroaryl, and optionally substituted heteroarylalkyl.

The "alkoxy" refers to formula -O-(alkyl), wherein alkyl is the alkyl defined herein. Non-limiting examples of alkoxy are methoxy, ethoxy, *n*-propoxy, 1-methylethoxy (isopropoxy), *n*-butoxy, isobutoxy, *sec*-butoxy, and *tert*-butoxy. Alkoxy may be substituted or unsubstituted.

The "halogen" refers to fluoro (F), chloro (Cl), bromo (Br), or iodo (I).

The "amino" refers to -NH₂.

The "cyano" refers to -CN.

The "nitro" refers to -NO₂.

The "hydroxy" refers to -OH.

The "carboxyl" refers to -COOH.

The "thiol" refers to -SH.

The "carbonyl" refers to C=O.

"Stereoisomers" refer to isomeric compounds having the same linking order of atoms but different spatial arrangements of the atoms. Stereoisomers may have one or more stereocenters and each center may exist as R or S, and stereoisomers may also be cis-trans isomers. Stereoisomers of the compounds provided herein include any one of their all diastereomeric, enantiomeric and cis-trans isomeric forms, or mixtures thereof.

The pharmaceutically acceptable salt includes those derived from the compound with a variety of organic and inorganic counterions well known in the art, and salts as examples only include organic or inorganic salts such as lithium salt, sodium salt, potassium salt, calcium salt, magnesium salt, ammonium salt, isopropylamine, trimethylamine, diethylamine, triethylamine, tripropylamine, ethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purine, piperazine, piperidine, *N*-ethyl, piperidine, polyamine resin, and tetraalkylammonium salt when the molecule contains an acidic functional group; and organic or inorganic acid salts such as hydrochloride salt, hydrobromide salt, tartrate salt, mesylate salt, acetate salt, maleate salt and oxalate salt when the molecule contains a basic functional group. Other non-limiting examples of acids include sulfuric acid, nitric acid, phosphoric acid, propionic acid, glycolic acid, pyruvic acid, malonic acid, succinic acid, fumaric acid, tartaric acid, citric acid, benzoic acid, cinnamic acid, mandelic acid, methanesulfonic acid, ethanesulfonic acid, *p*-toluenesulfonic acid, salicylic acid, and the like. These salts can generally be prepared by conventional methods by reacting, for example, an appropriate acid or base with the compound. The solvate includes hydrates.

Other chemical terms herein are used according to conventional usage in the art, such as the McGraw-Hill Dictionary of Chemical Terms (Ed. Parker, S., McGraw-Hill, San Francisco (1985)).

All the publications and patents cited herein are incorporated herein by reference in their entirety for all purposes.

### Anti-CLDN18.2 antibody

In one or more embodiments, the antibody or the antigen-binding unit of the present invention can specifically bind to CLDN18.2. In one or more embodiments, the antibody or the antigen-binding unit is a murine antibody, a chimeric antibody, or a humanized antibody. In one or more embodiments, the antibody or the antigen-binding unit of the present invention has one or more of the following properties: a) specifically binding to CLDN18.2; b) high affinity; c) high ADCC activity; and d) high CDC activity.

In one or more embodiments, the antibody or the antigen-binding unit comprises an amino acid sequence having one or more modification groups. For example, the antibody or the antigen-binding unit of the present invention may comprise a flexible linker sequence, or may be modified to add a functional group (e.g., PEG, a drug, a toxin, or a tag).

The antibody or the antigen-binding unit of the present invention includes modified derivatives, i.e., modified by covalent linking of any type of molecules to the antibody or the antigen-binding unit, wherein the covalent linking does not prevent the antibody or the antigen-binding unit from binding to an epitope. The following examples are included but not by way of limitation, wherein an antibody or antigen-binding unit may be modified by glycosylation, acetylation, pegylation, phosphorylation, amidation, derivatization by known protecting/blocking groups, proteolytic cleavage, attachment to cellular ligands or other proteins, or the like. Any of a number of chemical modifications may be made by techniques in the prior art, including but not limited to specific chemical cleavage, acetylation, formylation, metabolic synthesis of tunicamycin, and the like.

In one or more embodiments, the antibody or the antigen-binding unit may be conjugated with a therapeutic agent, a prodrug, a peptide, a protein, an enzyme, a virus, a lipid, a biological response modifier, an agent, or PEG.

The antibody or the antigen-binding unit may be detectably labeled by coupling to a chemiluminescent compound. The presence of the chemiluminescent-labeled antibody or antigen-binding unit is then determined by detecting the luminescence that occurs during the chemical reaction. Examples of chemiluminescent-labeled compounds include luminol, isoluminol, aromatic acridinium esters, imidazole, acridinium salts, and oxalate esters.

### Preparation of Antibodies

In certain embodiments, the prepared antibody or the antigen-binding unit does not elicit a deleterious immune response in the animal (e.g., human) to be treated. In one or more embodiments, the antibody, the antigen-binding unit or the derivative thereof of the present invention is modified using techniques well known in the art to reduce the immunogenicity. For example, the antibody can be humanized, primatized or deimmunized, or a chimeric antibody can be prepared. Such antibodies are derived from non-human antibodies, typically murine or primate antibodies, which retain or substantially retain the antigen-binding properties of the parent antibody but are less immunogenic in humans. This can be accomplished by a variety of methods, including: (a) grafting an entire variable region of a non-human origin to a constant region of a human origin to produce a chimeric antibody; (b) grafting at least a portion of one or more non-human complementarity determining regions (CDRs) to framework regions and a constant region of a human origin, with or without retention of critical framework residues; or (c) grafting an entire variable region of a non-human origin, but "hiding" the surface residues by replacing them with portions of a human-like origin. Generally, framework residues in the human framework regions will be substituted with corresponding residues from the CDR donor antibody, such as residues that may improve the antigen binding. Such framework substitutions can be identified by methods well known in the art, for example, by modeling the interaction of the CDR and framework residues to identify framework residues that play an important role in antigen binding and by sequence alignment to identify abnormal framework residues at particular positions (see U.S. Pat. No. 5,585,089); which are incorporated herein by reference in their entireties). The antibody can be humanized by a variety of techniques well known in the art, such as CDR grafting (Pat. WO1991009967, and U.S. Pat. Nos. 5,225,539, 5,530,101 and 5,585,089), repair or surface rearrangement (Pat. Nos. EP592,106 and EP519,596), and chain rearrangement (U.S. Pat. No. 5,565,332), which are incorporated herein by reference in their entireties.

Deimmunization may also be used to reduce the immunogenicity of the antibody. In the present invention, the term "deimmunization" includes the alteration of the antibody to modify T cell epitopes (see, e.g., Pat. Nos. WO2000034317 A2). For example, a heavy chain variable region sequence and a light chain variable region sequence from the original antibody are analyzed, and a "map" of human T cell epitopes from each variable region is generated, showing the positions of the epitopes relative to the complementarity determining regions (CDRs) and other critical residues within the sequence. Individual T cell epitopes from the T cell epitope map are analyzed to identify alternative amino acid substitutions with a lower risk of altering antibody activity. A series of alternative heavy chain variable region sequences and light chain variable region sequences comprising combinations of amino acid substitutions are designed and subsequently incorporated into a series of binding polypeptides. The genes of intact heavy and light chains comprising the modified variable regions and human constant regions are cloned into an expression vector, and the plasmid is then transferred into a cell line to produce an intact antibody. The antibodies are compared in appropriate biochemical and biological experiments to identify the optimal antibody.

An scFv can be prepared by the technique for producing a single chain unit (U.S. Pat. No. 4,946,778). Single chain fusion peptides are produced by amino acid bridging the heavy and light chain fragments of the Fv region to form a single chain unit. Techniques for the assembly of functional Fv fragments in *E. coli* may also be used (Skerra et al., Science 240: 1038-1041 (1988)).

Examples of techniques that can be used to produce a single chain Fv (scFv) and an antibody include, for example, those described in U.S. Pat. Nos. 4,946,778 and 5,258,498). For certain use, including *in vivo* use of antibodies in humans and *in vitro* detection assays, a chimeric antibody, a humanized antibody or a fully human antibody may be used. Chimeric antibodies are molecules in which different portions of the antibody are derived from different animal species, such as antibodies having variable regions of a murine monoclonal antibody and constant regions of a human immunoglobulin. Methods for producing chimeric antibodies are known in the art, see U.S. Pat. Nos. 5,807,715, 4,816,567 and 4,816,397, which are incorporated herein by reference in their entireties.

In one or more embodiments, the antibody of the present invention is prepared by using hybridoma techniques. For preparing monoclonal antibodies using, for example, hybridoma methods, see, e.g., those described by Kohler and Milstein, Nature, 256:495 (1975). In the hybridoma method, a mouse, a hamster or other appropriate host animal, is typically immunized with an immunizing agent to elicit production of lymphocytes or production of antibodies that can specifically bind to the immunizing agent.

The immunizing agent will generally include a protein antigen, a fragment thereof or a fusion protein thereof. Generally, if a human cell is desired, a peripheral blood lymphocyte is used; if a cell of non-human mammalian origin is desired, a spleen lymphocyte or lymph node cell is used. In some embodiments, a spleen lymphocyte is used; the lymphocyte is then fused with an immortalized cell line using a suitable fusing agent, such as polyethylene glycol, to form a hybridoma cell (Goding, Monoclonal Antibodies: Principles and Practice, Academic Press, (1986) pp. 59-103). The immortalized cell line is generally a transformed mammalian cell, particularly a myeloma cell of rodent, bovine or human origin. Generally, rat or mouse myeloma cell lines are used. In some embodiments, a spleen lymphocyte and a mouse myeloma cell are used for fusion. The hybridoma cell may be cultured in a suitable culture medium, and in some embodiments, the culture medium contains one or more substances that inhibit the growth or survival of unfused immortalized cells. For example, if the parent cells lack hypoxanthine-guanine phosphoribosyltransferase (HGPRT or HPRT), the culture medium for the hybridoma generally contains hypoxanthine, aminopterin and thymine ("HAT medium"), which prevent the growth of HGPRT-deficient cells. In some embodiments, the binding specificity of the monoclonal antibody produced by the hybridoma cells is determined by immunoprecipitation or an *in vitro* binding assay, such as radioimmunoassay (RIA) or enzyme-linked immunosorbent assay (ELISA). Such techniques and assays are known in the art. The binding affinity of the monoclonal antibody can be determined, for example, by Scatchard analysis described by Munson and Podbard, Anal. Biochem., 107:220 (1980). Furthermore, in the therapeutic application of the monoclonal antibody, it is important to identify antibodies with high specificity and high binding affinity for the target antigen.

After the desired hybridoma cells are identified, the clones can be subcloned using dilution cloning procedures and grown by standard methods (see Goding, Monoclonal Antibodies: Principles and Practice, Academic Press, (1986) pp. 59-103). Suitable media for this purpose include, for example, Dulbecco's modified Eagle's Medium and RPMI-1640 medium.

In one or more embodiments, the monoclonal antibody secreted by the subclone can be isolated or purified by conventional techniques, such as protein A-sepharose, hydroxyapatite chromatography, gel electrophoresis, dialysis, or affinity chromatography.

The monoclonal antibody can also be prepared by recombinant DNA methods, such as those described in U.S. Pat. No. 4,816,567. DNA encoding the monoclonal antibody of the present invention can be isolated and sequenced using conventional methods (e.g., by using oligonucleotide probes that can specifically bind to genes of the heavy chain and the light chain of antibodies). DNA encoding the antibody of the present invention can also be synthesized by conventional methods based on antibody sequence design. The isolated or synthetic DNA is inserted into an expression vector, and then transfected into a host cell such as a Chinese hamster ovary (CHO) cell, a human embryonic kidney (HEK) 293 cell, a simian COS cell, a PER. NS0 cell, an SP2/0 cell, a YB2/0 cell, or a myeloma cell that does not otherwise produce immunoglobulins, thereby obtaining a synthetic monoclonal antibody in the recombinant host cell.

In one or more embodiments, the antibody or the antigen-binding unit of the present invention is prepared by hybridoma techniques: a protein formulation comprising CLDN18.2 is used to immunize a mouse; spleen lymphocytes of the immunized mouse are fused with myeloma cells; the hybridoma cells are screened through the CLDN18.2 protein; the positive hybridoma cells are subjected to dilution cloning and further subcloning; and the binding capacity of the hybridoma cell strains to the CLDN18.2 protein is identified again for producing the anti-CLDN18.2 antibody. In some embodiments, the mouse is a female Balb/c mouse aged 6-8 weeks.

The binding specificity of the antibody or the antigen-binding fragment of the present invention can be detected by an *in vitro* assay, such as co-immunoprecipitation, radioimmunoassay (RIA) or enzyme-linked immunosorbent assay (ELISA).

For certain use, including *in vivo* use of antibodies in humans and *in vitro* detection assays, in some embodiments, a chimeric antibody, a humanized antibody, or a fully human antibody may be used. Chimeric antibodies are molecules in which different portions of the antibody are derived from different animal species, such as antibodies having variable regions of a murine monoclonal antibody and constant regions of a human immunoglobulin. Methods for producing chimeric antibodies are known in the art, see U.S. Patent Nos. 5,807,715, 4,816,567 and 4,816,397, which are incorporated herein by reference in their entireties.

Humanized antibodies can also be produced by transgenic mice that cannot express functional endogenous immunoglobulins but express human immunoglobulin genes. For example, human heavy and light chain immunoglobulin gene complexes can be introduced randomly or by homologous recombination into mouse embryonic stem cells. Alternatively, in addition to the human heavy and light chain genes, human variable, constant and diversity regions can be introduced into mouse embryonic stem cells. Mouse heavy and light chain immunoglobulin genes can be disabled by homologous recombination through introducing human immunoglobulin loci. In particular, homozygous deletion of the JH region can prevent the production of endogenous antibodies. The modified embryonic stem cells are expanded and microinjected into blastocysts to produce chimeric mice. The chimeric mice are then bred to produce homozygous progeny that express humanized antibodies. The transgenic mice are immunized by conventional methods with selected antigens, e.g., all or part of the desired polypeptide targets. Antigen-targeting monoclonal antibodies can be obtained from the immunized transgenic mice using conventional hybridoma techniques. The human immunoglobulin transgenes carried by the transgenic mice rearrange during B cell differentiation, resulting in class switching and somatic mutation. Accordingly, using this technique, IgG, IgA, IgM, and IgE antibodies that are useful for therapy can be produced.

In other embodiments, DNA encoding the desired monoclonal antibody can be readily isolated and sequenced using conventional methods (e.g., using oligonucleotide probes that can specifically bind to genes encoding the heavy and light chains of a murine antibody). Once isolated, the DNA can be inserted into an expression vector and then transfected into prokaryotic or eukaryotic host cells such as *E. coli* cells, simian COS cells, Chinese hamster ovary (CHO) cells, or myeloma cells that do not otherwise produce immunoglobulins. The isolated DNA (which may be synthetic as described herein) can be used to prepare sequences of the constant and variable regions of an antibody, as described in U.S. Pat. No. 5,658,570, which is incorporated herein by reference in its entirety. This method extracts RNA from the selected cells and converts it to cDNA, followed by amplification by PCR using Ig-specific primers. Suitable probes for this purpose are also described in U.S. Pat. No. 5,658,570.

In addition, using conventional recombinant DNA techniques, one or more CDRs of the antibody or the antigen-binding unit of the present invention can be inserted into framework regions, e.g., into human framework regions to construct a humanized non-fully human antibody. The framework regions may be naturally occurring or consensus framework regions, preferably human framework regions (see Chothia et al., J. Mol. Biol. 278:457-479 (1998), in which a range of human framework regions are listed). Some polynucleotides may encode antibodies produced by the combination of framework regions and CDRs that specifically bind to at least one epitope of a target antigen. One or more amino acid substitutions are made within the framework regions, and amino acid substitutions capable of improving the binding of the antibody to the antigen thereof may be selected. Additionally, substitution or deletion of one or more cysteine residues in the variable regions involved in interchain disulfide bond formation can be made in this manner, thereby producing an antibody molecule lacking one or more interchain disulfide bonds. Other alterations to the polynucleotides made within the technology scope of the art are also encompassed by the present invention.

In addition, another efficient method for producing recombinant antibodies, which in particular is capable of producing primate antibodies comprising monkey variable region and human constant region sequences, is disclosed in Newman, Biotechnology 10:1455-1460 (1992), which is incorporated herein by reference in its entirety. Furthermore, this technique is also described in commonly assigned U.S. Pat. Nos. 5,658,570, 5,693,780 and 5,756,096, each of which is incorporated herein by reference in its entirety.

Antibodies can be prepared by using conventional recombinant DNA techniques. Vectors and cell lines for antibody production can be selected, constructed and cultured using techniques well known to those skilled in the art. These techniques are described in various laboratory manuals and main publications, such as Recombinant DNA Technology for Production of Protein Therapeutics in Cultured Mammalian Cells, D. L. Hacker, F. M. Wurm, Reference Module in Life Sciences, 2017, which is incorporated by reference in its entirety, including the supplements.

In one or more embodiments, the DNA encoding the antibody can be designed and synthesized according to the antibody amino acid sequence described herein by conventional methods, inserted into an expression vector, and transfected into a host cell. The transfected host cell is then cultured in a medium to produce the monoclonal antibody. In some embodiments, the vector expressing the antibody comprises at least one promoter element, an antibody coding sequence, a transcription termination signal, and a polyA tail. Other elements include enhancers, Kozak sequences, and donor and recipient sites flanking the inserted sequence for RNA splicing. Efficient transcription can be obtained by early and late promoters of SV40, long terminal repeats from retroviruses such as RSV, HTLV1 and HIVI, and early promoters of cytomegalovirus, and promoters from other cells such as actin promoter can also be used. Suitable expression vectors may include pIRES1neo, pRetro-Off, pRetro-On, PLXSN, pLNCX, pcDNA3.1 (+/-), pcDNA/Zeo (+/-), pcDNA3.1/Hygro(+/-), PSVL, PMSG, pRSVcat, pSV2dhfr, pBC12MI, pCS2, and the like. Commonly used mammalian host cells include HEK293 cells, Cos1 cells, Cos7 cells, CV1 cells, murine L cells, CHO cells, and the like.

In one or more embodiments, the inserted gene fragment should comprise a screening label, common ones of which include screening genes such as dihydrofolate reductase, glutamine synthetase, neomycin resistance and hygromycin resistance, to facilitate the screening and separation of cells that have been successfully transfected. The constructed plasmid is transfected to a host cell without the above genes, and the successfully transfected cells are cultured in a large quantity in a selective culture medium to produce the desired target protein.

In addition, mutations can be introduced in the nucleotide sequence encoding the antibody of the present invention using standard techniques known to those skilled in the art, including but not limited to site-directed mutations resulting in amino acid substitutions and PCR-mediated mutations. The variant (including derivative) encodes a substitution of less than 50 amino acids, a substitution of less than 40 amino acids, a substitution of less than 30 amino acids, a substitution of less than 25 amino acids, a substitution of less than 20 amino acids, a substitution of less than 15 amino acids, a substitution of less than 10 amino acids, a substitution of less than 5 amino acids, a substitution of less than 4 amino acids, a substitution of less than 3 amino acids or a substitution of less than 2 amino acids relative to the original heavy chain variable region and light chain variable region. Alternatively, mutations can be introduced randomly along all or part of the coding sequence, for example by saturation mutagenesis, and the resulting mutants can be screened for the biological activity to identify mutants that retain the activity.

### Antibody-drug conjugate

The antibody or antigen-binding unit of the present invention can be conjugated to a drug to form an anti-CLDN18.2 antibody-drug conjugate (anti-CLDN18.2 ADC). Antibody-drug conjugates (ADCs) can increase the therapeutic efficacy of antibodies in treating diseases (e.g., cancer) due to their ability to selectively deliver one or more drugs to a target tissue (e.g., a cancer or tumor expressing CLDN18.2). In one or more embodiments, the antibody-drug conjugate (ADC) of the present invention comprises the anti-CLDN18.2 antibody or antigen-binding unit described herein and at least one drug (e.g., exatecan).

The antigen-binding fragment or antigen-binding unit of the anti-CLDN18.2 antibody of the present invention may be conjugated to the drug described herein. Thus, in one or more embodiments, the antigen-binding fragment or antigen-binding unit of the anti-CLDN18.2 antibody described herein is conjugated to a drug via a linker to form the anti-CLDN18.2 ADC.

The anti-CLDN18.2 ADC of the present invention comprises an antibody or antigen-binding unit that is linked to one or more drugs and specifically binds to CLDN18.2 (e.g., human CLDN18.2). The specificity of the ADC may be determined by the specificity of the antibody (e.g., anti-CLDN18.2 antibody) or the antigen-binding fragment. In one or more embodiments, the anti-CLDN18.2 antibody is linked to one or more drugs (e.g., DNA topoisomerase inhibitors) that are delivered to cells expressing CLDN18.2, particularly cancer cells expressing CLDN18.2. In one or more embodiments, the anti-CLDN18.2 antibody-drug conjugate comprises an anti-CLDN18.2 antibody conjugated to a drug (e.g., exatecan) via a linker. The anti-CLDN18.2 antibody described herein provides the ADC with the ability to bind to CLDN18.2, such that the drug attached to the antibody can be delivered to cells expressing CLDN18.2, particularly cancer cells expressing CLDN18.2.

In one or more embodiments, the ADC has a structure of Formula I or a stereoisomer thereof, or a pharmaceutically acceptable salt or solvate thereof, 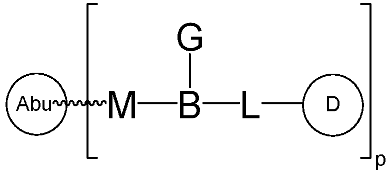 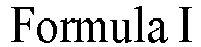 wherein Abu, M, B, G, L, D, and p are as defined herein.

In one or more embodiments, Abu is antibody H239H-2b-K-6a-1, H239H-2b-K-6a-2, or H239H-2b-K-6a-3.

In one or more embodiments, the ADC is ADC1, ADC2, ADC3, ADC4, ADC5, or ADC6, or a pharmaceutically acceptable salt or solvate thereof.

In one or more embodiments, the antibody-drug conjugate described herein has a significant bystander effect.

### Synthesis Method

The present invention further provides preparation method for intermediates and antibody-drug conjugates. The intermediates and the antibody-drug conjugates of the present invention can be prepared by using known formulations and methods. In one or more embodiments, the methods for preparing intermediate Compound 1-7 are described below. 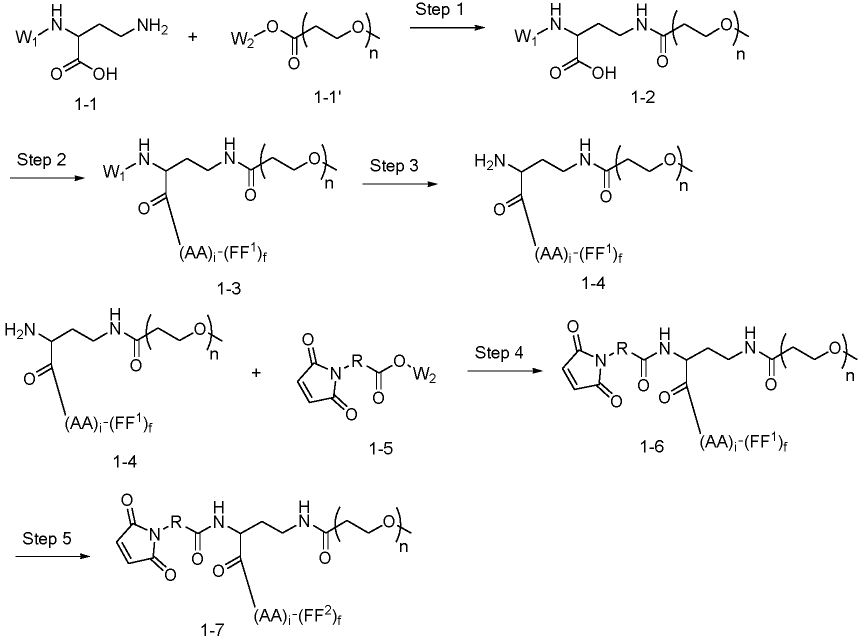
Step 1: reacting a compound of general Formula 1-1 with a compound of general Formula 1-1' under a basic condition to obtain a compound of general Formula 1-2;
Step 2: reacting the compound of general Formula 1-2 with general Formula (AA)ᵢ-(FF¹)_{f} in the presence of a condensing agent under a basic condition to obtain a compound of general Formula 1-3;
Step 3: removing the amino-protecting group W₁ of the compound of general Formula 1-3 to obtain a compound of general Formula 1-4;
Step 4: reacting the compound of general Formula 1-4 with a compound of general Formula 1-5 under a basic condition to obtain a compound of general Formula 1-6; and
Step 5: reacting the compound of general Formula 1-6 with bis(*p*-nitrophenyl)carbonate under a basic condition to obtain a compound of general Formula 1-7.
   wherein
   W₁ is an amino-protecting group, e.g., 9-fluorenylmethyloxycarbonyl; W₂ is a carboxylic acid active ester, for example, a succinimidyl ester;
   wherein n, AA, R, i and f are as described herein, and each FF¹ is independently 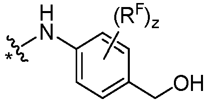 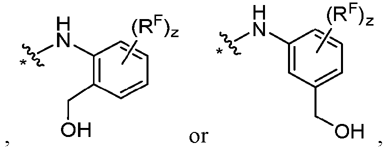 wherein each R^{F} is independently C1-C6 alkyl, C1-C6 alkoxy, -NO₂ or halogen; z is 0, 1, 2, 3 or 4; wherein * links to AA; each FF² is independently 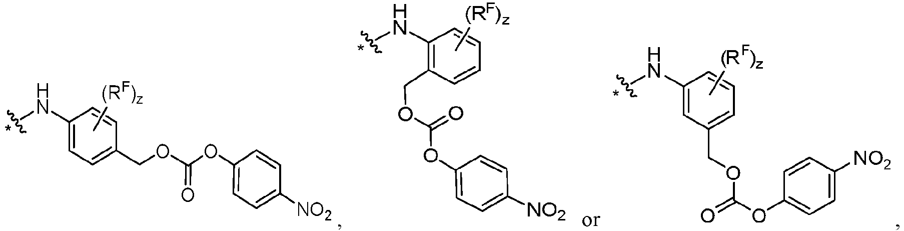
   wherein each R^{F} is independently C1-C6 alkyl, C1-C6 alkoxy, -NO₂ or halogen; z is 0, 1, 2, 3 or 4; wherein * links to AA;

In one or more embodiments, R^{F} is F.

In one or more embodiments, z is 0.

In one or more embodiments, z is 1 or 2.

In one or more embodiments, the intermediate is: 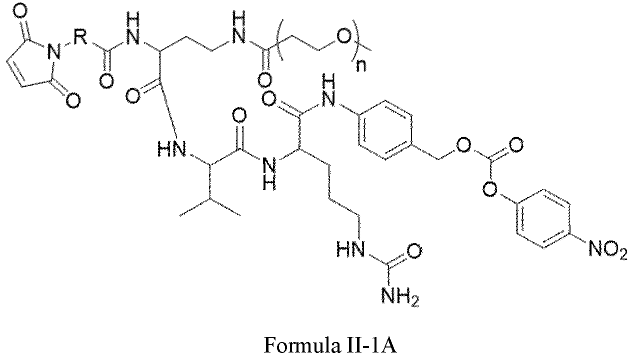 or 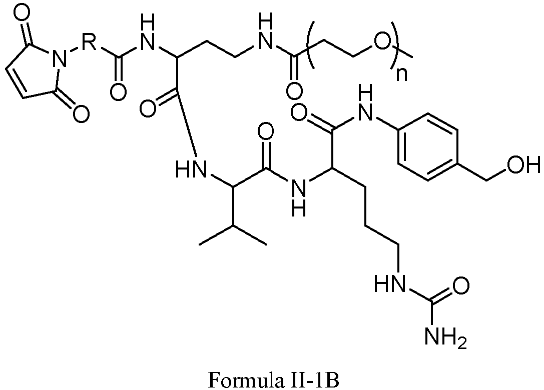 wherein R and n are as described herein.

In one or more embodiments, the intermediate is: 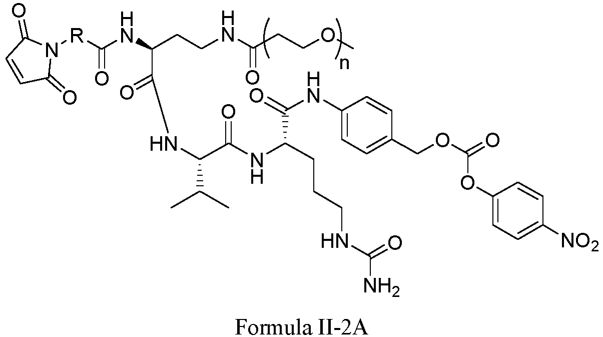 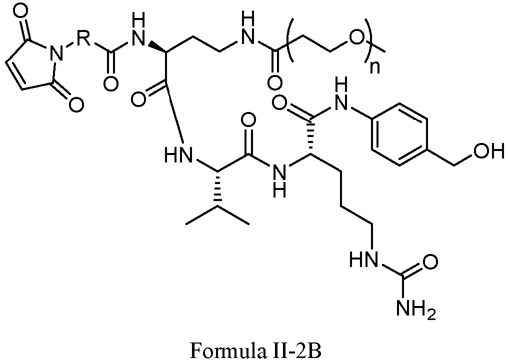 wherein R and n are as described herein.

In one or more embodiments, the intermediate is: 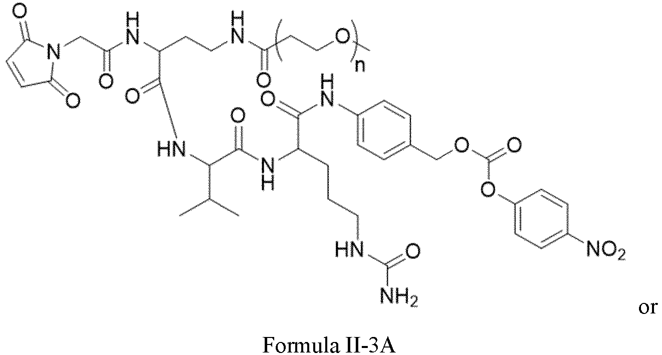 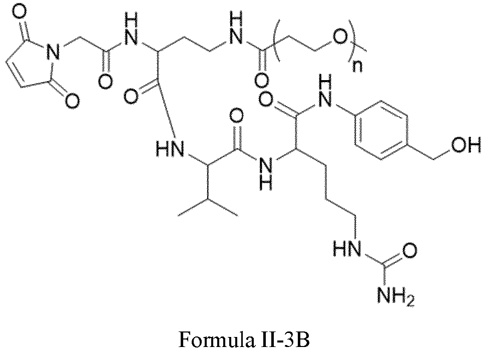 wherein n is as described herein.

In one or more embodiments, the intermediate is: 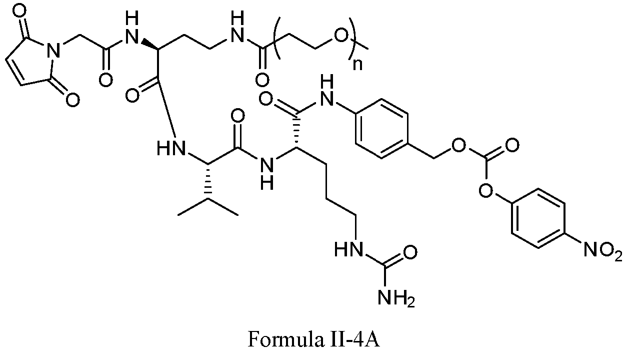 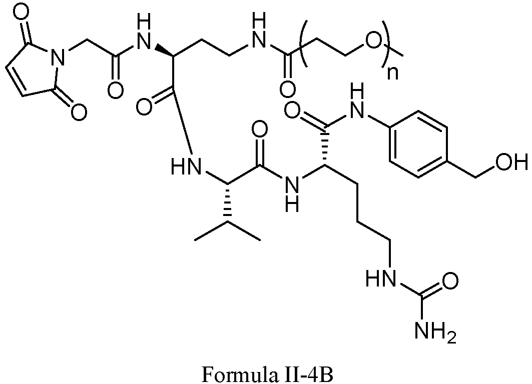 wherein n is as described herein.

The basic conditions described above can be provided using reagents including organic bases and inorganic bases, wherein the organic bases include, but are not limited to, triethylamine, diethylamine, N-methylmorpholine, pyridine, piperidine, *N,N-diisopropylethylamine, n*-butyllithium, lithium diisopropylamide, potassium acetate, sodium *tert*-butoxide, or potassium *tert*-butoxide, and the inorganic bases include, but are not limited to, sodium hydride, potassium phosphate, sodium carbonate, potassium carbonate, cesium carbonate, sodium hydroxide, and lithium hydroxide.

The condensing agent described above may be selected from *N*,*N*,*N*,*N*-tetramethyl-O-(7-azabenzotriazol-1-yl)uronium hexafluorophosphate, 4-(4,6-dimethoxy-1,3,5-triazin-2-yl)-4-methylmorpholinium chloride, 1-hydroxybenzotriazole, 1-(3-dimethylaminopropyl)-3-ethylcarbodiimide hydrochloride, *N,N'*-dicyclohexylcarbodiimide, *N,N*'-diisopropylcarbodiimide, *O-*benzotriazol-*N,N,N'*,*N'*-tetramethyluronium tetrafluoroborate, 1-hydroxybenzotriazole, 1-hydroxy-7-azobenzotriazol, *O*-benzotriazol-*N*,*N*,*N*,N-tetramethyluronium hexafluorophosphate, 2-(7-azobenzotriazol)-*N,N,N',N*'-tetramethylu*r*onium hexafluorophosphate, benzotriazol-1-yloxytris(dimethylamino)phosphonium hexafluorophosphate, and benzotriazol-1-yl-oxytripyrrolidinophosphonium hexafluorophosphate.

In one or more embodiments, the methods for preparing intermediate Compound 1-8 are described below: 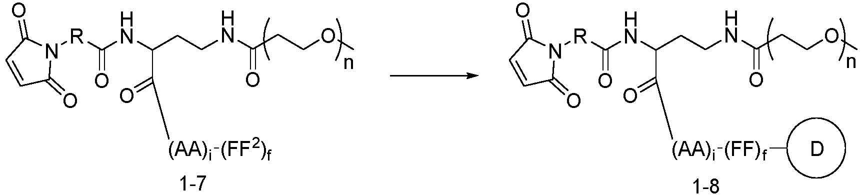
wherein n, AA, R, i, f, FF2, FF and D are as described herein.
the compound of general Formula 1-7 and D are reacted in the presence of a condensing agent under a basic condition to obtain a compound of general Formula 1-8.

In one or more embodiments, the intermediate is: 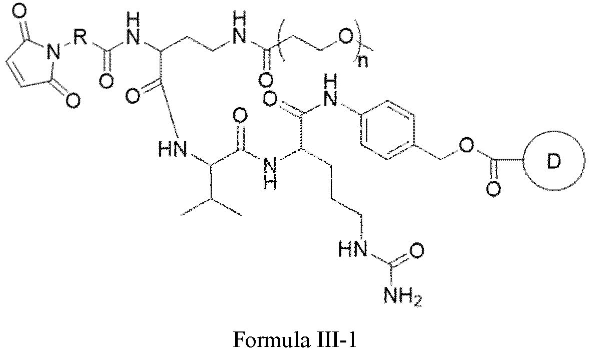 wherein R, n and D are as described herein.

In one or more embodiments, the intermediate is: 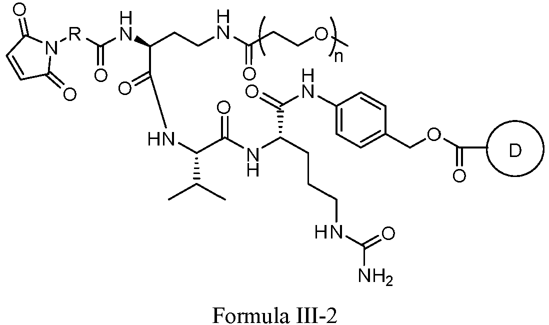 wherein R, n and D are as described herein.

In one or more embodiments, the intermediate is: 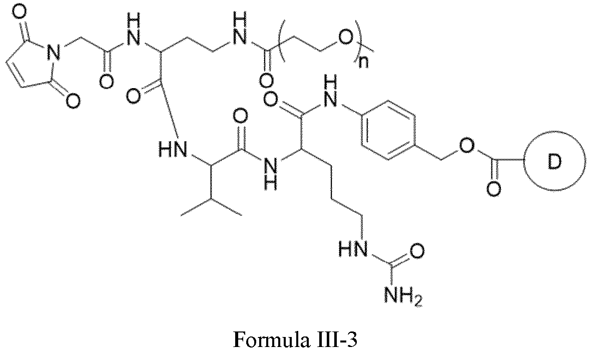 wherein n and D are as described herein.

In one or more embodiments, the intermediate is: 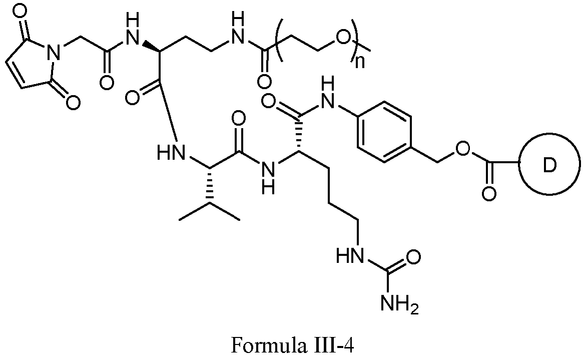 wherein n and D are as described herein.

The basic conditions described above can be provided using reagents including organic bases and inorganic bases, wherein the organic bases include, but are not limited to, triethylamine, diethylamine, *N*-methylmorpholine, pyridine, piperidine, *N,N-*diisopropylethylamine, *n*-butyllithium, lithium diisopropylamide, potassium acetate, sodium *tert*-butoxide, or potassium *tert*-butoxide, and the inorganic bases include, but are not limited to, sodium hydride, potassium phosphate, sodium carbonate, potassium carbonate, cesium carbonate, sodium hydroxide, and lithium hydroxide.

The condensing agent described above may be selected from *N*,*N*,*N*,*N*-tetramethyl-O-(7-azabenzotriazol-1-yl)uronium hexafluorophosphate, 4-(4,6-dimethoxy-1,3,5-triazin-2-yl)-4-methylmorpholinium chloride, 1-hydroxybenzotriazole, 1-(3-dimethylaminopropyl)-3-ethylcarbodiimide hydrochloride, *N*,*N*'-dicyclohexylcarbodiimide, *N*,*N*'-diisopropylcarbodiimide, *O-*benzotriazol-*N,N,N*',*N'*-tetramethyluronium tetrafluoroborate, 1-hydroxybenzotriazole, 1-hydroxy-7-azobenzotriazol, *O*-benzotriazol-*N*,*N*,*N*,*N*-tetramethyluronium hexafluorophosphate, 2-(7-azobenzotriazol)-*N,N,N',N*'-tetramethyluronium hexafluorophosphate, benzotriazol-1-yloxytris(dimethylamino)phosphonium hexafluorophosphate, and benzotriazol-1-yl-oxytripyrrolidinophosphonium hexafluorophosphate.

In one or more embodiments, the methods for preparing ADC 1-9 are described below: 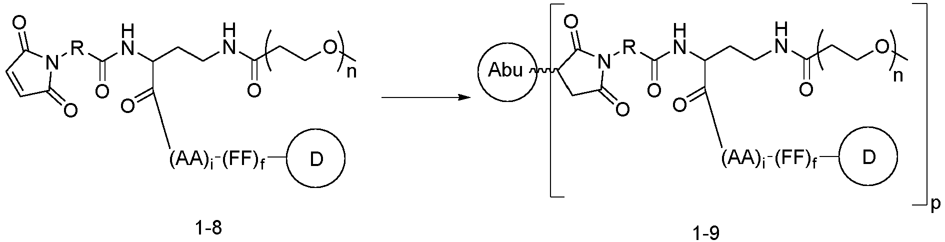 wherein n, AA, R, i, f, FF, D and Abu are as described herein.

The compound of general Formula 1-8 is conjugated to Abu under a weakly acidic condition to obtain a compound of general Formula 1-9. The interchain disulfide bond on the antibody provided by the present invention is reduced and opened by a thiol reducing agent to generate free sulfhydryl, and then the antibody undergoes a conjugation reaction with the compound of general Formula 1-8 to obtain an antibody-drug conjugate of general Formula 1-9.

The antibody-drug conjugate can be purified by a conventional method, e.g., preparative high performance liquid chromatography (prep-HPLC) or the like.

The weakly acidic condition described above may be provided using reagents including organic acids and inorganic acids, wherein the organic acids include, but are not limited to, acetic acid, benzoic acid, tartaric acid, oxalic acid, malic acid, citric acid, ascorbic acid, citric acid, salicylic acid, caffeic acid, sorbic acid, quinic acid, oleanolic acid, succinic acid, chlorogenic acid, formic acid, and propionic acid, and the inorganic acids include, but are not limited to, carbonic acid, nitrous acid, hypochlorous acid, hydrofluoric acid, sulfurous acid, hydrosulfuric acid, silicic acid, metasilicic acid, phosphoric acid, metaphosphoric acid, sodium bicarbonate, and sodium bisulfite.

### Treatment Method

The present invention further provides a treatment method and use. In one or more embodiments, provided is a method for treating or ameliorating various types of cancers or tumors and other related diseases, wherein the method comprises administering to a patient in need **thereof** an effective dose of the anti-CLDN18.2 antibody or the antigen-binding unit or the ADC. In one or more embodiments, provided is use of the anti-CLDN18.2 antibody or the antigen-binding unit or the ADC in treating or ameliorating cancers or tumors and other related diseases. In one or more embodiments, provided is use of the anti-CLDN18.2 antibody or the antigen-binding unit or the ADC in the manufacture of a medicament for treating or ameliorating cancers or tumors and other related diseases.

The specific dosage and therapeutic regimen for any particular patient will depend upon a variety of factors including the particular antibody, the antigen-binding fragment or derivative used, the age and body weight of the patient, general health condition, sex and diet, and the time of administration, frequency of excretion, drug combination, and the severity of the particular disease being treated. These factors are judged by medical caregivers included within the scope of those of ordinary skills in the art. The dosage will also depend on the individual patient to be treated, the route of administration, the type of the formulation, the nature of the compound used, the severity of the disease and the efficacy desired. The dosage employed can be determined by pharmacological and pharmacokinetic principles well known in the art. In some embodiments, the antibody or the antigen-binding unit or the ADC of the present invention is administered to a patient at a dose of 0.01 mg/kg to 100 mg/kg of patient body weight per administration. In some embodiments, the administration is performed once every week, every 2 weeks, every 3 weeks, or every month.

The modes of administration for the antibody or the antigen-binding unit or the ADC include, but are not limited to, intradermal, intramuscular, intraperitoneal, intravenous, subcutaneous, nasal and epidural injections and oral administration. The antibody, the antigen-binding unit, the ADC, or the composition can be administered by any convenient route, for example by infusion or bolus injection, or epithelial or mucocutaneous absorption (e.g., through oral mucosa, rectal, and intestinal mucosa), and can be co-administered with other biologically active agents. Thus, the pharmaceutical composition comprising the antibody or the antigen-binding unit or the ADC of the present invention can be administered orally, rectally, parenterally, intracisternally, intravaginally, intraperitoneally, topically (e.g. by powder, ointment, drop or transdermal patch) or buccally, or by oral or nasal spray.

The term "parenteral" as used herein refers to modes of administration including intravenous, intramuscular, intraperitoneal, intrasternal, subcutaneous and intraarticular injections and infusions.

The mode of administration may be systemic or local.

The antibody, the antigen-binding unit, the ADC, or the pharmaceutical composition of the present invention may be administered locally to an area in need of treatment; this may be achieved by (but not limited to) the following: local infusion during surgery (e.g., topical application in combination with a post-operative wound dressing), by injection, by means of a catheter, by means of a suppository, or by means of an implant, the implant being of a porous, non-porous, or gelatinous material, including membranes (such as silicone rubber membranes) or fibers. In some embodiments, when administering the protein (including the antibody) of the present invention, care must be taken to use materials that do not absorb the protein.

In one or more embodiments, the composition of the present invention comprises a nucleic acid or a polynucleotide encoding an antibody or antigen-binding unit, which can be administered *in vivo* to facilitate expression of the encoded protein by constructing the nucleic acid or the polynucleotide as part of a suitable nucleic acid expression vector; and a part of the vector is administered to be intracellular by the following methods, for example, by using a retroviral vector (see U.S. Pat. No. 4,980,286), by direct injection, by using microprojectile bombardment (e.g., gene gun; Biolistic, Dupont), by coating with lipids or cell surface receptors or transfection reagents, or by ligation with homeobox-like peptides with known capability of entering the nucleus (see, e.g., Joliot et al., 1991, Proc. Natl. Acad. Sci. USA 88:1864-1868). Alternatively, the nucleic acid can be introduced into the cell and integrated into the host cell DNA for expression by homologous recombination.

Methods for treating diseases comprising administering the antibody, the antigen-binding unit, the ADC or derivative described herein are generally tested *in vitro,* followed by *in vivo* tests for desired therapeutic or prophylactic activities in an acceptable animal model, and finally administration in humans. Suitable animal models (including transgenic animals) are well known to those of ordinary skills in the art. For example, *in vitro* assays for demonstrating therapeutic use of the antibody or the antigen-binding unit or the ADC of the present invention include the effect of the antibody or the antigen-binding unit or the ADC on a cell line or a patient tissue sample. The effect of the antibody or the antigen-binding unit or the ADC on the cell line and/or the tissue sample can be detected using techniques known to those skilled in the art, such as the techniques disclosed elsewhere herein. In accordance with the teachings of the present invention, *in vitro* assays that can be used to determine whether to administer a specific antibody or antigen-binding fragment include *in vitro* cell culture experiments, wherein a patient tissue sample is cultured in a culture medium and is exposed to or otherwise administered a compound, and the effect of the compound on the tissue sample is observed.

Various known delivery systems can be used to administer the antibody or the antigen-binding unit or the ADC of the present invention or the polynucleotide encoding the antibody or the antigen-binding unit of the present invention, e.g., encapsulation in liposomes, microparticles, microcapsules, recombinant cells capable of expressing the compound, receptor-mediated endocytosis (see, e.g., Wu and Wu, 1987, J. Biol. Chem. 262:4429-4432), construction of nucleic acids as part of a retrovirus or other vectors, or the like.

### Combination Therapy

In one or more embodiments, the anti-CLDN18.2 antibody or the antigen-binding unit or the ADC of the present invention can be administered in combination with other therapeutic or prophylactic regimens, including administration of one or more antibodies or antigen-binding units or the ADCs of the present invention together or in combination with one or more other therapeutic agents or methods. In one or more embodiments, other therapeutic regimens include, but are not limited to, radiation therapy, chemotherapy, hormone therapy, and the like. For combination therapy, the antibody or the antigen-binding unit or the ADC may be administered simultaneously or separately with other therapeutic agents. When administered separately, the antibody or the antigen-binding unit or the ADC of the present invention can be administered before or after administration of another additional therapeutic agent.

In one or more embodiments, the antibody or the antigen-binding unit or the ADC of the present invention can be administered in combination with a chemotherapeutic agent. In one or more embodiments, the antibody or the antigen-binding unit or the ADC of the present invention can be administered in combination with a cytokine, a chemokine, or a co-stimulatory molecule. In one or more embodiments, the antibody or the antigen-binding unit or the ADC of the present invention can be administered in combination with a immunotherapeutic agent.

### Diagnostic Methods

Expression of CLDN18.2 is observed in certain cancer or tumor samples, and patients positively expressing CLDN18.2 respond to treatment with the anti-CLDN18.2 antibody or the antigen-binding unit or the ADC of the present invention. Thus, the antibody or the antigen-binding unit of the present invention can also be used for diagnosis and prognosis.

A sample comprising cells may be obtained from a patient, which may be a cancer patient or a patient to be diagnosed. The cells are cells of a tumor tissue or tumor mass, a blood sample, a urine sample, or any sample from the patient. After an optional pretreatment of the sample, the sample can be incubated with the antibody or the antigen-binding unit of the present invention in a condition that allows the antibody or the antigen-binding unit to interact with CLDN18.2 protein that may be present in the sample. By methods such as ELISA, the presence and the expression level of CLDN18.2 protein in the sample can be detected using the anti-CLDN18.2 antibody or the antigen-binding unit.

The presence of CLDN18.2 protein (at any amount or concentration) in a sample can be used to diagnose a cancer, which may be an indication that the patient is eligible for an antibody therapy, or an indication that the patient has (or has not) responded to a cancer therapy. For prognostic methods, one, two or more tests may be performed at a particular stage after the beginning of a cancer therapy to indicate the progress of the therapy.

### Composition

The present invention further provides a pharmaceutical composition. Such a composition comprises an effective dose of the anti-CLDN18.2 antibody (or the antigen-binding unit) or the ADC and a pharmaceutically acceptable carrier. In some embodiments, the pharmaceutical composition comprises 0.1%-90% of the anti-CLDN18.2 antibody or the antigen-binding unit or the ADC. In some embodiments, the pharmaceutical composition further comprises an anti-cancer agent (e.g., an immune checkpoint inhibitor).

In one or more embodiments, the term "pharmaceutically acceptable" refers to materials listed in pharmacopoeia for use in animals, in particular in humans. In addition, the "pharmaceutically acceptable carrier" will generally be any type of non-toxic solid, semi-solid or liquid filler, diluent, encapsulating material, or formulation auxiliary.

In one or more embodiments, the composition is formulated into a pharmaceutical composition suitable for intravenous injection into a human body according to conventional steps. A composition for intravenous administration is a solution in sterile isotonic aqueous buffer. The composition may also comprise a solubilizer and a local anesthetic such as lidocaine to alleviate the pain at the injection site. In general, the active ingredients are provided in a unit dosage form individually or as a mixture, for example, the active ingredients are encapsulated in sealed containers (such as ampoule bottles or sachets) that can indicate the amount of the active agent, in the form of lyophilized powder or anhydrous concentrate. Where the composition is administered by infusion, the composition can be dispensed in infusion bottles containing sterile, pharmaceutical grade water or saline. Where the composition is administrated by injection, an ampoule bottle containing sterile water or saline for injection can be used, so that the active ingredients can be mixed before administration.

The compound of the present invention may be formulated in a neutral or salt form. Pharmaceutically acceptable salts include salts formed with anions derived from, e.g., hydrochloric acid, phosphoric acid, acetic acid, oxalic acid and tartaric acid, and salts formed with cations derived from, e.g., sodium, potassium, ammonium, calcium, iron hydroxide, isopropylamine, triethylamine, 2-ethylaminoethanol, histidine, and procaine.

### Examples

The materials, reagents, etc., used in the following examples are commercially available or may be obtained using known methods unless otherwise stated.

### Example 1. Preparation of antibodies

The variable and constant regions of the anti-CLDN18.2 antibodies are shown in Table 1. The heavy chains of the antibodies consist of VH and CH, and the light chains of the antibodies consist of VL and CL. The CDRs of the anti-CLDN18.2 antibodies are summarized in Table 2, the CDR region composition is shown in Table 3, and the variable region and constant region sequences are shown in Table 4. The light chains of the antibodies H239H-2b-K-6a-1, H239H-2b-K-6a-2, and H239H-2b-K-6a-3 have the same amino acid sequence, as set forth in SEQ ID NO: 72, and the corresponding gene sequence is set forth in SEQ ID NO: 73. The amino acid sequence of the heavy chain of the antibody H239H-2b-K-6a-1 is set forth in SEQ ID NO: 69, and the corresponding gene sequence is set forth in SEQ ID NO: 74. The amino acid sequence of the heavy chain of the antibody H239H-2b-K-6a-2 is set forth in SEQ ID NO: 70, and the corresponding gene sequence is set forth in SEQ ID NO: 75. The amino acid sequence of the heavy chain of the antibody H239H-2b-K-6a-3 is set forth in SEQ ID NO: 71, and the corresponding gene sequence is set forth in SEQ ID NO: 76 (see Tables 5 and 6). The antibody beginning with "CH" denotes a chimeric antibody (murine variable region + human constant region), and the antibody beginning with ''H'' denotes a humanized antibody (humanized variable region + human constant region).

**Table 1. Composition of variable and constant regions of the anti-CLDN18.2 antibodies**

| Antibody number | VH SEQ ID NO: | CH SEQ ID NO: | VL SEQ ID NO: | CL SEQ ID NO: |
|---|---|---|---|---|
| CH239H-2-K-6 | 47 | 65 | 60 | 68 |
| CH239H-9-K-4 | 48 | 65 | 59 | 68 |
| CH394H2-K-1 | 52 | 65 | 62 | 68 |
| CH394H1-K-4 | 51 | 65 | 63 | 68 |
| CH372H6-K-1 | 50 | 65 | 61 | 68 |
| CH239H-9-K-6 | 48 | 65 | 60 | 68 |
| CH239H-9-K-1 | 48 | 65 | 58 | 68 |
| CH101H1-K-1 | 46 | 65 | 56 | 68 |
| CH101H1-K-2 | 46 | 65 | 57 | 68 |
| CH372H1-K-1 | 49 | 65 | 61 | 68 |
| H239H-2a-K-6a | 53 | 65 | 64 | 68 |
| H239H-2b-K-6a | 54 | 65 | 64 | 68 |
| H239H-2b-K-6a-1 | 55 | 65 | 64 | 68 |
| H239H-2b-K-6a-2 | 55 | 66 | 64 | 68 |
| H239H-2b-K-6a-3 | 55 | 67 | 64 | 68 |

**Table 2. VH CDR1-3 and VL CDR1-3**

| Amino acid sequence of VH CDR1 | SE Q ID NO | Amino acid sequence of VH CDR2 | SE Q ID NO | Amino acid sequence of VH CDR3 | SE Q ID NO |
|---|---|---|---|---|---|
| SYYIH | 1 | WIYPGSGNTKYNEK FKG | 7 | GMDYGNYYLDY | 14 |
| DYGMH | 2 | YISRGRSTTYSTDTV KG | 8 | GSYYGNAMDY | 15 |
| NTYIY | 3 | RIDPANGNTKYAPKF QG | 9 | SPAYYINYYAMDY | 16 |
| DYYMH | 4 | YISSGRSTIYSADTVK G | 10 | GGYYGNAMDY | 17 |
| TSGMS | 5 | EIYPGSGNTYYNEKF KG | 11 | GGDYYDYDGTGYYA MDY | 18 |
| GYWIE | 6 | LINTYSGVPTYADDF KG | 12 | WSRAWFPY | 19 |
| / | / | EILPGSGSTNYNEKF KG | 13 | APLEGLRSGFAY | 20 |
| / | / | / | / | GSYYGNALDY | 21 |

| Amino acid sequence of VL CDR1 | SE Q ID NO | Amino acid sequence of VL CDR2 | SE Q ID NO | Amino acid sequence of VL CDR3 | SE Q ID NO |
|---|---|---|---|---|---|
| KASQDVGTAVA | 22 | WASTRHT | 30 | QQYSSYLT | 38 |
| HASQNINVWLS | 23 | KASNLHT | 31 | QQGQSYPYT | 39 |
| SASSSVSYMH | 24 | DTSKLAS | 32 | QQWSSNPFT | 40 |
| KASQNVGTNVA | 25 | STSYRYS | 33 | QQYNSYPLT | 41 |
| KSSQSLLNSGNQRN YLT | 26 | WASTRES | 34 | QSAYSYPFT | 42 |
| SASSSVSSSYLY | 27 | STSNLAS | 35 | HQWSSYPPT | 43 |
| RASESVDSYGNSFM H | 28 | RASNLES | 36 | QQSNEDPRT | 44 |
| RASQSISDYLH | 29 | YASQSIS | 37 | QNGHSFPYT | 45 |

**Table 3. Composition of CDRs of antibodies**

| Name | VH CDR1 SEQ ID N O: | VH CDR2 SEQ ID N O: | VH CDR3 SEQ ID N O: | VL CDR1 SEQ ID N O: | VL CDR2 SEQ ID N O: | VL CDR3 SEQ ID N O: |
|---|---|---|---|---|---|---|
| CH101H1-K-1 | 1 | 7 | 14 | 22 | 30 | 38 |
| CH101H1-K-2 | 1 | 7 | 14 | 23 | 31 | 39 |
| CH239H-2-K-6 | 2 | 8 | 15 | 26 | 34 | 42 |
| CH239H-9-K-4 | 3 | 9 | 16 | 25 | 33 | 41 |
| CH239H-9-K-6 | 3 | 9 | 16 | 26 | 34 | 42 |
| CH239H-9-K-1 | 3 | 9 | 16 | 24 | 32 | 40 |
| CH372H1-K-1 | 2 | 10 | 17 | 27 | 35 | 43 |
| CH372H6-K-1 | 4 | 11 | 18 | 27 | 35 | 43 |
| CH394H1-K-4 | 5 | 12 | 19 | 29 | 37 | 45 |
| CH394H2-K-1 | 6 | 13 | 20 | 28 | 36 | 44 |
| H239H-2a-K-6a | 2 | 8 | 15 | 26 | 34 | 42 |
| H239H-2b-K-6a | 2 | 8 | 15 | 26 | 34 | 42 |
| H239H-2b-K-6a-1 | 2 | 8 | 21 | 26 | 34 | 42 |
| H239H-2b-K-6a-2 | 2 | 8 | 21 | 26 | 34 | 42 |
| H239H-2b-K-6a-3 | 2 | 8 | 21 | 26 | 34 | 42 |

**Table 4. Amino acid sequence of the variable region and constant region**

| SEQ ID NO: | Amino acid sequence |
|---|---|
| 46 | EVQLQESGPELVKPGASVKISCKASGYSFTSYYIHWVKQRPGQGLEWIGWIYP GSGNTKYNEKFKGKATLTADTSSSTAYMQLSSLTSEDSAVYYCARGMDYGN YYLDYWGQGTTLTVSS (VH CDR1, 2 and 3 was underlined sequentially) |
| 47 | EVKLQESGGGLVKPGGSLKVSCAASGFTFSDYGMHWVRQAPEKGLEWVAYI SRGRSTTYSTDTVKGRFTISRDSAKHTLFLQMTSLRSEDTAMYYCARGSYYG NAMDYWGQGTSVTVSS(VH CDR1, 2 and 3 was underlined sequentially) |
| 48 | EVKLQQSVAELVRPGASVKLSCTASGFNIKNTYIYWVKQRPEQGLEWIGRIDP ANGNTKYAPKFQGKATITADTSSNTAYLQLSSLTSEDTAIYYCARSPAYYINY YAMDYWGQGTSVTVSS(VH CDR1, 2 and 3 was underlined sequentially) |
| 49 | EVQLQESGGGLVKPGGSLKLSCAASGFTFSDYGMHWVRQAPEKGLEWVAYI SSGRSTIYSADTVKGRFTISRDNAKNTLFLQMTSLRSEDTAMYYCARGGYYG NAMDYWGQGTSVTVSS(VH CDR1, 2 and 3 was underlined sequentially) |
| 50 | EVKLQESGPELVKPGASVKMSCKASGYTFTDYYMHWVKQKPGKGLEWIGEI YPGSGNTYYNEKFKGKATLTADTSSSTAYMQLSSLTSEDSAVYFCAGGGDYY DYDGTGYYAMDYWGQGTSVTVSS(VH CDR1, 2 and 3 was underlined sequentially) |
| 51 | EVQLQESGETVKISCKASGYIFTTSGMSWVIQAPGKGLKWMGLINTYSGVPTY ADDFKGRFAFSLETSASTAYLQINNLENEDTATYFCAIWSRAWFPYWGQGTL VTVSA(VH CDR1, 2 and 3 was underlined sequentially) |
| 52 | EVKLQESGAELMKPGASVKLSCKATGYTFTGYWIEWVKQRPGHGLEWIGEIL PGSGSTNYNEKFKGKATFTADTSSNTAYMQLSSLTTEDSAIYYCARAPLEGLR SGFAYWGQGTLVTVSA(VH CDR1, 2 and 3 was underlined sequentially) |
| 53 | EVQLLESGGGLVQPGGSLRLSCAASGFTFSDYGMHWVRQAPGKGLEWVAYIS RGRSTTYSTDTVKGRFTISRDSSKNTLYLQMNSLRAEDTAVYYCARGSYYGN AMDYWGQGTLVTVSS(VH CDR1, 2 and 3 was underlined sequentially) |
| 54 | EVQLLESGGGLVQPGGSLRLSCAASGFTFSDYGMHWVRQAPGKGLEWVAYIS RGRSTTYSTDTVKGRFTISRDNSKNTLYLQMNSLRAEDTAVYYCARGSYYGN AMDYWGQGTLVTVSS(VH CDR1, 2 and 3 was underlined sequentially) |
| 55 | EVQLLESGGGLVQPGGSLRLSCAASGFTFSDYGMHWVRQAPGKGLEWVAYIS RGRSTTYSTDTVKGRFTISRDNSKNTLYLQMNSLRAEDTAVYYCARGSYYGN ALDYWGQGTLVTVSS(VH CDR1, 2 and 3 was underlined sequentially) |
| 56 | DIVMTQSHKFMSTSVGDRVSITCKASQDVGTAVAWYQQKPGQSPKLLIYWAS TRHTGVPDRFTGSGSGTDFTLTISNVQSEDLADYFCQQYSSYLTFGAGTKLEL K(VL CDR1, 2 and 3 was underlined sequentially) |
| 57 | DIQLTQSPSSLSASLGDTITITCHASQNINVWLSWYQQKPGNIPKLLIYKASNLH TGVPSRFSGSGSGTGFTLTISSLQPEDIATYYCQQGQSYPYTFGGGTKLEIK(VL CDR1, 2 and 3 was underlined sequentially) |
| 58 | QIVLTQSPAIMSASPGEKVTMTCSASSSVSYMHWYQQKSGTSPKRWIYDTSKL ASGVPARFSGSGSGTSYSLTISSMEAEDAATYYCQQWSSNPFTFGSGTKLEIK( VL CDR1, 2 and 3 was underlined sequentially) |
| 59 | DIVMTQSQKFMSTSVGDRVSVTCKASQNVGTNVAWYQLKPGQSPKPLIYSTS YRYSGVPDRFTGSGSGTDFTLTISNVQSEDLAEYFCQQYNSYPLTFGAGTKLE LT(VL CDR1, 2 and 3 was underlined sequentially) |
| 60 | DIVMTQSPSSLTVTAGERVTMSCKSSQSLLNSGNQRNYLTWYQQKPGQPPKL LIYWASTRESGVPDRFTGSGSGTDFTLTISSVQAEDLAVYFCQSAYSYPFTFGS GTKLEIK(VL CDR1, 2 and 3 was underlined sequentially) |
| 61 | QIVLTQSPAIMSASPGEKVTLTCSASSSVSSSYLYWYQQKPGSSPKLWIYSTSN LASGVPARFSGSGSGTSYSLTISSMEAEDAASYFCHQWSSYPPTFGGGTKLEIK (VL CDR1, 2 and 3 was underlined sequentially) |
| 62 | DIVLTQSPASLAVSLGQRATISCRASESVDSYGNSFMHWYQQKPGQPPKLLIY RASNLESGIPARFSGSGSRTDFTLTINPVEADDVATYYCQQSNEDPRTFGGGTK LEIK(VL CDR1, 2 and 3 was underlined sequentially) |
| 63 | DIVMTQSPATLSVTPGDRVSLSCRASQSISDYLHWYQQKSHESPRLLIKYASQS ISGIPSRFSGSGSGSDFTLSINSVEPEDVGVYYCQNGHSFPYTFGGGTKLEIK(VL CDR1, 2 and 3 was underlined sequentially) |
| 64 | DIVMTQSPDSLAVSLGERATINCKSSQSLLNSGNQRNYLTWYQQKPGQPPKLL IYWASTRESGVPDRFSGSGSGTDFTLTISSLQAEDVAVYYCQSAYSYPFTFGGG TKLEIK(VL CDR1, 2 and 3 was underlined sequentially) |
| 65 | |
| 66 | ASTKGPSVFPLAPSSKSTSGGTAALGCLVKDYFPEPVTVSWNSGALTSGVHTF PAVLQSSGLYSLSSVVTVPSSSLGTQTYICNVNHKPSNTKVDKKVEPKSCDKT HTCPPCPAPELLGGPSVFLFPPKPKDTLMISRTPEVTCVVVDVSHEDPEVKFNW YVDGVEVHNAKTKPREEQYASTYRVVSVLTVLHQDWLNGKEYKCKVSNKA LPAPIEKTISKAKGQPREPQVYTLPPSRDELTKNQVSLTCLVKGFYPSDIAVEW ESNGQPENNYKTTPPVLDSDGSFFLYSKLTVDKSRWQQGNVFSCSVMHEALH NHYTQKSLSLSPGK(N297A mutation) |
| 67 | ASTKGPSVFPLAPSSKSTSGGTAALGCLVKDYFPEPVTVSWNSGALTSGVHTF PAVLQSSGLYSLSSVVTVPSSSLGTQTYICNVNHKPSNTKVDKKVEPKSCDKT HTCPPCPAPEAAGGPSVFLFPPKPKDTLMISRTPEVTCVVVDVSHEDPEVKFN WYVDGVEVHNAKTKPREEQYNSTYRVVSVLTVLHQDWLNGKEYKCKVSNK ALGAPIEKTISKAKGQPREPQVYTLPPSRDELTKNQVSLTCLVKGFYPSDIAVE WESNGQPENNYKTTPPVLDSDGSFFLYSKLTVDKSRWQQGNVFSCSVMHEAL HNHYTQKSLSLSPGK(LALA P329G mutation) |
| 68 | |

**Table 5. The heavy chain and light chain of the example anti-CLDN18.2 antibodies**

| Name | SEQ ID NO: | Amino acid sequence |
|---|---|---|
| heavy chain | 69 | EVQLLESGGGLVQPGGSLRLSCAASGFTFSDYGMHWVRQAPGKGL EWVAYISRGRSTTYSTDTVKGRFTISRDNSKNTLYLQMNSLRAEDT AVYYCARGSYYGNALDYWGQGTLVTVSSASTKGPSVFPLAPSSKS TSGGTAALGCLVKDYFPEPVTVSWNSGALTSGVHTFPAVLQSSGL YSLSSVVTVPSSSLGTQTYICNVNHKPSNTKVDKKVEPKSCDKTHT CPPCPAPELLGGPSVFLFPPKPKDTLMISRTPEVTCVVVDVSHEDPE VKFNWYVDGVEVHNAKTKPREEQYNSTYRVVSVLTVLHQDWLN GKEYKCKVSNKALPAPIEKTISKAKGQPREPQVYTLPPSRDELTKN QVSLTCLVKGFYPSDIAVEWESNGQPENNYKTTPPVLDSDGSFFLY SKLTVDKSRWQQGNVFSCSVMHEALHNHYTQKSLSLSPGK |
| | 70 | EVQLLESGGGLVQPGGSLRLSCAASGFTFSDYGMHWVRQAPGKGL EWVAYISRGRSTTYSTDTVKGRFTISRDNSKNTLYLQMNSLRAEDT AVYYCARGSYYGNALDYWGQGTLVTVSSASTKGPSVFPLAPSSKS TSGGTAALGCLVKDYFPEPVTVSWNSGALTSGVHTFPAVLQSSGL YSLSSVVTVPSSSLGTQTYICNVNHKPSNTKVDKKVEPKSCDKTHT CPPCPAPELLGGPSVFLFPPKPKDTLMISRTPEVTCVVVDVSHEDPE VKFNWYVDGVEVHNAKTKPREEQYASTYRVVSVLTVLHQDWLN GKEYKCKVSNKALPAPIEKTISKAKGQPREPQVYTLPPSRDELTKN QVSLTCLVKGFYPSDIAVEWESNGQPENNYKTTPPVLDSDGSFFLY SKLTVDKSRWQQGNVFSCSVMHEALHNHYTQKSLSLSPGK |
| | 71 | EVQLLESGGGLVQPGGSLRLSCAASGFTFSDYGMHWVRQAPGKGL EWVAYISRGRSTTYSTDTVKGRFTISRDNSKNTLYLQMNSLRAEDT AVYYCARGSYYGNALDYWGQGTLVTVSSASTKGPSVFPLAPSSKS TSGGTAALGCLVKDYFPEPVTVSWNSGALTSGVHTFPAVLQSSGL YSLSSVVTVPSSSLGTQTYICNVNHKPSNTKVDKKVEPKSCDKTHT CPPCPAPEAAGGPSVFLFPPKPKDTLMISRTPEVTCVVVDVSHEDPE VKFNWYVDGVEVHNAKTKPREEQYNSTYRVVSVLTVLHQDWLN GKEYKCKVSNKALGAPIEKTISKAKGQPREPQVYTLPPSRDELTKN QVSLTCLVKGFYPSDIAVEWESNGQPENNYKTTPPVLDSDGSFFLY SKLTVDKSRWQQGNVFSCSVMHEALHNHYTQKSLSLSPGK |
| light chain | 72 | DIVMTQSPDSLAVSLGERATINCKSSQSLLNSGNQRNYLTWYQQKP GQPPKLLIYWASTRESGVPDRFSGSGSGTDFTLTISSLQAEDVAVYY CQSAYSYPFTFGGGTKLEIKRTVAAPSVFIFPPSDEQLKSGTASVVC LLNNFYPREAKVQWKVDNALQSGNSQESVTEQDSKDSTYSLSSTL TLSKADYEKHKVYACEVTHQGLSSPVTKSFNRGEC |

**Table 6. The gene sequence of the example anti-CLDN18.2 antibodies**

| Name | SEQ ID NO: | Nucleotide sequence |
|---|---|---|
| light chain | 73 | gacatcgtgatgacccagtctccagattctctggcagtgtctctgggagaaagagctacaatcaactgcaa gtcctctcagtctctgctgaactccggcaaccagagaaactacctgacttggtaccagcagaaaccaggac agcctcctaagctgctgatctattgggcttctaccagggaatccggagtgccagatagattttccggatctgg atccggcaccgattttacactgacaatctcttctctgcaggcagaagacgtggctgtgtattattgccagtcc gcctactcctacccttttacctttggcggaggaacaaagctggagatcaagcgtacggtggctgcaccatct gtcttcatcttcccgccatctgatgagcagttgaaatctggaactgcctctgttgtgtgcctgctgaataacttc tatcccagagaggccaaagtacagtggaaggtggataacgccctccaatcgggtaactcccaggagagt gtcacagagcaggacagcaaggacagcacctacagcctcagcagcaccctgacgctgagcaaagcag actacgagaaacacaaagtctacgcctgcgaagtcacccatcagggcctgagctcgcccgtcacaaaga gcttcaacaggggagagtgt |
| heavy chain | 74 | gaagtgcaacttcttgaatctggaggaggacttgtgcaacctggaggatctcttagactttcttgcgcagcat ctggatttacattcagcgactatggaatgcattgggtgagacaagcacctggaaagggtctagaatgggtg gcatatatctctagaggaagatctacaacatattctacagatacagtgaaaggaagatttacaatctctagag ataactctaagaataccctttatcttcaaatgaactctcttagagcagaagatacagcagtgtattattgcgcaa gaggatcttattatggaaacgcacttgattattgggggcagggaacacttgtgacagtgtcttctgcatctac aaagggtcctagcgtgtttcctcttgcaccttcttctaaatctacatctggaggaacagcagcacttggatgcc ttgtgaaagattatttccccgaaccggtgacggtgtcgtggaactcaggcgccctgaccagcggcgtgcac accttcccggctgtcctacagtcctcaggactctactccctcagcagcgtggtgaccgtgccctccagcag cttgggcacccagacctacatctgcaacgtgaatcacaagcccagcaacaccaaggtggacaagaaagtt gagcccaaatcttgtgacaaaactcacacatgcccaccgtgcccagcacctgaactcctggggggaccgt cagtcttcctcttccccccaaaacccaaggacaccctcatgatctcccggacccctgaggtcacatgcgtg gtggtggacgtgagccacgaagaccctgaggtcaagttcaactggtacgtggacggcgtggaggtgcat aatgccaagacaaagccgcgggaggagcagtacaacagcacgtaccgtgtggtcagcgtcctcaccgtc ctgcaccaggactggctgaatggcaaggagtacaagtgcaaggtctccaacaaagccctcccagccccc atcgagaaaaccatctccaaagccaaagggcagccccgagaaccacaggtgtacaccctgcccccatcc cgggatgagctgaccaagaaccaggtcagcctgacctgcctggtcaaaggcttctatcccagcgacatcg ccgtggagtgggagagcaatgggcagccggagaacaactacaagaccacgcctcccgtgctggactcc gacggctccttcttcctctacagcaagctcaccgtggacaagagcaggtggcagcaggggaacgtcttct catgctccgtgatgcatgaggctctgcacaaccactacacgcagaagagcctctccctgtctccgggtaaa |
| | 75 | gaagtgcaacttcttgaatctggaggaggacttgtgcaacctggaggatctcttagactttcttgcgcagcat ctggatttacattcagcgactatggaatgcattgggtgagacaagcacctggaaagggtctagaatgggtg gcatatatctctagaggaagatctacaacatattctacagatacagtgaaaggaagatttacaatctctagag ataactctaagaataccctttatcttcaaatgaactctcttagagcagaagatacagcagtgtattattgcgcaa gaggatcttattatggaaacgcacttgattattgggggcagggaacacttgtgacagtgtcttctgcatctac aaagggtcctagcgtgtttcctcttgcaccttcttctaaatctacatctggaggaacagcagcacttggatgcc ttgtgaaagattatttccccgaaccggtgacggtgtcgtggaactcaggcgccctgaccagcggcgtgcac accttcccggctgtcctacagtcctcaggactctactccctcagcagcgtggtgaccgtgccctccagcag cttgggcacccagacctacatctgcaacgtgaatcacaagcccagcaacaccaaggtggacaagaaagtt gagcccaaatcttgtgacaaaactcacacatgcccaccgtgcccagcacctgaactcctggggggaccgt cagtcttcctcttccccccaaaacccaaggacaccctcatgatctcccggacccctgaggtcacatgcgtg gtggtggacgtgagccacgaagaccctgaggtcaagttcaactggtacgtggacggcgtggaggtgcat aatgccaagacaaagccgcgggaggagcagtacgccagcacgtaccgtgtggtcagcgtcctcaccgt cctgcaccaggactggctgaatggcaaggagtacaagtgcaaggtctccaacaaagccctcccagcccc catcgagaaaaccatctccaaagccaaagggcagccccgagaaccacaggtgtacaccctgcccccatc ccgggatgagctgaccaagaaccaggtcagcctgacctgcctggtcaaaggcttctatcccagcgacatc gccgtggagtgggagagcaatgggcagccggagaacaactacaagaccacgcctcccgtgctggactc cgacggctccttcttcctctacagcaagctcaccgtggacaagagcaggtggcagcaggggaacgtcttc tcatgctccgtgatgcatgaggctctgcacaaccactacacgcagaagagcctctccctgtctccgggtaa a |
| | 76 | gaagtgcaacttcttgaatctggaggaggacttgtgcaacctggaggatctcttagactttcttgcgcagcat ctggatttacattcagcgactatggaatgcattgggtgagacaagcacctggaaagggtctagaatgggtg gcatatatctctagaggaagatctacaacatattctacagatacagtgaaaggaagatttacaatctctagag ataactctaagaataccctttatcttcaaatgaactctcttagagcagaagatacagcagtgtattattgcgcaa gaggatcttattatggaaacgcacttgattattgggggcagggaacacttgtgacagtgtcttctgcctctac aaaggggcctagtgtatttccactggccccttcttctaaatccaccagtggtggtacggcagcactcggttg cctggtgaaggattacttcccggaaccggtcaccgtaagttggaactcaggcgctcttaccagcggagttc acacctttccagccgtgctgcagagctccgggctgtattctctgtcctctgtggtgaccgttcctagttcctcc ctgggaacacagacctacatatgcaacgtgaatcataagccaagcaacactaaggtcgacaaaaaggtgg agcccaagagctgtgacaagacccacacctgtccgccatgtccagcccctgaagctgcaggaggtccat cagtgttcctgttccctccgaagccaaaggacaccctgatgatctcacgaactcccgaagtgacttgcgtcg tggtggacgtgagtcacgaagaccccgaagtcaagttcaattggtatgtcgatggcgttgaggttcacaatg caaagaccaagccaagagaggagcagtacaactcaacataccgggttgtgtctgtgctgaccgtgctgca tcaggattggttgaacgggaaagagtataaatgcaaagttagcaacaaggcactcggtgctccaatagag aagaccataagcaaggctaaggggcagccacgcgaacctcaggtttacaccttgcctccatctagggatg aactgactaaaaaccaagtcagcctgacatgcctcgtgaagggattctatccttctgacattgcagtcgagt gggagtccaacggccagccagaaaacaactacaaaacaactcctcctgtgctcgacagcgacggatcttt ctttctctattccaagttgactgtggacaaaagccgctggcagcaggggaacgtcttctcttgctccgtgatg cacgaggccttgcataaccattacacccagaaatctctctcactgtctccaggcaaa |

A ribosome binding site (kozak sequence: GCCGCCACC, SEQ ID NO: 77) and the gene sequence of a signal peptide were added at the 5' end of the antibody gene sequence.

The light chain signal peptide was MDMRVLAQLLGLLLLCFPGARC (SEQ ID NO: 78), and the corresponding nucleotide sequence was atggacatgagggtgetggcccagetgetgggactgetgetgetgtgettcccaggcgccagatge (SEQ ID NO: 79).

The heavy chain signal peptide was MGWSLELLFLVAVATRVLS (SEQ ID NO: 80), and the gene sequence of the signal peptide added to the heavy chain gene sequence of the antibodies H239H-2b-K-6a-1, H239H-2b-K-6a-2, and H239H-2b-K-6a-3 was atgggttggtctcttatccttctattgctggttocagtggcaacaagagtgctttct (SEQ ID NO: 81), while the gene sequence of the signal peptide added to the heavy chain gene sequence of other antibodies was atgggttggtctctgattctcctgtttctggtggcagtggctacaagagtcctgtca (SEQ ID NO: 82).

A stop codon TGA was designed at the 3' end of the antibody gene sequence.

The heavy chain and light chain gene sequences of the antibody were connected to an expression vector to construct an expression plasmid with full-length light chain and heavy chain. The plasmid expressing the light and heavy chains of the antibody was transiently transfected into HEK293F cells in a ratio of 1:1. The cells were cultured, and the culture was purified to give the antibody.

### Example 2. Synthesis Procedures for Compound (1S,9S)-1-amino-9-ethyl-4,5-difluoro-9-hydroxy-1,2,3,9,12,15-hexahydro-10H,13H-benzo[de]pyrano[3',4':6,7]indolizino[1,2-b]quinolin-10,13-dione hydrochloride (D-1)

### 1. Synthesis ofN,N'-(3,4-difluoro-8-oxo-5,6,7,8-tetrahydronaphthalene-1,7-diyl)diacetamide

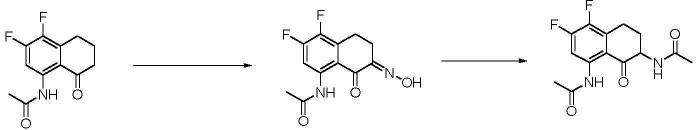

A solution of potassium tert-butoxide in tetrahydrofuran (42 mL, 1 M) was added to a dry reaction flask under a nitrogen atmosphere, stirred, and cooled to 0-5 °C. N-(3,4-difluoro-8-oxo-5,6,7,8-tetrahydronaphthalen-1-yl)acetamide (CAS No.: 143655-49-6, 5 g, 21 mmol) dissolved in tetrahydrofuran (25 mL) was slowly added dropwise to the reaction flask, followed by tert-butyl nitrite (4.32 g, 2 eq) (with temperature controlled at 0-5 °C). The resulting mixture was warmed to 15-20 °C and stirred for 2 hours (h). After the completion of the reaction, the mixture was cooled to 0-5 °C. Acetic acid (25 mL) and acetic anhydride (25 mL) were added dropwise (with temperature controlled at no more than 10 °C). After the dropwise addition, the mixture was stirred for 20 minutes (min). With temperature maintained at 5-10 °C, zinc powder (8 eq) was added according to the amount of N-(3,4-difluoro-8-oxo-5,6,7,8-tetrahydronaphthalen-1-yl)acetamide. The mixture was stirred at 20-25 °C for 1 h. The mixture was filtered, and the solid was rinsed with ethyl acetate (50 mL). The temperature was reduced to 0-5 °C, and 15% aqueous solution of NaCO₃ (50 mL) was added dropwise for three washings. Ethyl acetate (25 mL) was added for extraction. The organic phases were pooled and washed with a saturated aqueous solution of NaCl. Ethyl acetate (10 mL) was added, and the mixture was stirred at 40 °C for 30 min, slowly cooled to 0-5 °C, and stirred for 2 h. The mixture was filtered, and the solid was washed with ethyl acetate/petroleum ether (1/2, 10 mL). Drying was performed in vacuo to obtain a gray powder (2.1 g, 33.7%). LC-MS: [M+H]⁺=297.

### 2. Synthesis of N-(8-amino-5,6-difluoro-1-oxo-1,2,3,4-tetrahydronaphthalen-2-yl)acetamide

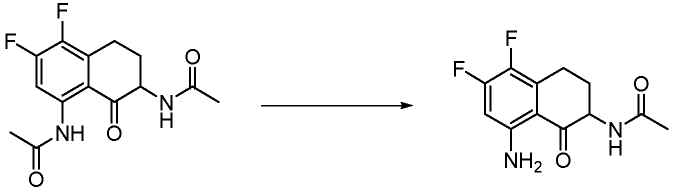

N,N-(3,4-difluoro-8-oxo-5,6,7,8-tetrahydronaphthalene-1,7-diyl)diethylamide (500 mg, 1.68 mmol) was added to a 2 M solution of hydrochloric acid in ethanol (5 mL). The resulting mixture was stirred at 50 °C for 4 h. After the completion of the reaction as detected, water (7.5 mL) was added. The mixture was cooled to 0-5 °C, and triethylamine (1.03 g) was added dropwise. The mixture was stirred for 3 h. The mixture was filtered, and washing was performed successively with 40% cold aqueous solution of ethanol (3 mL) and water (3 mL). Drying was performed *in vacuo* to obtain a gray powder (320 mg, 74.6%). LC-MS: [M+H]⁺=255.

### 3. Synthesis of N-((9S)-9-ethyl-4,5-difluoro-9-hydroxy-10,13-dioxo-2,3,9,10,13,15-hexahydro-1H, 12H-benzo[de]pyrano[3';4':6,7]indolizino[1,2-b]quinolin-1-yl)acetamide

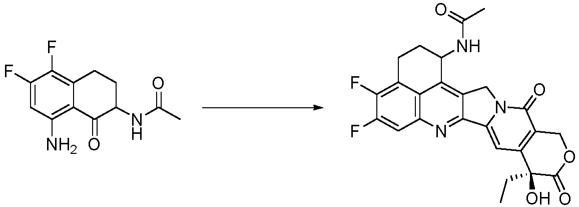

N-(8-amino-5,6-difluoro-1-oxo-1,2,3,4-tetrahydronaphthalen-2-yl)acetamide (1.1 g, 1 eq), (*S*)-4-ethyl-4-hydroxy-7,8-dihydro-1*H*-pyrano[3,4-*f*]indolizine-3,6,10(4*H*)-trione (1.15 g, 1 eq) and toluene (50 ml) were added to a reaction flask under a nitrogen atmosphere. The mixture was warmed to reflux and stirred for 1 h. Then pyridinium 4-toluenesulfonate (100 mg) was added, and the mixture was stirred at reflux for another 20 h. The mixture was cooled to room temperature and stirred for 1 h. The mixture was filtered, and the solid was successively washed with acetone (10 mL) and cold ethanol (5 mL). Drying was performed *in vacuo* to obtain a taupe powder (1.1 g, 53%). LC-MS: [M+H]⁺=482.

### 4. Synthesis of (1S,9S)-1-amino-9-ethyl-4,5-difluoro-9-hydroxy-1,2,3,9,12,15-hexahydro-10H,13H-benzo[de]pyrano[3',4':6,7]indolizino[1,2-b]quinolin-10,13-dione hydrochloride

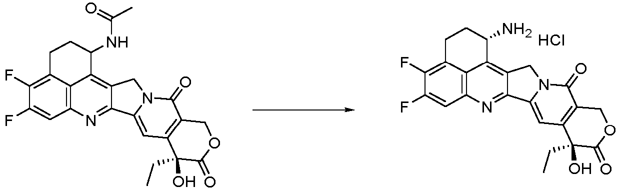

N-((9S)-9-ethyl-4,5-difluoro-9-hydroxy-10,13-dioxo-2,3,9,10,13,15-hexahydro-1H,12H-benzo[de]pyrano[3',4':6,7]indolizino[1,2-b]quinolin-1-yl)acetamide (1.1 g, 2.28 mmol) and a 6 M aqueous solution of hydrochloric acid (44 mL) were added to a reaction flask. The mixture was refluxed with stirring under a nitrogen atmosphere for 4 h. The mixture was concentrated to remove the solvent, and the residue was purified by HPLC to obtain a white powder (200 mg, 18%). LC-MS: [M+H]⁺=440.

### Example 3. Synthesis Procedures for 4-((30S,33S,36S)-30-(2-(2,5-dioxo-2,5-dihydro-1H-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amino)benzyl (4-nitrophenyl) carbonate (CB07)

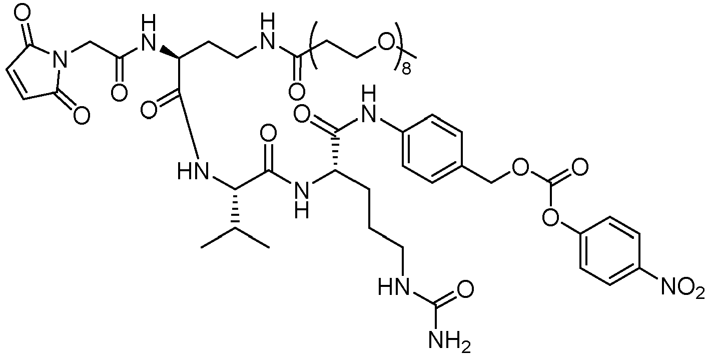

### 1) Synthesis of (S)-30-((((9H-fluoren-9-yl)methoxy)carbonyl)amino)-26-oxo-2,5,8,11,14,17,20,23-octaoxa-27-aza-hentriacontan-31-oic acid (CB01).

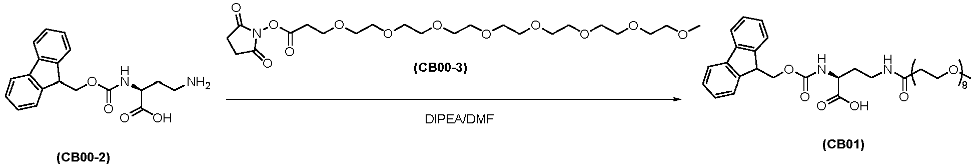

Under a nitrogen atmosphere at 0-5 °C, 8.14 g of N2-fluorenylmethoxycarbonyl-L-2,4-diaminobutyric acid (CB00-2) was dissolved in 40 mL of dimethylformamide (DMF), and 10 g of N-succinimidyl 4,7,10,13,16,19,22,25-octaoxahexacosanoate (CB00-3) and 10 mL of DMF were added. 6.5 mL of DIPEA was dropwise added with the temperature maintained at 0-5 °C. 1 h after the addition, the mixture was stirred at room temperature for 4 h. After the completion of the reaction, DMF was removed under reduced pressure, and the residue was purified by silica gel column chromatography (with dichloromethane and methanol volume ratio 20:1 as elution solvents) to obtain CB01 in the form of a pale yellow oil (14.2 g).

### 2) Synthesis of (9H-fluoren-9-yl)methyl [(S)-1-[[(S)-1-[[4-(hydroxymethyl)phenyl] amino]-1-oxo-5-ureidopentan-2-yl]amino]-3-methyl-1-oxobutan-2-yl]carbamate (CB02).

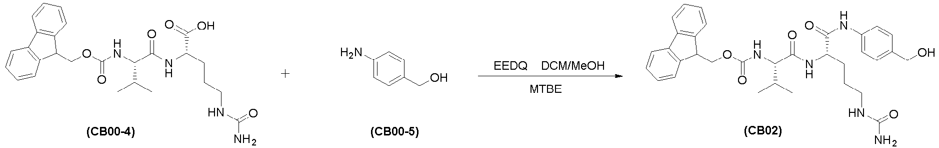

11 g of (*S*)-2-((*S*)-2-((((9*H*-fluoren-9-yl)methoxy)carbonyl)amino)-3-methylbutanamido)-5-ureidopentanoic acid (CB00-4) and 5.5 g of (4-aminophenyl)methanol (CB00-5) were dissolved in 400 mL of dichloromethane and 200 mL of methanol at room temperature under a nitrogen atmosphere, and 17 g of 2-ethoxy-1-ethoxycarbonyl-1,2-dihydroquinoline (EEDQ) were added in portions with mechanical stirring. The resulting mixture was allowed to react for 15 h in the absence of light. After the completion of the reaction, the solvent was removed under reduced pressure to obtain a paste solid, which was subjected to silica gel column chromatography (with dichloromethane and methanol volume ratio 20:1 as elution solvents) to obtain an off-white solid (11.9 g).

### 3) Synthesis of (S)-2-((S)-2-amino-3-methylbutanamido)-N-(4-(hydroxymethyl)phenyl)-5-ureidopentanamide (CB03)

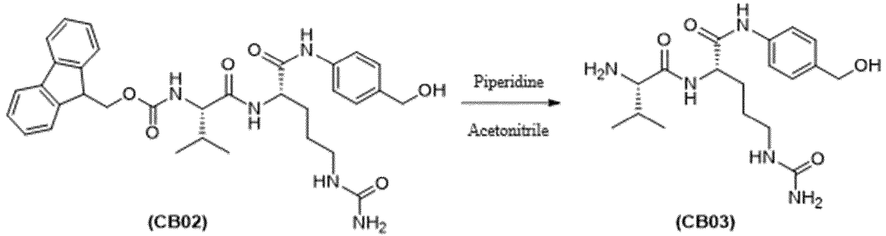

Under a nitrogen atmosphere at room temperature, 300 mL of acetonitrile was added to 11.9 g of (9H-fluoren-9-yl)methyl [(S)-1-[[(S)-1-[[4-(hydroxymethyl)phenyl]amino]-1-oxo-5-ureidopentan-2-yl]amino]-3-methyl-1-oxobutan-2-yl]carbamate (CB02), and 18 mL of piperidine was added dropwise with stirring. After the dropwise addition, the mixture was allowed to react at room temperature for 2 h. After the completion of the reaction, the mixture was distilled under reduced pressure to remove the solvent and piperidine, and the residue was subjected to silica gel column chromatography (with dichloromethane and methanol volume ratio 20:1 as elution solvents) to obtain CB03 in the form of a white solid (7.5 g).

### 4) Synthesis of (9H-fluoren-9-yl)methyl ((30S,33S,36S)-41-amino-36-((4-(hydroxymethyl)phenyl)carbamoyl)-33-isopropyl-26,31,34,41-tetraoxo-2,5,8, 11, 14, 17,20,23-octaoxa-27,32,35,40-tetraaza-30-yl)carbamate (CB04).

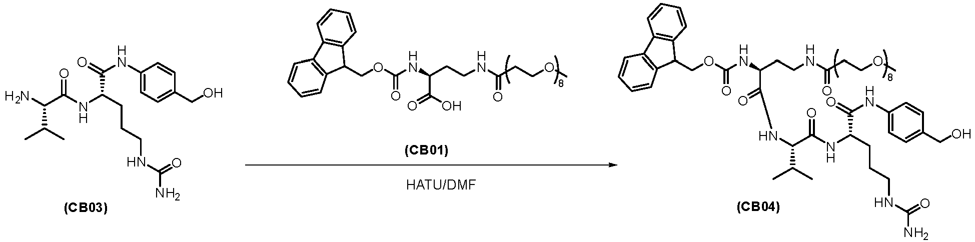

At 0 °C under a nitrogen atmosphere, 14.2 g of (*S*)-30-((((9*H*-fluoren-9-yl)methoxy)carbonyl)amino)-26-oxo-2,5,8,11,14,17,20,23-octaoxa-27-aza-hentriacontan-31-oic acid (CB01) was dissolved in 100 mL of DMF, and 11 g of *N,N,N*'*,N*'-tetramethyl-*O*-(7-azabenzotriazol-1-yl)urea hexafluorophosphate (HATU) was added in portions. After 30 min of stirring, 7.5 g of (*S*)-2-((*S*)-2-amino-3-methylbutanamido)-*N*-(4-(hydroxymethyl)phenyl)-5-ureidopentanamide (CB03) was added, and the mixture was allowed to react for 2.5 h with 0 °C maintained. After the completion of the reaction, the mixture was distilled under reduced pressure to remove the solvent, and the residue was subjected to silica gel column chromatography (with dichloromethane and methanol volume ratio 10:1 as elution solvents) to obtain CB04 in the form of a solid (9.66 g).

### 5) Synthesis of N-((S)-3-amino-4-(((S)-1-(((S)-1-((4-(hydroxymethyl)phenyl)amino)-1-oxo-5-ureidopentan-2-yl)amino)-3-methyl-1-oxobutan-2-yl)amino)-4-oxy)-2,5,8,11,14,17,20,23-octaoxahexacosan-26-amide (CB05).

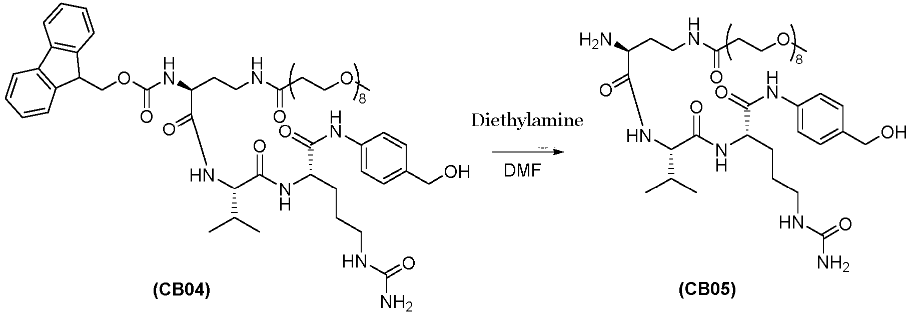

9.66 g of (9*H*-fluoren-9-yl)methyl ((30*S*,33*S*, 36S)-41-amino-36-((4-(hydroxymethyl)phenyl)carbamoyl)-33-isopropyl-26,31,34,41-tetraoxo-2,5,8, 11, 14, 17,20,23-octaoxa-27,32,35,40-tetraaza-30-yl)carbamate (CB04) was dissolved in 50 mL of DMF at room temperature under a nitrogen atmosphere, and 12 mL of diethylamine was added. The mixture was stirred for 1.5 h. After the completion of the reaction, the mixture was distilled under reduced pressure to remove the solvent, and the residue was subjected to silica gel column chromatography (with dichloromethane and methanol volume ratio 7.5:1 as elution solvents) to obtain CB05 in the form of a pale yellow solid (7.7 g).

### 6) Synthesis of N-((S)-3-(2-(2,5-dioxo-2,5-dihydro-1H-pyrrol-1-yl)acetamido)-4-(((S)-1-(((S)-1-((4-(hydroxymethyl)phenyl)amino)-1-oxo-5-ureidopentan-2-yl)amino)-3-methyl-1-butanone-2-yl)amino)-4-oxo)-2,5,8,11,14,17,20,23-octaoxahexacosan-26-amide (CB06).

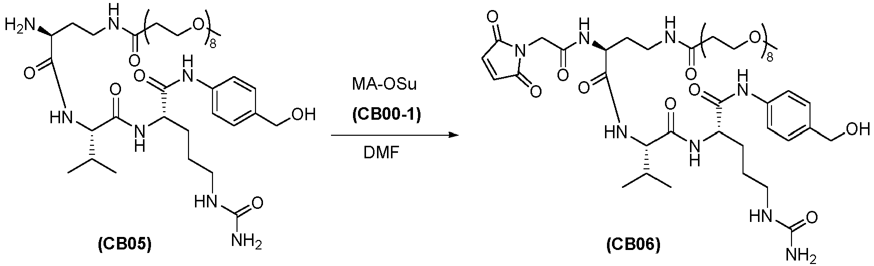

7.7 g of *N*-((*S*)-3-amino-4-(((S)-1-(((*S*)-1-((4-(hydroxymethyl)phenyl)amino)-1-oxo-5-ureidopentan-2-yl)amino)-3-methyl-1-oxobutan-2-yl)amino)-4-oxy)-2,5,8,11,14,17,20,23-octaoxahexacosan-26-amide (CB05) was dissolved in 40 mL of DMF, and succinimidyl maleimidoacetate (CB00-1) was added in portions under a nitrogen atmosphere at 0-5°C. The mixture was allowed to react at 0-5°C for 4 h. After the completion of the reaction, the mixture was distilled under reduced pressure to remove the solvent, and the residue was subjected to silica gel column chromatography (with dichloromethane and methanol as volume ratio 10:1 elution solvents) to obtain CB06 in the form of a pale yellow solid (9.5 g).

### 7) Synthesis of 4-((30S,33S,36S)-30-(2-(2,5-dioxo-2,5-dihydro-1H-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amino)benzyl (4-nitrophenyl) carbonate (CB07).

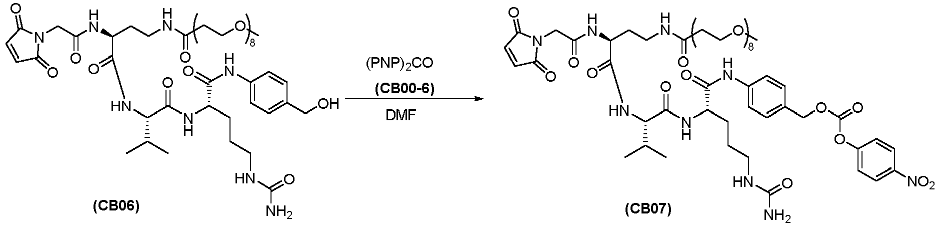

9.5 g of *N*-((*S*)-3-(2-(2,5-dioxo-2,5-dihydro-1*H*-pyrrol-1-yl)acetamido)-4-(((*S*)-1-(((S)-1-((4-(hydroxymethyl)phenyl)amino)-1-oxo-5-ureidopentan-2-yl)amino)-3-methyl-1-butanone-2-yl)amino)-4-oxo)-2,5,8,11,14,17,20,23-octaoxahexacosan-26-amide (CB06) was dissolved in 50 mL of DMF, and 14.0 g of bis(p-nitrophenyl)carbonate ((PNP)₂CO) was added under a nitrogen atmosphere at 0 °C, followed by 8.2 mL of *N,N*-diisopropylethylamine (DIPEA) after dissolution. The mixture was allowed to react for 4 h with the 0 °C maintained. After the completion of the reaction, the mixture was distilled under reduced pressure to remove the solvent, and the residue was subjected to silica gel column chromatography (with dichloromethane and methanol volume ratio 8:1 as elution solvents) to obtain CB07 in the form of a white solid (2.6 g).

### Example 4. Synthesis of 4-((18S,21S,24S)-18-(2-(2,5-dioxo-2,5-dihydro-1H-pyrrol-1-yl)acetamido)-21-isopropyl-14,19,22-trioxo-24-(3-ureidopropyl)-2,5,8,11-tetraoxo-15,20,23-triazapentacosan-25-amino)benzyl(4-nitrophenyl)carbonate (CB14).

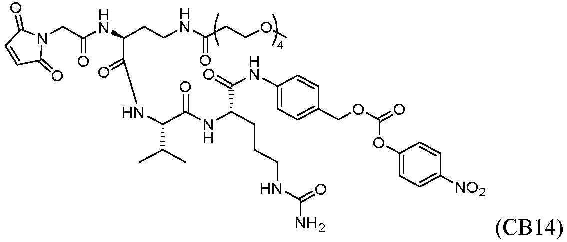

CB14 was prepared by referring to the synthesis of CB07 in Example 3, with *N*-succinimidyl 4,7,10,13,16,19,22,25-octaoxahexacosanoate replaced by *N*-succinimidyl 4,7,10,13-tetraoxatetradecanoate. CB14 was eventually obtained in the form of a white solid.

### Example 5. Synthesis of Intermediate (CB07-Exatecan)

### Synthesis of 4-((30S,33S,36S)-30-(2-(2,5-dioxo-2,5-dihydro-1H-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl ((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate.

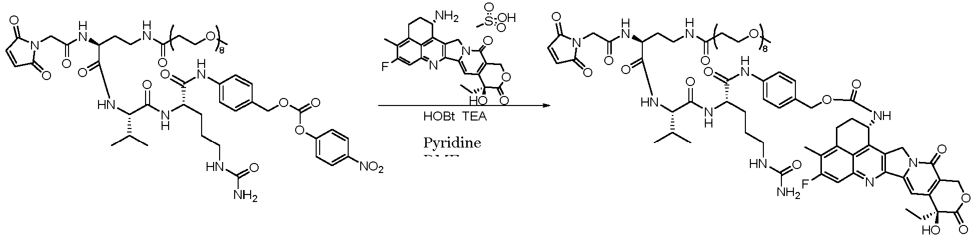

4-((30S,33 S,36S)-30-(2-(2,5-dioxo-2,5-dihydro-1H-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl (4-nitrophenyl) carbonate (CB07, 2.6 g, 2.21 mmol) and N,N-dimethylformamide (23 mL) were added to a reaction flask R1, stirred under a nitrogen atmosphere and cooled to 0-5 °C. At the same time, (1S,9S)-1-amino-9-ethyl-5-fluoro-9-hydroxy-4-methyl-1,2,3,9,12,15-hexahydro-10H,13H-benzo[3',4':6,7]indolizino[1,2-b]quinolin-10,13-dione methanesulfonate (Exatecan, 0.98 g, 1.84 mmol, Advanced ChemBlocks) and N,N-dimethylformamide (5 mL) were taken and added to another reaction flask R2. Triethylamine (230 mg, 2.27 mmol) was added dropwise at 0-5 °C. The mixture was stirred until complete dissolution was achieved. The solution in reaction flask R2 was added dropwise to reaction flask R1. Reaction flask R2 was then washed with N,N-dimethylformamide (2 mL), and the washing solution was added to reaction flask R1. 1-Hydroxybenzotriazole (497 mg, 3.68 mmol) and pyridine (1.45 g, 18.4 mmol) were weighed into reaction flask R1. The mixture was stirred at 0-5°C for 10 min, warmed to room temperature, and stirred for 5.5 h. After the reaction was completed, the mixture was concentrated at 35 °C under reduced pressure to remove the solvent. The residue was purified by preparative high performance liquid chromatography (prep-HPLC) and lyophilized to obtain a white powder (1.6 g, 59%). LC-MS: [1/2M+H]⁺=737.

### Example 6. Synthesis of Intermediate (CB07-D-1)

### Synthesis of 4-((30S,33S,36S)-30-(2-(2,5-dioxo-2,5-dihydro-1H-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl ((1S,9S)-9-ethyl-4,5-difluoro-9-hydroxy-10,13-dioxo-2,3,9,10,13,15-hexahydro-1H,12H-benzo[de]pyrano[3',4':6,7]indolizino[1,2-b]quinolin-1-yl)carbamat.

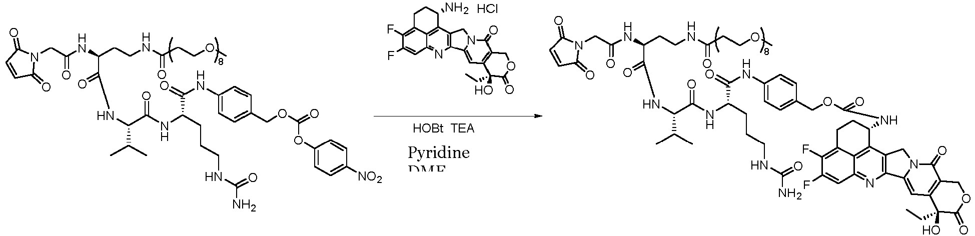

4-((30S,33S,36S)-30-(2-(2,5-dioxo-2,5-dihydro-1*H*-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl(4-nitrocarbonate) (CB07, 220 mg, 0.189 mmol) and *N,N*-dimethylformamide (5 mL) were added to a reaction flask R1, stirred under a nitrogen atmosphere and cooled to 0-5 °C. At the same time, (1*S*,9*S*)-1-amino-9-ethyl-4,5-difluoro-9-hydroxy-1,2,3,9,12,15-hexahydro-10*H*,13*H-*benzo[*de*]pyrano[3',4':6,7]indolizino[1,2-b]quinolin-10,13-dione hydrochloride (D-1) (90 mg, 0.189 mmol) and *N,N*-dimethylformamide (5 mL) were taken and added to another reaction flask R2. Three drops of triethylamine were added at 0-5 °C. The mixture was stirred until complete dissolution was achieved. The solution in reaction flask R2 was added dropwise to reaction flask R1, and 1-hydroxybenzotriazole (60 mg, 0.44 mmol) and pyridine (0.5 mL) were weighed into reaction flask R1. The mixture was stirred at 0-5°C for 10 min, warmed to room temperature, and stirred for 3 h. After the reaction was completed, the mixture was concentrated under reduced pressure to remove the solvent. The residue was purified by preparative high performance liquid chromatography (prep-HPLC) and lyophilized to obtain a white powder (55 mg, 20%). LC-MS: [1/2M+H]⁺=739.

### Example 7. Synthesis of Intermediate (CB14-Exatecan)

### Synthesis of 4-((18S,21S,24S)-18-(2-(2,5-dioxane-2,5-dihydro-1H-pyrrol-1-yl)acetamido)-21-isopropyl-14,19,22-trioxo-24-(3-ureidopropyl)-2,5,8,11-tetraoxo-15,20,23-triazapentacosan-25-amino)-(1S,9S)-9-ethyl-5-fluoro-9-hydroxy-4-methyl-10,13-dioxo-2,3,9,10,13,15-hexahydro-1H,12H-benzo[de]pyran[3',4':6,7]indolizino[1,2-b]quinolin-1-carbamate.

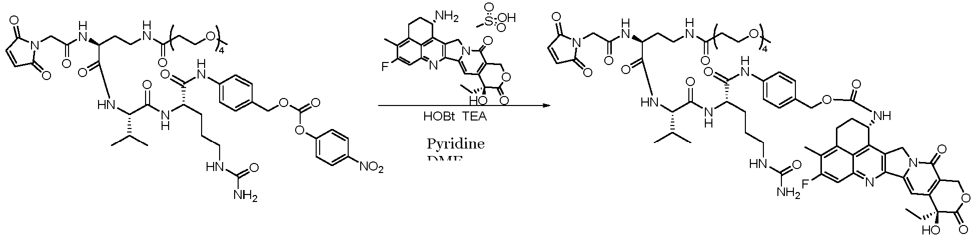

Similarly, 4-((18*S*,21*S*,24*S*)-18-(2-(2,5-dioxo-2,5-dihydro-1*H*-pyrrol-1-yl)acetamido)-21-isopropyl-14,19,22-trioxo-24-(3-ureidopropyl)-2,5,8,11-tetraoxo-15,20,23-triazapentacosan-25-amino)benzyl(4-nitrophenyl)carbonate (190 mg, 0.19 mmol)and *N,N*-dimethylformamide (23 mL) were added to a reaction flask R1, stirred under a nitrogen atmosphere, and cooled to 0-5 °C. At the same time, (1*S*,9*S*)-1-amino-9-ethyl-5-fluoro-9-hydroxy-4-methyl-1,2,3,9,12,15-hexahydro-10*H*,13*H-*benzo[3',4':6,7]indolizino[1,2-*b*]quinolin-10,13-dione methanesulfonate (Exatecan methanesulfonate; 101 mg, 0.19 mmol; Advanced ChemBlocks) and *N,N*-dimethylformamide (5 mL) were taken and added to another reaction flask R2. Triethylamine (3 drops) was added dropwise at 0-5 °C. The mixture was stirred until complete dissolution was achieved. The solution in reaction flask R2 was added dropwise to reaction flask R1. Reaction flask R2 was then washed with *N,N*-dimethylformamide (1 mL), and the washing solution was added to reaction flask R1. 1-hydroxybenzotriazole (60 mg, 0.44 mmol) and pyridine (0.5 mL) were weighed into reaction flask R1. The mixture was stirred at 0-5 °C for 10 min, warmed to room temperature, and stirred for 5.5 h. After the reaction was completed, the mixture was concentrated at 35 °C under reduced pressure to remove the solvent. The residue was purified by preparative high performance liquid chromatography (prep-HPLC) and lyophilized to obtain a white powder.

### Example 8. Synthesis of ADC1 (Abu is the antibody H239H-2b-K-6a-1)

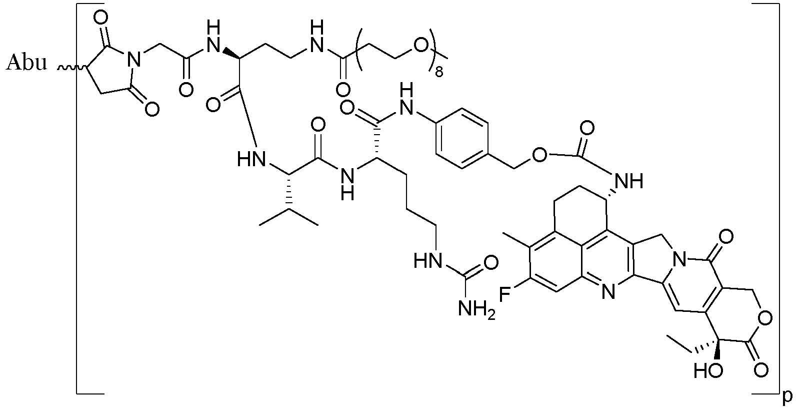

100 µl of 0.1M aqueous EDTA solution was added to 5 ml of 11.5 mg/ml Antibody H239H-2b-K-6a-1 solution, and 2.7 molar equivalents of 10 mM aqueous TCEP solution was added according to the amount of antibody substance. The solution was reacted in an incubator at 25 °C for 2 hours, and the reducing agent TCEP in the solution was removed by ultrafiltration. Then, according to the amount of the antibody substance, 6 molar equivalents of 10 mM solution of a CB07-Exatecan in dimethylacetamide (DMA) was added for conjugating, and the organic solvent DMA was added until its volume accounted for 20% of the conjugating system. The system was reacted in an incubator at 25 °C for 2 hours and 15 minutes, and then a 0.1 M aqueous NAC (N-acetylcysteine) solution was added until its final concentration reached 1.5 mM. The system was then reacted for 15 min to terminate the reaction.

After purification, the DAR, i.e., p, of ADC1 was about 4.05 as measured by RP-UPLC.

### Example 9. Synthesis of ADC2 (Abu is the antibody H239H-2b-K-6a-1)

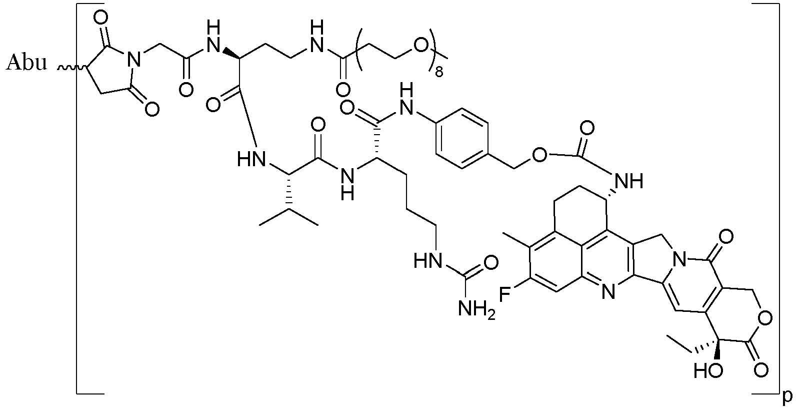

326 µl of 0.1M aqueous EDTA solution was added to 15.2 ml of 11.5 mg/ml Antibody H239H-2b-K-6a-1 solution, and 7 molar equivalents of 10 mM aqueous TCEP solution was added according to the amount of antibody substance. The solution was reacted in an incubator at 25 °C for 2 hours, and the reducing agent TCEP in the solution was removed by ultrafiltration. Then, according to the amount of the antibody substance, 12 molar equivalents of 10 mM solution of a CB07-Exatecan in dimethylacetamide (DMA) was added for conjugating, and the organic solvent DMA was added until its volume accounted for 20% of the conjugating system. The system was reacted in an incubator at 25 °C for 2.5 hours, and then a 0.1 M aqueous NAC solution was added until its final concentration reached 2 mM. The system was then reacted for 15 min to terminate the reaction.

After purification, the DAR, i.e., p, of ADC2 was about 7.49 as measured by RP-UPLC.

### Example 10. Synthesis of ADC3 (Abu is the antibody H239H-2b-K-6a-1)

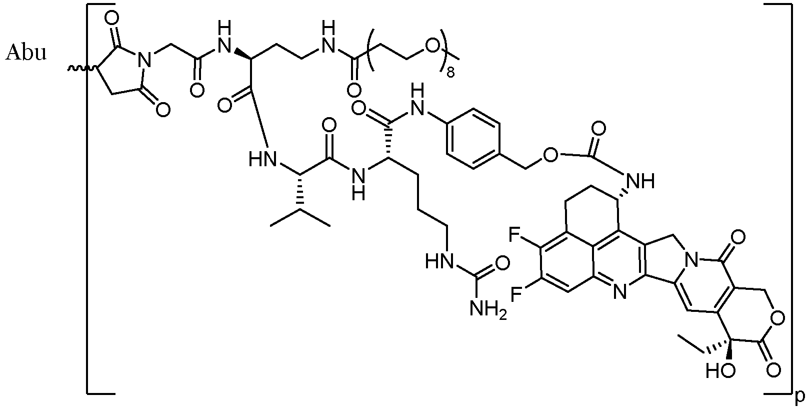

50 µl of 0.1M aqueous EDTA solution was added to 2.5 ml of 11.5 mg/ml Antibody H239H-2b-K-6a-1 solution, and 6 molar equivalents of 15 mM aqueous TCEP solution was added according to the amount of antibody substance. The solution was reacted in an incubator at 25 °C for 2 hours, and the reducing agent TCEP in the solution was removed by ultrafiltration. Then, according to the amount of the antibody substance, 12 molar equivalents of 10 mM solution of a CB07-D-1 in dimethylacetamide (DMA) was added for conjugating, and the organic solvent DMA was added until its volume accounted for 20% of the conjugating system. The system was reacted in an incubator at 25 °C for 2.5 hours, and then a 0.1 M aqueous NAC solution was added until its final concentration reached 2 mM. The system was then reacted for 15 min to terminate the reaction.

After purification, the DAR, i.e., p, of ADC3 was about 7.65 as measured by RP-UPLC.

### Example 11. Synthesis of ADC4 (Abu is the antibody H239H-2b-K-6a-1)

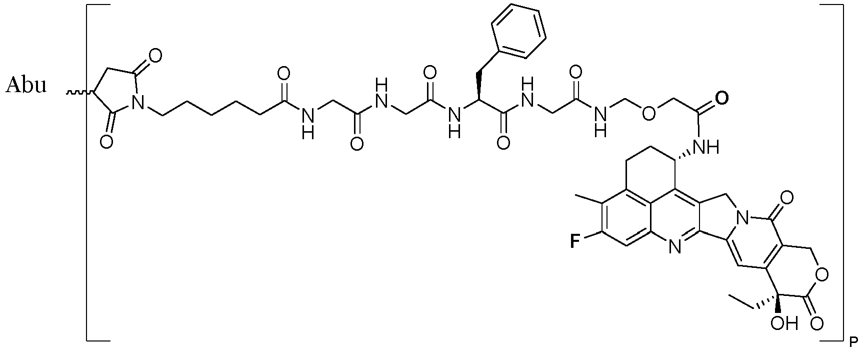

100 µl of 0.1M aqueous EDTA solution was added to 5 ml of 11.5 mg/ml Antibody H239H-2b-K-6a-1 solution, and 2.7 molar equivalents of 10 mM aqueous TCEP solution was added according to the amount of antibody substance. The solution was reacted in an incubator at 25 °C for 2 hours, and the reducing agent TCEP in the solution was removed by ultrafiltration. Then, according to the amount of the antibody substance, 6 molar equivalents of 10 mM solution of a MC-GGFG-DXD (Deruxtecan, MCE, Cat.#HY-13631E/CS-0045125) in dimethylacetamide (DMA) was added for conjugating, and the organic solvent DMA was added until its volume accounted for 20% of the conjugating system. The system was reacted in an incubator at 25 °C for 2 hours, and then a 0.1 M aqueous NAC solution was added until its final concentration reached 1.5 mM. The system was then reacted for 15 min to terminate the reaction.

After purification, the DAR, i.e., p, of ADC4 was about 3.43 as measured by RP-UPLC.

### Example 12. Synthesis of ADC5 (Abu is the antibody H239H-2b-K-6a-1)

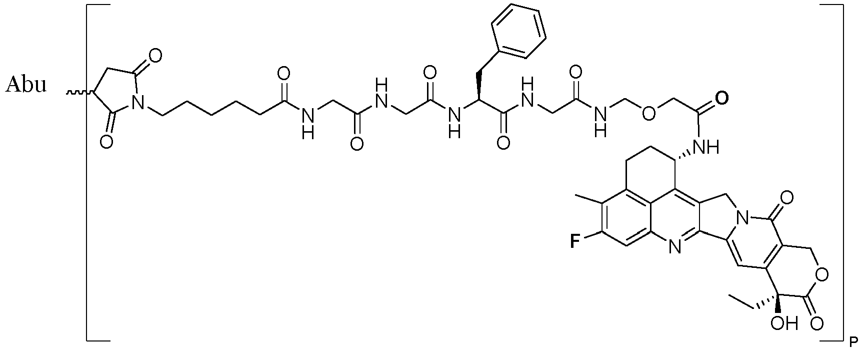

100 µl of 0.1M aqueous EDTA solution was added to 5 ml of 11.5 mg/ml Antibody H239H-2b-K-6a-1 solution, and 7 molar equivalents of 10 mM aqueous TCEP solution was added according to the amount of antibody substance. The solution was reacted in an incubator at 25 °C for 2 hours, and the reducing agent TCEP in the solution was removed by ultrafiltration. Then, according to the amount of the antibody substance, 12 molar equivalents of 10 mM solution of a MC-GGFG-DXD (Deruxtecan, MCE, Cat.#HY-13631E/CS-0045125) in dimethylacetamide (DMA) was added for conjugating, and the organic solvent DMA was added until its volume accounted for 20% of the conjugating system. The system was reacted in an incubator at 25 °C for 2 hours, and then a 0.1 M aqueous NAC solution was added until its final concentration reached 2 mM. The system was then reacted for 15 min to terminate the reaction.

After purification, the DAR, i.e., p, of ADC5 was about 7.67 as measured by RP-UPLC.

### Example 13. Synthesis of ADC6 (Abu is the antibody H239H-2b-K-6a-2)

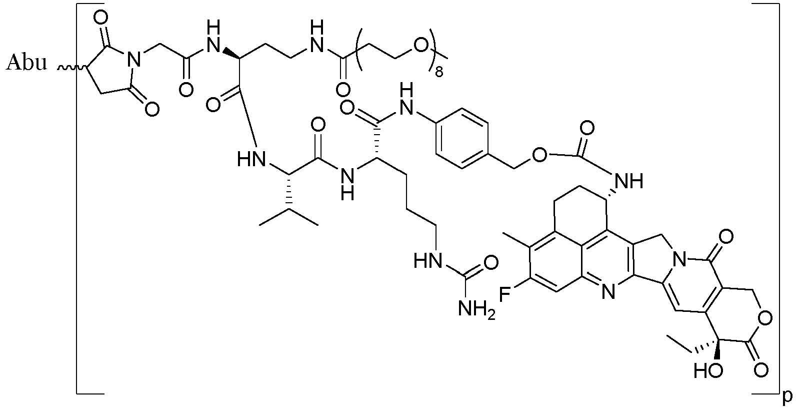

60 µl of 0.1M aqueous EDTA solution was added to 3 ml of 7.89 mg/ml Antibody H239H-2b-K-6a-2 solution, and 7 molar equivalents of 10 mM aqueous TCEP solution was added according to the amount of antibody substance. The solution was reacted in an incubator at 25 °C for 2 hours, and the reducing agent TCEP in the solution was removed by ultrafiltration. Then, according to the amount of the antibody substance, 15 molar equivalents of 100 mM solution of a CB07-Exatecan in dimethylacetamide (DMA) was added for conjugating, and the organic solvent DMA was added until its volume accounted for 20% of the conjugating system. The system was reacted in an incubator at 25 °C for 2 hours, and then a 0.1 M aqueous NAC solution was added until its final concentration reached 2 mM. The system was then reacted for 15 min to terminate the reaction.

After purification, the DAR, i.e., p, of ADC6 was about 7.19 as measured by RP-UPLC.

### Example 14. Binding of anti-CLDN18.2 antibodies to CLDN18.2 expressed on cell surface

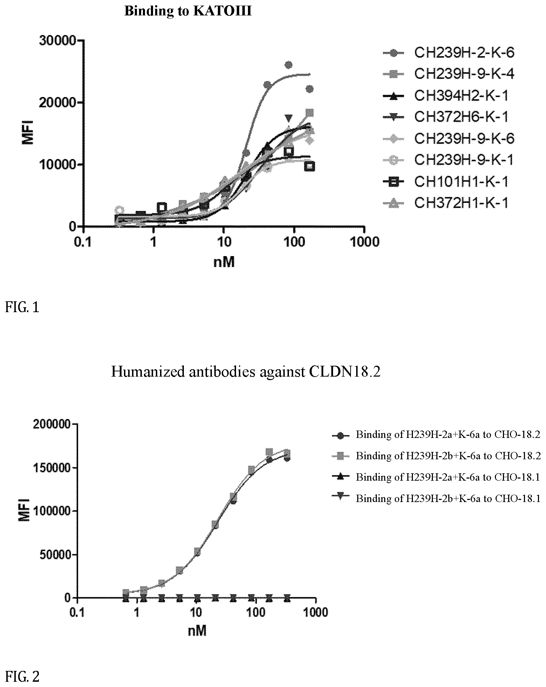

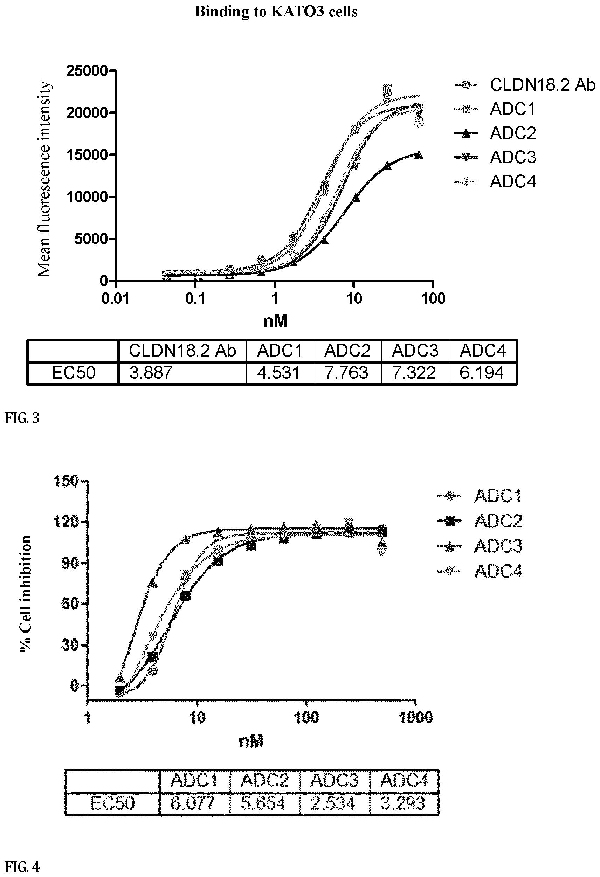

Flow cytometry was used to detect the binding capacity of anti-CLDN18.2 antibodies and ADCs to cells expressing CLDN18.2, and the specificity of not binding to CLDN18.1 of the antibody was further detected. The experimental procedure overview was as follows: CLDN18.2 positive cells (KATOIII or CHO-CLDN18.2) and negative cells (CHO-CLDN18.1) were collected, washed once with PBS, resuspended in PBS, and plated on a 96-well V-bottom plate (Cat. No. 3897, Costar) at 0.1 million cells/50 µL/well. The antibodies and ADCs were separately pre-diluted to 333.2 nM with PBS, then diluted 2-fold with 9 gradients, added at 50 µL/well to the cells in the 96-well V-bottom plate described above, and mixed well (final antibody or ADC concentrations: 166.6, 83.3, 41.6, 20.8, 10.4, 5.2, 2.6, 1.3, 0.65, and 0.325 nM). The mixture was incubated at 4 °C for 1 h and washed twice with PBS. Then 100 µL of 1:500 diluted goat anti-human IgG-Fc PE fluorescent secondary antibody (Cat. No. 12-4998-82, eBioscience) was added. The mixture was incubated at 4 °C for 1 h, washed twice with PBS, and analyzed with a CytoFLEX flow cytometer (model: AOO-1-1102, Beckman).
1) The detection of the chimeric antibodies and the cells expressing CLDN18.2 is shown in FIG. 1. The chimeric antibodies CH239H-2-K-6, CH239H-9-K-4, CH394H2-K-1, CH372H6-K-1, CH239H-9-K-6, CH239H-9-K-1, CH101H1-K-1, and CH372H1-K-1 all could bind to CLDN18.2 on KATOIII cells, wherein the binding activity of the chimeric Antibody CH239H-2-K-6 was better.
2) The binding capacity of the chimeric Antibody CH239H-2-K-6 to CLDN18.2 on KATOIII cells was better than that of the positive Antibody IMAB362(the sequence of which is the same as that of the antibody expressed by hybridoma cells 175D10 in Patent No. US20090169547): the EC₅₀ values of IMAB362 and CH239H-2-K-6 were 8.082 nM and 6.290 nM, respectively.
3) After the chimeric Antibody CH239H-2-K-6 was humanized, the binding capacity of the humanized antibodies H239H-2a-K-6a and H239H-2b-K-6a to CLDN18.2 positive cells KATOIII and CHO-CLDN18.2 was substantially identical to that of the chimeric Antibody CH239H-2-K-6, and the affinity was not reduced; the Antibody CH239H-2-K-6, the Antibody H239H-2a-K-6a, and the Antibody H239H-2b-K-6a bound to KATOIII cells with EC₅₀ values of 6.108 nM, 6.203 nM, and 6.920 nM, respectively; the Antibody CH239H-2-K-6, the Antibody H239H-2a-K-6a, the Antibody H239H-2b-K-6a, and the positive Antibody IMAB362 bound to CHO-CLDN18.2 cells with EC₅₀ values of 19.92 nM, 22.83 nM, 23.31 nM, and 30.02 nM, respectively.
4) As shown in FIG. 2, the humanized Antibodies H239H-2a-K-6a and H239H-2b-K-6a bound only to CLDN18.2 and not to CLDN18.1.
5) As shown in FIG. 3, the EC₅₀ value for the binding of humanized antibody H239H-2b-K-6a-1 to KATOIII cells was 3.887 nM; the EC₅₀ values for the binding of ADC1, ADC2, ADC3, and ADC4 to KATOIII cells were 4.531 nM, 7.763 nM, 7.322 nM, and 6.194 nM, respectively.

Construction of CHO-CLDN18.2 stable cell line :

The full-length amino acid sequence of human CLDN18.2 (from NCBI, NM_001002026.3) is as follows:

MAVTACQGLGFVVSLIGIAGIIAATCMDQWSTQDLYNNPVTAVFNYQGLWRSCVRESSGFTE
CRGYFTLLGLPAMLQAVRALMIVGIVLGAIGLLVSIFALKCIRIGSMEDSAKANMTLTSGIMFI
VSGLCAIAGVSVFANMLVTNFWMSTANMYTGMGGMVQTVQTRYTFGAALFVGWVAGGLT
LIGGVMMCIACRGLAPEETNYKAVSYHASGHSVAYKPGGFKASTGFGSNTKNKKIYDGGAR
TEDEVQSYPSKHDYV (SEQ ID NO: 85).

The corresponding nucleic acid sequence is as follows:

atggccgtgactgcctgtcagggcttggggttcgtggtttcactgattgggattgcgggcatcattgctgccacctgcatggaccagtggagcaccca
agacttgtacaacaaccccgtaacagctgttttcaactaccaggggctgtggcgctcctgtgtccgagagagctctggcttcaccgagtgccggggc
tacttcaccctgctggggctgccagccatgctgcaggcagtgcgagccctgatgatcgtaggcatcgtcctgggtgccattggcctcctggtatccat
ctttgccctgaaatgcatccgcattggcagcatggaggactctgccaaagccaacatgacactgacctccgggatcatgttcattgtctcaggtctttgt
gcaattgctggagtgtctgtgtttgccaacatgctggtgactaacttctggatgtccacagctaacatgtacaccggcatgggtgggatggtgcagact
gttcagaccaggtacacatttggtgcggctctgttcgtgggctgggtcgctggaggcctcacactaattgggggtgtgatgatgtgcatcgcctgccg
gggcctggcaccagaagaaaccaactacaaagccgtttcttatcatgcctcaggccacagtgttgcctacaagcctggaggcttcaaggccagcac
tggctttgggtccaacaccaaaaacaagaagatatacgatggaggtgcccgcacagaggacgaggtacaatcttatccttccaagcacgactatgt
g (SEQ ID NO: 86).

The nucleic acid sequence corresponding to human CLDN18.2 described above was synthesized, and sequences of enzyme digestion sites HindIII and EcoRI were added at both ends of the sequence. The sequence was then constructed into the pcDNA3.1 expression vector (Invitrogen, Cat. No. V79020). The expression vector was transfected into CHO cells (Life technologies, Cat. No. A13696-01) by electroporation. The electroporation conditions were as follows: voltage 300 V, time 17 ms, and 4 mm electroporation cuvette. After 48 h, 50 µM MSX (methionine iminosulfone) was added as a screening pressure, and positive cells were selected after 2 weeks. High-expression cell lines were selected by FACS. The cells were collected and washed once with PBS (phosphate-buffered saline, 1 L of PBS containing 8.0 g of NaCl, 0.9 g of Na₂HPO₄, 0.156 g of KH₂PO₄, and 0.125 g of KCl; pH 7.2-7.4). 3 µg/mL IMAB362 antibody was added, and the mixture was incubated at 4 °C for 1 h and washed twice with PBS. 100 µL of 1:500 diluted goat anti-human IgG-Fc PE fluorescent secondary antibody (Cat. No. 12-4998-82, eBioscience) was added, and the mixture was incubated at 4 °C for 1 h, washed twice with PBS, and analyzed by a C6 flow cytometer (BD, model: C6 flow cytometer). The cells obtained were named CHO-CLDN18.2 cells.

Construction of CHO-CLDN18.1 stable cell line :
The full-length amino acid sequence of human CLDN18.1 (from NCBI, NM_016369.4) is as follows:

MSTTTCQVVAFLLSILGLAGCIAATGMDMWSTQDLYDNPVTSVFQYEGLWRSCVRQSSGFTE
CRPYFTILGLPAMLQAVRALMIVGIVLGAIGLLVSIFALKCIRIGSMEDSAKANMTLTSGIMFIV
SGLCAIAGVSVFANMLVTNFWMSTANMYTGMGGMVQTVQTRYTFGAALFVGWVAGGLTLI
GGVMMCIACRGLAPEETNYKAVSYHASGHSVAYKPGGFKASTGFGSNTKNKKIYDGGARTE
DEVQSYPSKHDYV (SEQ ID NO: 87).

The corresponding nucleic acid sequence is as follows:

atgtccaccaccacatgccaagtggtggcgttcctcctgtccatcctggggctggccggctgcatcgcggccaccgggatggacatgtggagcac
ccaggacctgtacgacaaccccgtcacctccgtgttccagtacgaagggctctggaggagctgcgtgaggcagagttcaggcttcaccgaatgca

ggccctatttcaccatcctgggacttccagccatgctgcaggcagtgcgagccctgatgatcgtaggcatcgtcctgggtgccattggcctcctggta
tccatctttgccctgaaatgcatccgcattggcagcatggaggactctgccaaagccaacatgacactgacctccgggatcatgttcattgtctcaggt
ctttgtgcaattgctggagtgtctgtgtttgccaacatgctggtgactaacttctggatgtccacagctaacatgtacaccggcatgggtgggatggtgc
agactgttcagaccaggtacacatttggtgcggctctgttcgtgggctgggtcgctggaggcctcacactaattgggggtgtgatgatgtgcatcgcc
tgccggggcctggcaccagaagaaaccaactacaaagccgtttcttatcatgcctcaggccacagtgttgcctacaagcctggaggcttcaaggcc
agcactggctttgggtccaacaccaaaaacaagaagatatacgatggaggtgcccgcacagaggacgaggtacaatcttatccttccaagcacga
ctatgtg (SEQ ID NO: 88).

The nucleic acid sequence corresponding to human CLDN18.1 described above was synthesized, and sequences of enzyme digestion sites HindIII and EcoRI were added at both ends of the sequence. The sequence was then constructed into the pcDNA3.1 expression vector. The expression vector was transfected into CHO cells by electroporation. The electroporation conditions were as follows: voltage 300 V, time 17 ms, and 4 mm electroporation cuvette. After 48 h, 50 µM MSX was added as a screening pressure, and positive cells were selected after 2 weeks. DotBlot hybridization was used for detection. The cells were collected, and the cell lysate supernatant was extracted, with blank CHO cell lysate supernatant as negative control and CHO-CLDN18.2 cell lysate supernatant as positive control. 20 µL of cell lysate was taken, spotted on an activated PVDF membrane (millipore), and left to stand for 3-5 min so that the membrane fully adsorbed the proteins. The membrane was soaked in 5% skimmed milk powder, blocked at 37 °C for 2 h, and washed thrice with PBST (PBS + 0.05% Tween). Then the membrane was soaked in rabbit anti-human Claudin18 antibody (Cat. No. ab230224, abcam, the antibody can recognize the same intracellular segment of CLDN18.1 and CLDN18.2, so CHO-CLDN18.2 cells can be used as positive control), incubated for 2 h at room temperature, and washed thrice with PBST. Then the membrane was soaked in goat anti-rabbit HRP antibody (Cat. No. ab6721, abcam), incubated for 2 h at room temperature, and washed thrice with PBST. Then DAB (Cat. No. AR1000, Boster Biological Technology) was used for color development. The cells obtained were named CHO-CLDN18.1 cells.

### Example 15. ADCC (Antibody Dependent Cell-Mediated Cytotoxicity) Activity of Anti-CLDN18.2 Antibodies

ADCC Reporter Bioassay was selected in this experiment to evaluate ADCC activity of CLDN18.2 antibodies. This is a bioluminescent reporter gene assay system, which replaces NK cells using artificially constructed effector cells and quantifies the biological activity of the therapeutic antibody medicine based on the ADCC mechanism of action in the activation pathway with a high-sensitivity detection reagent, is a mechanism of action detection method. The cells expressing CLDN18.2 (CHO-CLDN18.2) were used as target cells and Jurkat-NFAT-luc-FcγRIIIa as effector cells (FcγRIIIa (sequence from NCBI, NP_001121065.1) was transfected into Jurkat (Cell Bank of Chinese Academy of Sciences, Shanghai, Clone E6-1) cells by lentivirus infection, and a positive clone was selected using 0.3 µg/mL puromycin (Cat. No. A11138-03, Gibco) and named Jurkat-FcγRIIIa; then the plasmid pGL4.30 [luc2P/NFAT-RE/Hygro] (Cat. No. E848A, Promega) containing a fluorescein reporter gene was transfected into the Jurkat-FcγRIIIa cells by electrotransfection, a positive clone was selected using hygromycin B (Cat. No. A600230-0001, Sangon Biotech), and the cells finally obtained were named Jurkat-NFAT-luc-FcγRIIIa), and the detection was performed using ONE-Glo^{™} Luciferase Assay System (Cat. No. E6120-100ml, Promega). The specific procedures were as follows: The CHO-CLDN18.2 cells were collected and washed once with RPMI1640 medium. Then the cell density was adjusted to 1.2 × 10⁶ cells/mL with RPMI1640 medium. The cells were inoculated in a 96-well white plate (Cat. No. 3917, Costar) at 25 µL/well, i.e., 30 thousand cells/well, and the plate was gently tapped to well mix the cells. The antibodies were pre-diluted to 10 µg/mL using an RPMI1640 medium containing 1% FBS as the antibody diluent, then diluted 5-fold with 8 gradients, and added to the cell plate described above at 50 µL/well. The plate was gently tapped to well mix the mixture. Then Jurkat-NFAT-lue-FcγRIIIa cells in the logarithmic phase were collected and washed once with RPMI1640 medium. Then the cell density was adjusted to 2.4 × 10⁶ cells/mL with RPMI1640 medium. The cells were added to the cell culture plate described above at 25 µL/well, i.e., 60 thousand cells/well, and mixed well. The culture plate was cultured in a 37 °C/5% CO₂ incubator for 6 h, and then taken out and placed at room temperature for 10 min to return to room temperature. 50 µL of ONE-GLO substrate (Cat. No. E6120-100ml, promega) was added. The culture plate was tested in a microplate reader (model: SpectraMax M3, Molecular Devices) to read the relative fluorescence unit (RLU).

CHO-CLDN18.1 cells were used as cell negative control, IMAB362 as antibody positive control, and the IgG isotype (Cat. No. BE0297, Bio cell) as antibody negative control.

The results show that the Antibody H239H-2b-k-6a had a strong ADCC effect, and the Antibody H239H-2b-k-6a had a stronger ADCC effect than the positive Antibody IMAB362: the EC₅₀ values corresponding to the Antibody IMAB362 and the Antibody H239H-2b-k-6a were 0.7953 nM and 0.06253 nM, respectively.

### Example 16. CDC (Complement Dependent Cytotoxicity) Activity of Anti-CLDN18.2 Antibodies

The CHO-CLDN18.2 cells were collected and washed once with PBS. The cell density was adjusted to 6.6 × 10⁵ cells/mL with RPMI1640 medium. The cells were plated on a 96-well flat-bottom plate at 45 µL/well. The antibodies were pre-diluted to 50 µg/mL using an RPMI1640 medium, and then diluted 4-fold with 9 gradients, and wells without added antibodies were used as control wells. Human serum complement (Cat. No. Quidel, A113) with a final concentration of 5% was used as a complement source, followed by incubation at 37 °C for 5 h. CCK-8 reagent (Cat. No. CK04, DojinDo) was added. The number of live cells was detected, and the cell killing rate [% cell killing rate = (1 - antibody well reading / control well reading) × 100] was calculated.

The results show that the Antibody H239H-2b-k-6a had a significant CDC effect in the presence of complement, and the Antibody H239H-2b-k-6a had a stronger CDC effect than the positive Antibody IMAB362: the IC₅₀ values corresponding to the Antibody IMAB362 and the Antibody H239H-2b-k-6a were 5.867 nM and 0.2909 nM, respectively.

### Example 17. Assay for proliferation inhibition by anti-CLDN18.2 ADCs

The anti-CLDN18.2 ADC was incubated with cells expressing CLDN18.2, and the inhibition rate was determined by detecting the number of viable cells using CCK-8 (Cat. No. CK04, DojinDo) reagent. The procedures were as follows: The CHO-CLDN18.2 cells in the logarithmic growth phase were collected, centrifuged at 800 rpm for 5 minutes, removed the supernatant, washed once with CD-CHO-AGT (Cat. No. 12490; life technologies) culture medium, centrifuged at 800 rpm for 5 minutes again, and removed the supernatant. The cells were resuspend with 0.5% FBS (Cat. No. FSP500; Excell Bio) CD-CHO-AGT medium, and the cells density were adjusted to 5×10⁴ cells/ml, 100 µl/well were spread in 96-well cell culture plate (Cat. No. 3599; costar). 100 µl of ADC diluted with 0.5% FBS CD-CHO-AGT gradient was added to each well, and the final concentrations of ADC were: 500, 250, 125, 62.5, 31.25, 15.625, 7.813, 3.9, 1.95 nM. After 5 days of culture in a 37 degree 5% CO₂ incubator, 20 µl of CCK-8 was added to each well. After incubating for 1 hour at 37 °C, the OD450 nm absorbance value was measured using a microplate reader (SpectraMax M3, Molecular Devices) to calculate the cell inhibition rate. Inhibition rate% = (1-absorbance value of sample well/absorbance value of control well) ×100.

The results show that different ADCs had obvious inhibitory effect on the proliferation of CHO-CLDN18.2 cells, as shown in FIG. 4.

### Example 18. Bystander effect of ADC

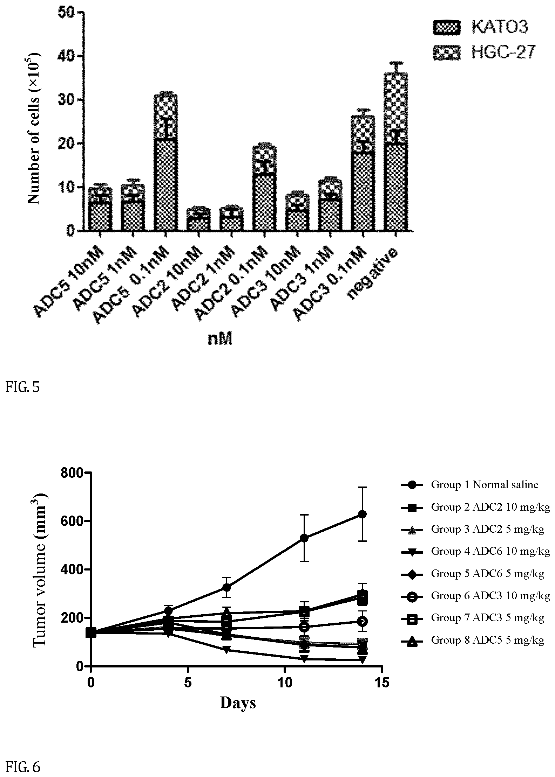

Under the condition of co-cultivating the CLDN18.2 positive cells KATOIII and the negative cells HGC-27 in vitro, the killing effect of different ADCs on these two cells was observed. The procedures were as follows: The KATOIII cells (the CLDN18.2 positive cells) and the HGC-27 cells (the CLDN18.2 negative cells) in the logarithmic growth phase were collected, resuspended with 2% FBS RPMI 1640, and spread in 6-well plate (Cat. No. 3516; costar), 120,000 cells per well for KATOIII and 80,000 cells per well for HCG-27. After culture overnight in a 37 degree 5% CO₂ incubator, 3ml of ADC9, ADC11, ADC12 were added the next day, and the final concentrations of the ADC were 10 nM, 1 nM and 0.1 nM with no ADC wells added as negative controls. After 5 days of culture in a 37 degree 5% CO₂ incubator, the cells were digested with trypsin ( Cat. No. 25200-072; gibco), and calculated the total number of living cells. In order to determine the ratio of KATOIII and HGC-27 in the total surviving cells, the cells were stained with an FITC-labeled anti-Trop2 antibody (The heavy chain and light chain sequences of the anti-Trop2 antibody were SEQ ID NO: 83 and SEQ ID NO: 84, respectively, as shown in Table 7) (KATOIII was the Trop2 positive cells) and incubated on ice for 30min. After washing, the fluorescent signal of the stained cells was measured using a CytoFLEX flow cytometer (model: AOO- 1-1102, Beckmann). According to the number and ratio of the KATOIII-positive and the HGC-27-negative cells in each treatment well, the number of KAOT3 and HGC-27 cells was calculated, and the results were shown in FIG. 5.

The results show that different ADCs had killing effects on both the positive and negative cells, with ADC2 having the strongest bystander effect on negative cells.

**Table 7. Amino acid sequence of anti-Trop2 antibody**

| Name | Sequence No. | Amino acid sequence |
|---|---|---|
| Heavy chain | 83 | QVQLQQSGSELKKPGASVKVSCKASGYTFTNYGMNWVKQAPGQGL KWMGWINTYTGEPTYTDDFKGRFAFSLDTSVSTAYLQISSLKADDTA VYFCARGGFGSSYWYFDVWGQGTLVTVSSASTKGPSVFPLAPSSKST SGGTAALGCLVKDYFPEPVTVSWNSGALTSGVHTFPAVLQSSGLYSL SSVVTVPSSSLGTQTYICNVNHKPSNTKVDKKVEPKSCDKTHTCPPC PAPELLGGPSVFLFPPKPKDTLMISRTPEVTCVVVDVSHEDPEVKFN WYVDGVEVHNAKTKPREEQYNSTYRVVSVLTVLHQDWLNGKEYK CKVSNKALPAPIEKTISKAKGQPREPQVYTLPPSRDELTKNQVSLTCL VKGFYPSDIAVEWESNGQPENNYKTTPPVLDSDGSFFLYSKLTVDKS RWQQGNVFSCSVMHEALHNHYTQKSLSLSPGK DIQLTQSPSSLSASVGDRVSITCKASQDVSIAVAWYQQKPGKAPKLLI YSASYRYTGVPDRFSGSGSGTDFTLTISSLQPEDFAVYYCQQHYITPL TFGAGTKVEIKRTVAAPSVFIFPPSDEQLKSGTASVVCLLNNFYPREA KVQWKVDNALQSGNSQESVTEQDSKDSTYSLSSTLTLSKADYEKHK VYACEVTHQGLSSPVTKSFNRGEC |
| Light chain | 84 | DIQLTQSPSSLSASVGDRVSITCKASQDVSIAVAWYQQKPGKAPKLLI YSASYRYTGVPDRFSGSGSGTDFTLTISSLQPEDFAVYYCQQHYITPL TFGAGTKVEIKRTVAAPSVFIFPPSDEQLKSGTASVVCLLNNFYPREA KVQWKVDNALQSGNSQESVTEQDSKDSTYSLSSTLTLSKADYEKHK VYACEVTHQGLSSPVTKSFNRGEC |

### Example 19. Affinity assay of anti-CLDN18.2 antibodies and Fc-mutant antibodies thereof for Fc receptors

The affinity of the anti-CLDN18.2 antibodies and the Fc-mutant antibodies thereof for the Fc receptors was determined using the BLI (bio-layer interferometry) technology. The instrument used was Octet Platform (Fortebio, Octet QK, S/N 33-8094-2244, equipment ID TBIO001). The sensor was SA Biosensor (Fortebio, Cat. No. 18-5019). The Fc receptors included FcRn (ACRO Biosystems, Cat# FCM-H8286); FcγRIa (ACRO Biosystems, Cat# FCA-H82E8); FcγRIIa H131 (ACRO Biosystems, Cat# CDA-H82E6); FcγRIIa R131 (ACRO Biosystems, Cat# CDA-H82E7); FcγRIIb (ACRO Biosystems, Cat# CDB-H82E0); FcγRIIIa 158V (ACRO Biosystems, Cat# CDA-H82E9); FcγRIIIa 158F (ACRO Biosystems, Cat# CDA-H82E8); FcγRIIIb (NA2; ACRO Biosystems, Cat# CDB-H82Ea).

The SA sensor was placed in PBS for pre-equilibration for 10 min before capturing the Fc receptor, and then the binding and dissociation signals for the antibody were acquired. After the acquisition, the data were analyzed using data analysis software *Acquisition 8.2* of the instrument to give the affinity constant KD. The results are shown in Table 8.

The results show that the Fc mutation in antibody H239H-2b-K-6a-1 did not affect the binding of the antibody to FcRn. After the N297A mutation, the affinity of antibody H239H-2b-K-6a-2 for FcγRIa was reduced by about 100 folds, and the antibody exhibited no binding capacity to both FcγRII and FcγRIII. After the LALA P329G mutation, antibody H239H-2b-K-6a-3 exhibited no binding capacity to FcγRI, FcγRII, and FcγRIII.

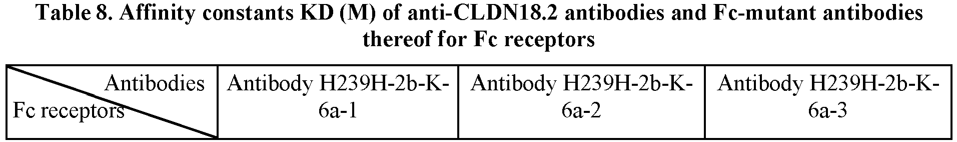

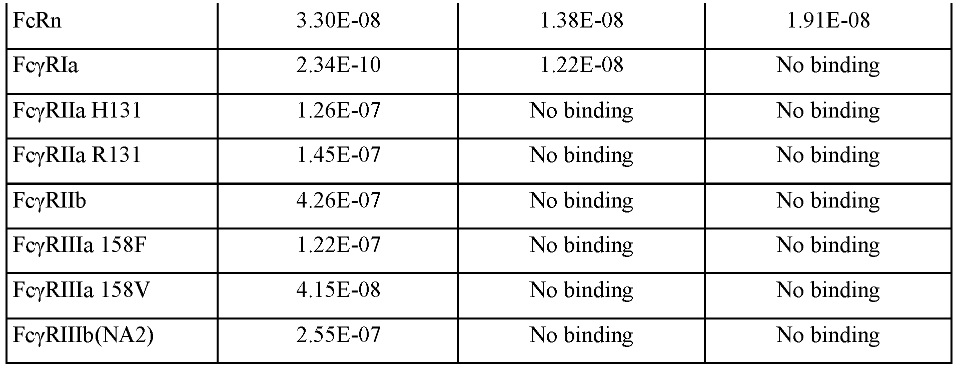

### Example 20. Anti-tumor effect of ADCs in HuPrime^{®} gastric cancer GA0006 xenograft female BALB/c nude mouse model

Different ADCs were tested for the anti-tumor effect in HuPrime^{®} gastric cancer GA0006 xenograft mouse model. A tumor mass with a diameter of 2~3 mm was grafted subcutaneously at the right anterior scapula of BALB/c nude mice. When the average tumor volume of tumor-bearing mice reached about 139.15mm³, the mice were randomly divided into groups. The day of grouping was set as the 0th day, and the administration started on the 0th day, with a single administration. The body weight and tumor growth of the mice were monitored. FIG. 6 showed the tumor growth in each treatment group and the control group of the GA0006 xenograft model. The calculation formula for tumor volume: Tumor volume (mm³) = 1/2 × (a × b²) (where a represents long diameter and b represents short diameter). TGI_{TV}%=[1-(Ti-T0)/(Ci-C0)]×100, where T0 and C0 are the mean tumor volumes of the administration group and vehicle control group at the day of grouping (day 0), respectively, and Ti and Ci are the mean tumor volumes of the administration group and vehicle control group at day 14, respectively. ^{ns}*P>*0.05, **P<0.05,* ***P<*0.01 and ****P* < 0.001 compared to the tumor volume of the vehicle control group.

The control group (group 1) of this model achieved an average tumor volume of 628.62 mm³ after 14 days of the administration.

After 14 days of the administration of 10mg/kg and 5mg/kg ADC2 (i.e. Group 2 and Group 3), the average tumor volume was 78.17mm³ and 91.47mm³, respectively, the relative tumor inhibition rates were TGI 112.45% (P = 3.43e-09***) and 109.75% (P=6.53e-08***), respectively.

After 14 days of the administration of 10mg/kg and 5mg/kg ADC6 (i.e. Group 4 and Group 5), the average tumor volume was 25.77mm³ and 78.07mm³, respectively, the relative tumor inhibition rates were TGI 123.17% (P =7.58e-12***) and 112.48% (P = 1.23e-08***), respectively.

After 14 days of the administration of 10mg/kg and 5mg/kg ADC3 (i.e. Group 6 and Group 7), the average tumor volume was 185.69 mm³ and 284.02 mm³, respectively, the relative tumor inhibition rates were TGI 90.50% (P =5.92e-04***) and 70.40% (P =3.51e-01^{ns}), respectively.

After 14 days of the administration of 5mg/kg ADC5 (i.e. Group 8), the average tumor volume was 296.86 mm³, the relative tumor inhibition rate were TGI 67.79% (P =3.51e-0^{ns}).

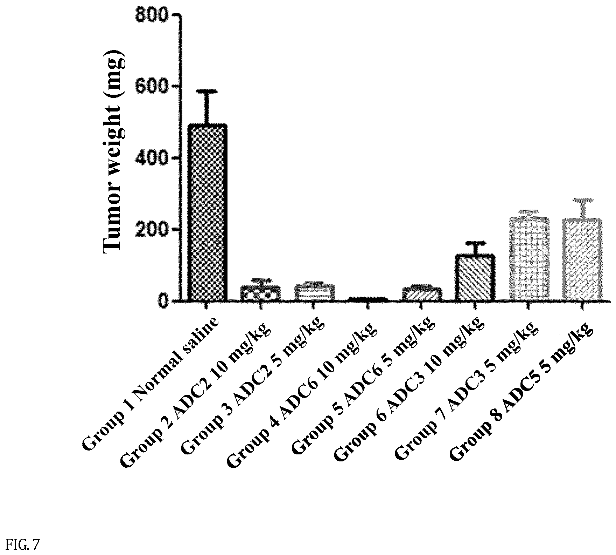

Consistent with the trend of tumor volume data, using changes in the tumor weight as an indicator, after 14 days of the administration of each treatment group also showed varying degrees of anti-tumor efficacy, as shown in FIG. 7.

## Claims

1. An antibody or antigen-binding unit, wherein the antibody or antigen-binding unit specifically binds to CLDN18.2 and comprises one or more amino acid sequences of (a)-(f):
(a) a VH CDR1 comprising the amino acid sequence set forth in any one of SEQ ID NOs: 1-6;
(b) a VH CDR2 comprising the amino acid sequence set forth in any one of SEQ ID NOs: 7-13;
(c) a VH CDR3 comprising the amino acid sequence set forth in any one of SEQ ID NOs: 14-21;
(d) a VL CDR1 comprising the amino acid sequence set forth in any one of SEQ ID NOs: 22-29;
(e) a VL CDR2 comprising the amino acid sequence set forth in any one of SEQ ID NOs: 30-37; and
(f) a VL CDR3 comprising the amino acid sequence set forth in any one of SEQ ID NOs: 38-45.

2. The antibody or antigen-binding unit according to claim 1, comprising a VH CDR1 set forth in any one of SEQ ID NOs: 1-6, a VH CDR2 set forth in any one of SEQ ID NOs: 7-13, and a VH CDR3 set forth in any one of SEQ ID NOs: 14-21.

3. The antibody or antigen-binding unit according to claim 1 or 2, comprising a VL CDR1 set forth in any one of SEQ ID NOs: 22-29, a VL CDR2 set forth in any one of SEQ ID NOs: 30-37, and a VL CDR3 set forth in any one of SEQ ID NOs: 38-45.

4. An antibody or antigen-binding unit, wherein the antibody or antigen-binding unit specifically binds to CLDN18.2 and comprises a VH CDR1 set forth in SEQ ID NO: 2, a VH CDR2 set forth in SEQ ID NO: 8, a VH CDR3 set forth in SEQ ID NO: 15, a VL CDR1 set forth in SEQ ID NO: 26, a VL CDR2 set forth in SEQ ID NO: 34, and a VL CDR3 set forth in SEQ ID NO: 42; or
the antibody or antigen-binding unit comprises a VH CDR1 set forth in SEQ ID NO: 3, a VH CDR2 set forth in SEQ ID NO: 9, a VH CDR3 set forth in SEQ ID NO: 16, a VL CDR1 set forth in SEQ ID NO: 25, a VL CDR2 set forth in SEQ ID NO: 33, and a VL CDR3 set forth in SEQ ID NO: 41; or
the antibody or antigen-binding unit comprises a VH CDR1 set forth in SEQ ID NO: 6, a VH CDR2 set forth in SEQ ID NO: 13, a VH CDR3 set forth in SEQ ID NO: 20, a VL CDR1 set forth in SEQ ID NO: 28, a VL CDR2 set forth in SEQ ID NO: 36, and a VL CDR3 set forth in SEQ ID NO: 44; or
the antibody or antigen-binding unit comprises a VH CDR1 set forth in SEQ ID NO: 5, a VH CDR2 set forth in SEQ ID NO: 12, a VH CDR3 set forth in SEQ ID NO: 19, a VL CDR1 set forth in SEQ ID NO: 29, a VL CDR2 set forth in SEQ ID NO: 37, and a VL CDR3 set forth in SEQ ID NO: 45; or
the antibody or antigen-binding unit comprises a VH CDR1 set forth in SEQ ID NO: 4, a VH CDR2 set forth in SEQ ID NO: 11, a VH CDR3 set forth in SEQ ID NO: 18, a VL CDR1 set forth in SEQ ID NO: 27, a VL CDR2 set forth in SEQ ID NO: 35, and a VL CDR3 set forth in SEQ ID NO: 43; or
the antibody or antigen-binding unit comprises a VH CDR1 set forth in SEQ ID NO: 3, a VH CDR2 set forth in SEQ ID NO: 9, a VH CDR3 set forth in SEQ ID NO: 16, a VL CDR1 set forth in SEQ ID NO: 26, a VL CDR2 set forth in SEQ ID NO: 34, and a VL CDR3 set forth in SEQ ID NO: 42; or
the antibody or antigen-binding unit comprises a VH CDR1 set forth in SEQ ID NO: 3, a VH CDR2 set forth in SEQ ID NO: 9, a VH CDR3 set forth in SEQ ID NO: 16, a VL CDR1 set forth in SEQ ID NO: 24, a VL CDR2 set forth in SEQ ID NO: 32, and a VL CDR3 set forth in SEQ ID NO: 40; or
the antibody or antigen-binding unit comprises a VH CDR1 set forth in SEQ ID NO: 1, a VH CDR2 set forth in SEQ ID NO: 7, a VH CDR3 set forth in SEQ ID NO: 14, a VL CDR1 set forth in SEQ ID NO: 22, a VL CDR2 set forth in SEQ ID NO: 30, and a VL CDR3 set forth in SEQ ID NO: 38; or
the antibody or antigen-binding unit comprises a VH CDR1 set forth in SEQ ID NO: 1, a VH CDR2 set forth in SEQ ID NO: 7, a VH CDR3 set forth in SEQ ID NO: 14, a VL CDR1 set forth in SEQ ID NO: 23, a VL CDR2 set forth in SEQ ID NO: 31, and a VL CDR3 set forth in SEQ ID NO: 39; or
the antibody or antigen-binding unit comprises a VH CDR1 set forth in SEQ ID NO: 2, a VH CDR2 set forth in SEQ ID NO: 10, a VH CDR3 set forth in SEQ ID NO: 17, a VL CDR1 set forth in SEQ ID NO: 27, a VL CDR2 set forth in SEQ ID NO: 35, and a VL CDR3 set forth in SEQ ID NO: 43; or
the antibody or antigen-binding unit comprises a VH CDR1 set forth in SEQ ID NO: 2, a VH CDR2 set forth in SEQ ID NO: 8, a VH CDR3 set forth in SEQ ID NO: 21, a VL CDR1 set forth in SEQ ID NO: 26, a VL CDR2 set forth in SEQ ID NO: 34, and a VL CDR3 set forth in SEQ ID NO: 42.

5. The antibody or antigen-binding unit according to any one of claims 1-4, comprising a heavy chain variable region and/or a light chain variable region, wherein,
the heavy chain variable region comprises the amino acid sequence set forth in any one of SEQ ID NOs: 46-55, or an amino acid sequence having at least 80% or 90% identity to the amino acid sequence set forth in any one of SEQ ID NOs: 46-55, or an amino acid sequence having one or more conservative amino acid substitutions compared with the amino acid sequence set forth in any one of SEQ ID NOs: 46-55; and/or
the light chain variable region comprises the amino acid sequence set forth in any one of SEQ ID NOs: 56-64, or an amino acid sequence having at least 80% or 90% identity to the amino acid sequence set forth in any one of SEQ ID NOs: 56-64, or an amino acid sequence having one or more conservative amino acid substitutions compared with the amino acid sequence set forth in any one of SEQ ID NOs: 56-64.

6. An antibody or antigen-binding unit, wherein the antibody or antigen-binding unit specifically binds to CLDN18.2 and comprises a heavy chain variable region and a light chain variable region;
the heavy chain variable region comprises the amino acid sequence set forth in SEQ ID NO: 47, and the light chain variable region comprises the amino acid sequence set forth in SEQ ID NO: 60; or
the heavy chain variable region comprises the amino acid sequence set forth in SEQ ID NO: 48, and the light chain variable region comprises the amino acid sequence set forth in SEQ ID NO: 59; or
the heavy chain variable region comprises the amino acid sequence set forth in SEQ ID NO: 52, and the light chain variable region comprises the amino acid sequence set forth in SEQ ID NO: 62; or
the heavy chain variable region comprises the amino acid sequence set forth in SEQ ID NO: 51, and the light chain variable region comprises the amino acid sequence set forth in SEQ ID NO: 63; or
the heavy chain variable region comprises the amino acid sequence set forth in SEQ ID NO: 50, and the light chain variable region comprises the amino acid sequence set forth in SEQ ID NO: 61; or
the heavy chain variable region comprises the amino acid sequence set forth in SEQ ID NO: 48, and the light chain variable region comprises the amino acid sequence set forth in SEQ ID NO: 60; or
the heavy chain variable region comprises the amino acid sequence set forth in SEQ ID NO: 48, and the light chain variable region comprises the amino acid sequence set forth in SEQ ID NO: 58; or
the heavy chain variable region comprises the amino acid sequence set forth in SEQ ID NO: 46, and the light chain variable region comprises the amino acid sequence set forth in SEQ ID NO: 56; or
the heavy chain variable region comprises the amino acid sequence set forth in SEQ ID NO: 46, and the light chain variable region comprises the amino acid sequence set forth in SEQ ID NO: 57; or
the heavy chain variable region comprises the amino acid sequence set forth in SEQ ID NO: 49, and the light chain variable region comprises the amino acid sequence set forth in SEQ ID NO: 61; or
the heavy chain variable region comprises the amino acid sequence set forth in SEQ ID NO: 53, and the light chain variable region comprises the amino acid sequence set forth in SEQ ID NO: 64; or
the heavy chain variable region comprises the amino acid sequence set forth in SEQ ID NO: 54, and the light chain variable region comprises the amino acid sequence set forth in SEQ ID NO: 64; or
the heavy chain variable region comprises the amino acid sequence set forth in SEQ ID NO: 55, and the light chain variable region comprises the amino acid sequence set forth in SEQ ID NO: 64.

7. The antibody or antigen-binding unit according to any one of claims 1-6, wherein the antibody or antigen-binding unit is a humanized antibody or antigen-binding unit.

8. The antibody or antigen-binding unit according to any one of claims 1-7, wherein the antibody or antigen-binding unit is of IgG isotype; or the antibody or antigen-binding unit is of IgG1 or IgG4 isotype.

9. The antibody or antigen-binding unit according to any one of claims 1-8, comprising a heavy chain constant region and/or a light chain constant region.

10. The antibody or antigen-binding unit according to claim 9, wherein the heavy chain constant region comprises one or more of the following amino acid mutations: N297A, L234A, L235A, and P329G; or the heavy chain constant region comprises the following amino acid mutations: L234A, L235A, and P329G.

11. The antibody or antigen-binding unit according to any one of claims 1-9, comprising a heavy chain constant region and a light chain constant region, wherein,
the heavy chain constant region comprises the amino acid sequence set forth in any one of SEQ ID NOs: 65-67, or an amino acid sequence having at least 80% or 90% identity to the amino acid sequence set forth in any one of SEQ ID NOs: 65-67, or an amino acid sequence having one or more conservative amino acid substitutions compared with the amino acid sequence set forth in any one of SEQ ID NOs: 65-67; and/or
the light chain constant region comprises the amino acid sequence set forth in SEQ ID NO: 68, or an amino acid sequence having at least 80% or at least 90% identity to the amino acid sequence set forth in SEQ ID NO: 68, or an amino acid sequence having one or more conservative amino acid substitutions compared with the amino acid sequence set forth in SEQ ID NO: 68.

12. The antibody or antigen-binding unit according to any one of claims 1-3, wherein the heavy chain of the antibody comprises the amino acid sequence set forth in any one of SEQ ID NOs: 69-71, or an amino acid sequence having at least 80% or 90% identity to the amino acid sequence set forth in any one of SEQ ID NOs: 69-71, or an amino acid sequence having one or more conservative amino acid substitutions compared with the amino acid sequence set forth in any one of SEQ ID NOs: 69-71; and/or
the light chain of the antibody comprises the amino acid sequence set forth in SEQ ID NO: 72, or an amino acid sequence having at least 80% or 90% identity to the amino acid sequence set forth in SEQ ID NO: 72, or an amino acid sequence having one or more conservative amino acid substitutions compared with the amino acid sequence set forth in SEQ ID NO: 72.

13. An antibody or antigen-binding unit, wherein the heavy chain of the antibody comprises the amino acid sequence set forth in any one of SEQ ID NOs: 69-71, and/or the light chain of the antibody comprises the amino acid sequence set forth in SEQ ID NO: 72.

14. A biomaterial, wherein the biomaterial is
a polynucleotide encoding the antibody or antigen-binding unit according to any one of claims 1-13; or an expression vector comprising the polynucleotide encoding the antibody or antigen-binding unit according to any one of claims 1-13; or
a cell comprising the polynucleotide encoding the antibody or antigen-binding unit according to any one of claims 1-13, wherein optionally, the cell is a CHO cell or a HEK293 cell.

15. An antibody-drug conjugate, comprising the antibody or antigen-binding unit according to any one of claims 1-13 conjugated to a drug via a linker, or a pharmaceutically acceptable salt or solvate thereof, wherein optionally, the linker is a cleavable linker.

16. An antibody-drug conjugate having a structure of Formula I or a stereoisomer thereof, or a pharmaceutically acceptable salt or solvate thereof: 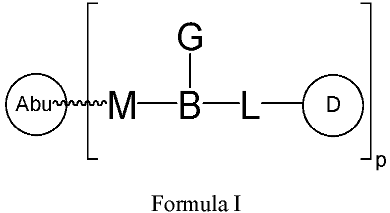 wherein,
Abu is an antibody or antigen-binding unit that binds to CLDN18.2;
D is a drug;
M is 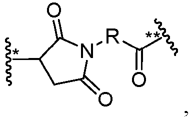
wherein * links to Abu, ** links to B, and R is selected from: -(CH₂)ᵣ-, -(CHR^{m})ᵣ-, C3-C8 carbocyclyl, -O-(CH₂)ᵣ-, arylene, -(CH₂)ᵣ-arylene-, -arylene-(CH₂)ᵣ-, -(CH₂)ᵣ-(C3-C8 carbocyclyl)-, -(C3-C8 carbocyclyl)-(CH₂)ᵣ-, C3-C8 heterocyclyl, -(CH₂)ᵣ-(C3-C8 heterocyclyl)-, -(C3-C8 heterocyclyl)-(CH₂)ᵣ-, - (CH₂)ᵣC(O)NR^{™}(CH₂)ᵣ-, -(CH₂CH₂O)ᵣ-, -(CH₂CH₂O)ᵣ-CH₂-, -(CH₂)ᵣC(O)NR^{™}(CH₂CH₂O)ᵣ-, - (CH₂)ᵣC(O)NR^{m}(CH₂CH₂O)ᵣ-CH₂-, -(CH₂CH₂O)ᵣC(O)NR^{m}(CH₂CH₂O)ᵣ-, -(CH₂CH₂O)ᵣC(O)NR^{™}(CH₂CH₂O)ᵣ-CH₂-, and -(CH₂CH₂O)ᵣC(O)NR^{™}(CH₂)ᵣ-; wherein each R^{m} is independently H, C1-C6 alkyl, C3-C8 carbocyclyl, phenyl, or benzyl, and each r is independently 1, 2, 3, 4, 5, 6, 7, 8, 9, or 10;
B is 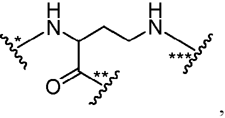 wherein * links to M, ** links to L, and *** links to G;
L is -(AA)ᵢ-(FF)_{f}-, wherein AA is an amino acid or a polypeptide, and i is 1, 2, 3, 4, 5, 6, 7, 8, 9, 10, 11, 12, 13, 14, 15, 16, 17, 18, 19, or 20; each FF is independently 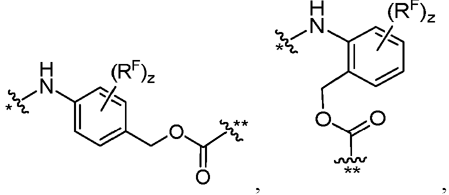 or 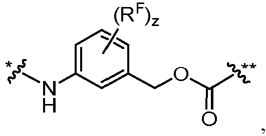 wherein each R^{F} is independently C1-C6 alkyl, C1-C6 alkoxy, -NO₂, or halogen;
z is 0, 1, 2, 3, or 4, wherein * links to AA, and ** links to D; f is 1, 2, 3, 4, 5, 6, 7, 8, 9, or 10;
G is 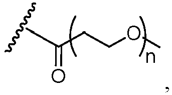 wherein n is 1-24;
p is 1-10.

17. The antibody-drug conjugate according to claim 16, wherein Abu is the antibody or antigen-binding unit according to any one of claims 1-13.

18. The antibody-drug conjugate according to claim 16 or 17, wherein B is 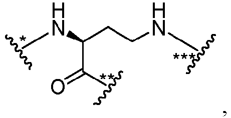 wherein * links to M, ** links to L, and *** links to G.

19. The antibody-drug conjugate according to any one of claims 16-18, wherein each AA is independently selected from the following amino acid or peptide sequences: Val-Cit, Val-Lys, Phe-Lys, Lys-Lys, Ala-Lys, Phe-Cit, Leu-Cit, Ile-Cit, Trp, Cit, Phe-Ala, Phe-Phe-Lys, D-Phe-Phe-Lys, Gly-Phe-Lys, Leu-Ala-Leu, Ile-Ala-Leu, Val-Ala-Val, Ala-Leu-Ala-Leu, β-Ala-Leu-Ala-Leu, and Gly-Phe-Leu-Gly.

20. The antibody-drug conjugate according to claim 19, wherein AA is Val-Cit.

21. The antibody-drug conjugate according to any one of claims 16-20, wherein i is 1.

22. The antibody-drug conjugate according to any one of claims 16-21, wherein FF is independently 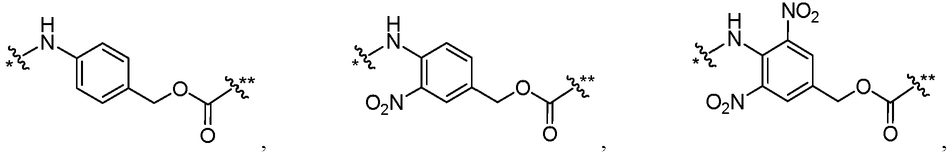 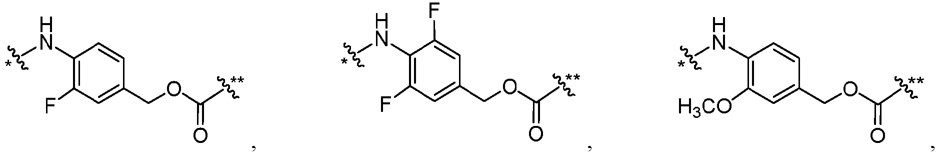 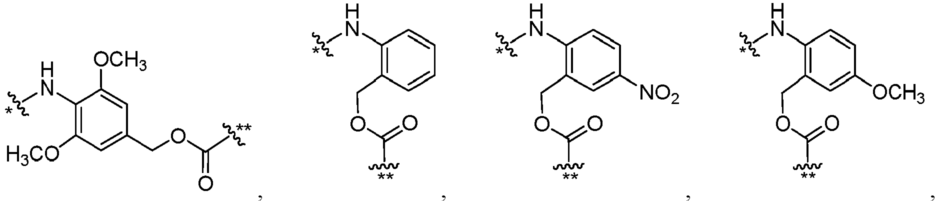 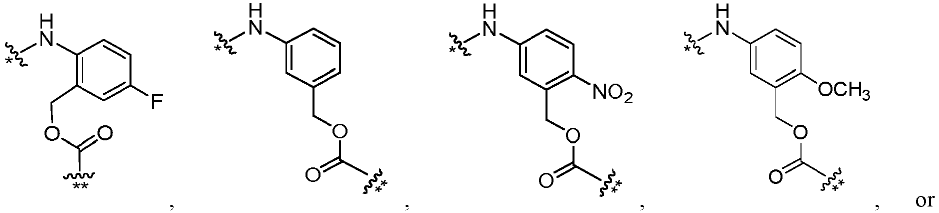 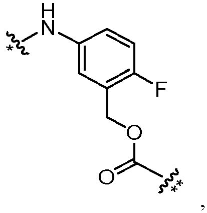 wherein * links to AA, and ** links to D.

23. The antibody-drug conjugate according to claim 22, wherein FF is 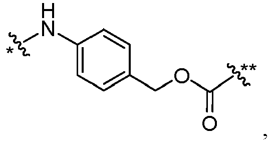 wherein * links to AA, and ** links to D.

24. The antibody-drug conjugate according to any one of claims 16-23, wherein f is 1.

25. The antibody-drug conjugate according to claim 24, wherein L is 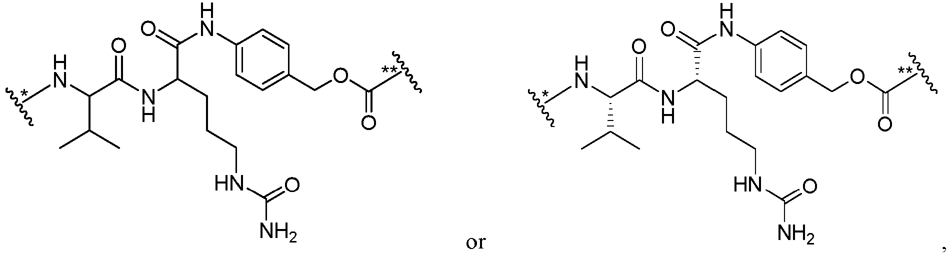 wherein * links to B, and ** links to D.

26. An antibody-drug conjugate having a structure of Formula I-1 or I-2 or a stereoisomer thereof, or a pharmaceutically acceptable salt or solvate thereof, wherein
the Formula I-1 is: 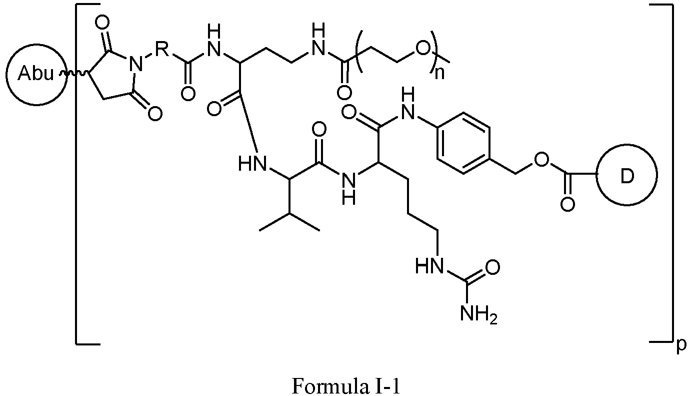
the Formula I-2 is: 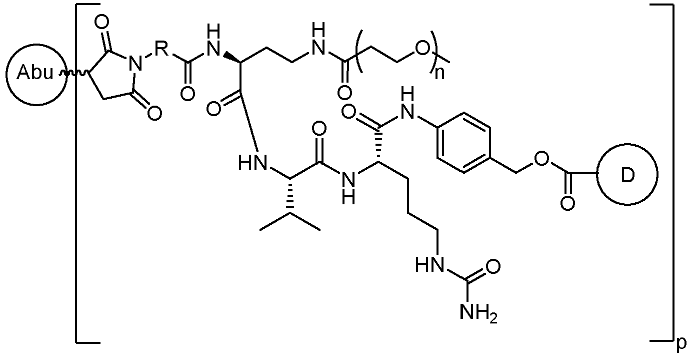 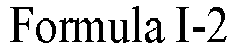 wherein
Abu is the antibody or antigen-binding unit according to any one of claims 1-13;
R is selected from: -(CH₂)ᵣ-, -(CHR^{m})ᵣ-, C3-C8 carbocyclyl, -O-(CH₂)ᵣ-, arylene, -(CH₂)ᵣ-arylene-, -arylene-(CH₂)r-, -(CH₂)ᵣ-(C3-C8 carbocyclyl)-, -(C3-C8 carbocyclyl)-(CH₂)ᵣ-, C3-C8 heterocyclyl, -(CH₂)ᵣ-(C3-C8 heterocyclyl)-, - (C3-C8 heterocyclyl)-(CH₂)ᵣ-, -(CH₂)ᵣC(O)NR^{m}(CH₂)ᵣ-, -(CH₂CH₂O)ᵣ-, -(CH₂CH₂O)ᵣ-CH₂-, - (CH₂)ᵣC(O)NR^{m}(CH₂CH₂O)ᵣ-, -(CH₂)ᵣC(O)NR^{m}(CH₂CH₂O)ᵣ-CH₂-, -(CH₂CH₂O)ᵣC(O)NR^{m}(CH₂CH₂O)ᵣ-, - (CH₂CH₂O)ᵣC(O)NR^{m}(CH₂CH₂O)ᵣ-CH₂-, and -(CH₂CH₂O)ᵣC(O)NR^{m}(CH₂)ᵣ-; wherein each R^{m} is independently H, C1-C6 alkyl, C3-C8 carbocyclyl, phenyl, or benzyl, and each r is independently 1, 2, 3, 4, 5, 6, 7, 8, 9, or 10;
D is a drug;
n is an integer of 1-24;
p is 1-10.

27. The antibody-drug conjugate according to any one of claims 16-26, wherein R is -(CH₂)ᵣ-, and r is 1 or 5.

28. An antibody-drug conjugate having a structure of Formula I-3 or I-4 or a stereoisomer thereof, or a pharmaceutically acceptable salt or solvate thereof, wherein
the Formula I-3 is: 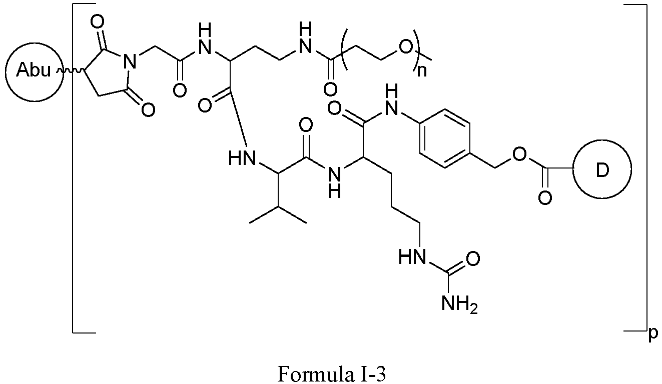
the Formula I-4 is: 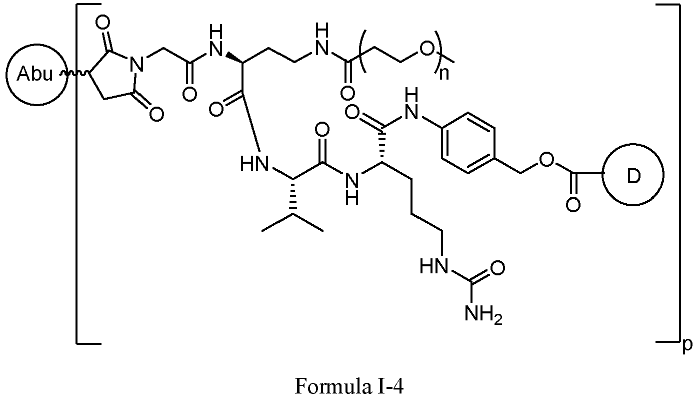 wherein
Abu is the antibody or antigen-binding unit according to any one of claims 1-13;
D is a drug;
n is an integer of 1-24;
p is 1-10.

29. An antibody-drug conjugate having a structure of Formula I-5, I-6, I-7, I-8, I-9, I-10 or I-11 or a stereoisomer thereof, or a pharmaceutically acceptable salt or solvate thereof, wherein
the Formula I-5 is: 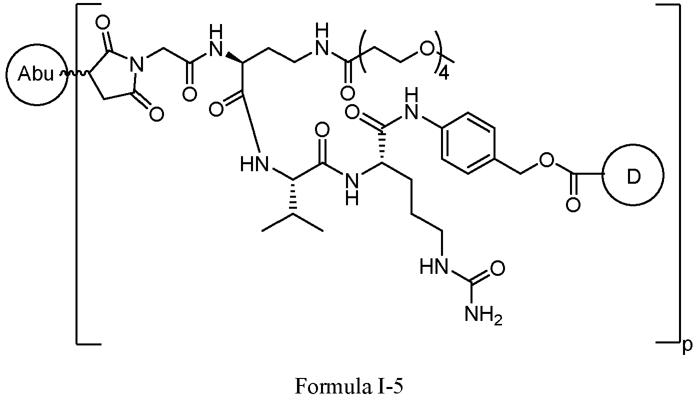
the Formula I-6 is: 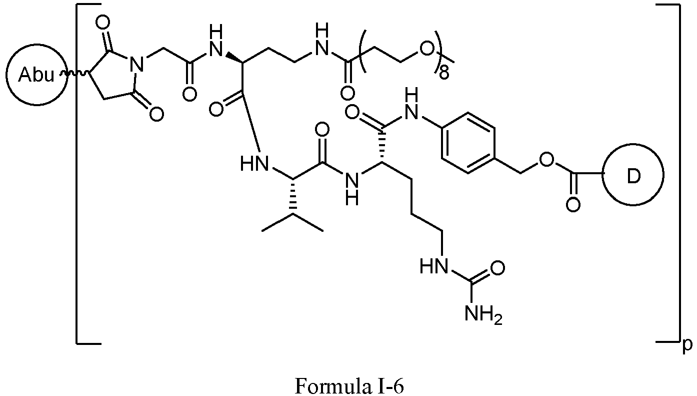
the Formula I-7 is: 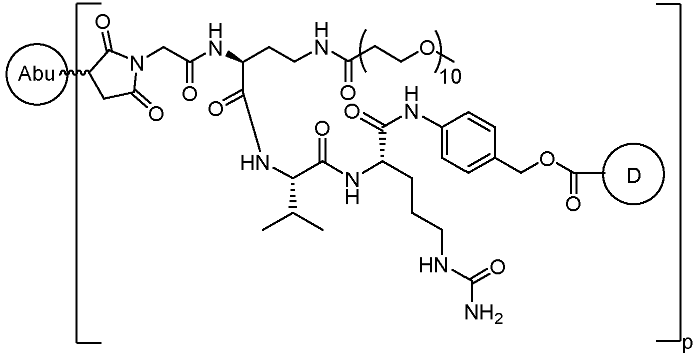 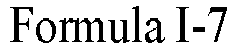
the Formula I-8 is: 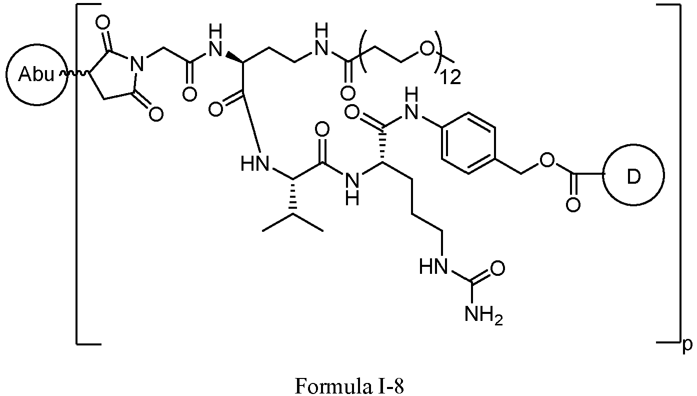
the Formula I-9 is: 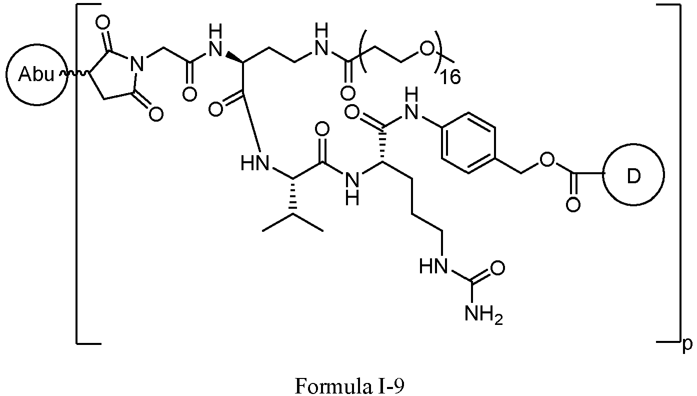
the Formula I-10 is: 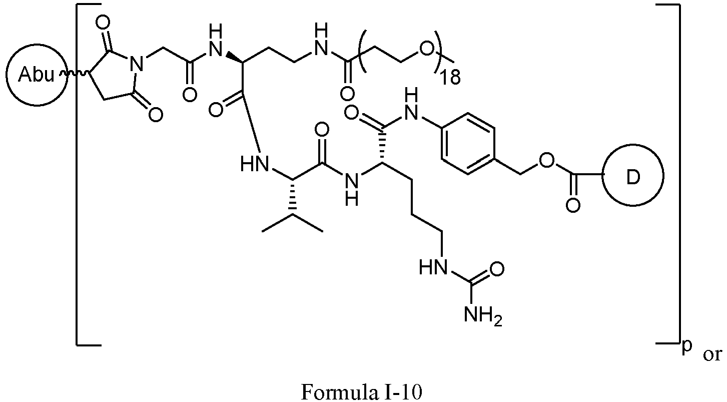
the Formula I-11 is: 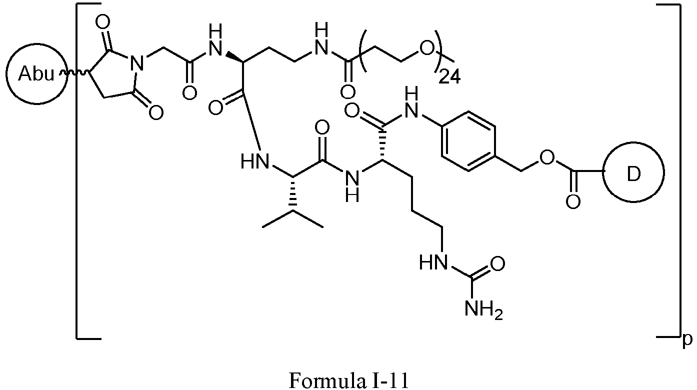 wherein
Abu is the antibody or antigen-binding unit according to any one of claims 1-13;
D is a drug;
p is 1-10.

30. The antibody-drug conjugate according to any one of claims 15-29, wherein the drug is an anti-cancer drug, a cytotoxic drug, a cell differentiation factor, a stem cell trophic factor, a steroid drug, a drug for treating autoimmune diseases, an anti-inflammatory drug or a drug for treating infectious diseases; or the drug is an anti-cancer drug; or the drug is a tubulin inhibitor, a DNA damaging agent or a DNA topoisomerase inhibitor; or the tubulin inhibitor is selected from dolastatin, auristatins and maytansinoids; or the drug is an auristatin selected from MMAE, MMAF or AF; or the drug is a DNA damaging agent selected from calicheamicins, duocarmycins and an anthramycin derivative PBD; or the drug is a DNA topoisomerase inhibitor or a salt thereof selected from irinotecan, irinotecan hydrochloride, an exatecan derivative, camptothecin, 9-aminocamptothecin, 9-nitrocamptothecin, 10-hydroxycamptothecin, 9-chloro-10-hydroxycamptothecin, a camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3H)-quinazolinone, 2-cyano-3-(3,4-dihydroxyphenyl)-N-(phenylmethyl)-(2E)-2-propenamide, 2-cyano-3-(3,4-dihydroxyphenyl)-N-(3-hydroxyphenylpropyl)-(E)-2-propenamide, 12-β-D-glucopyranosyl-12,13-dihydro-2,10-dihydroxy-6-[[2-hydroxy-1-(hydroxymethyl)ethyl]amino]-5H-indolo[2,3-a]pyrrolo[3,4-c]carbazole-5,7(6H)-dione, N-[2-(dimethylamino)ethyl]-4-acridinecarboxamide dihydrochloride, and N-[2-(dimethylamino)ethyl]-4-acridinecarboxamide; or the DNA topoisomerase inhibitor is camptothecin, 10-hydroxycamptothecin, topotecan, belotecan, irinotecan, 22-hydroxyacuminatine, or exatecan; or the pharmaceutically acceptable salt or solvate thereof.

31. The antibody-drug conjugate according to any one of claims 15-29, wherein the drug is 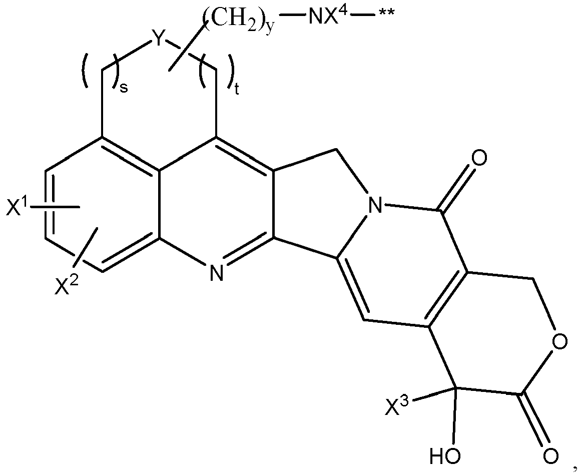 wherein
X¹ and X² are each independently:
H,
hydroxy,
C1-C6 alkyl,
C1-C6 alkyl substituted with one or more hydroxy, halogen, nitro, or cyano groups,
C2-C6 alkenyl,
C2-C6 alkynyl,
C1-C6 alkoxy,
C1-C6 aminoalkoxy,
halogen,
nitro,
cyano,
sulfhydryl,
alkylthio,
amino, amino substituted with an amino-protecting group, C1-C6 aminoalkyl optionally substituted at the amino moiety with an amino-protecting group or C1-C6 alkyl,
C1-C6 aminoalkylamino optionally substituted at the amino moiety with an amino-protecting group or C1-C6 alkyl, C1-C6 alkyl linking to a heterocyclyl, wherein the heterocyclyl is optionally substituted with one or more C1-C6 alkyl, C1-C6 alkoxy, amino, halogen, nitro, or cyano groups,
C1-C6 alkylamino linking to a heterocyclyl, wherein the heterocyclyl is optionally substituted with C1-C6 alkyl or C1-C6 alkoxy, and the amino is optionally substituted with an amino-protecting group, halogen, nitro, cyano, or a protecting group,
amino-substituted heterocyclyl optionally substituted at a nitrogen atom of the heterocyclyl moiety or at the amino moiety with a protecting group or one or more C1-C6 alkyl groups,
heterocyclylamino optionally substituted at a nitrogen atom of the heterocyclyl moiety or at the amino moiety with a protecting group or C1-C6 alkyl,
carbamoyl optionally substituted with a carbamoyl-protecting group or C1-C6 alkyl,
morpholin-1-yl, or
piperidin-1-yl;
X³ is C1-C6 alkyl;
X⁴ is H, -(CH₂)_{q}-CH₃, -(CHRⁿ)_{q}-CH₃, C3-C8 carbocyclyl, -O-(CH₂)_{q}-CH₃, arylene-CH₃, -(CH₂)_{q}-arylene-CH₃, - arylene-(CH₂)_{q}-CH₃, -(CH₂)_{q}-(C3-C8 carbocyclyl)-CH₃, -(C3-C8 carbocyclyl)-(CH₂)_{q}-CH₃, C3-C8 heterocyclyl, - (CH₂)_{q}-(C3-C8 heterocyclyl)-CH₃, -(C3-C8 heterocyclyl)-(CH₂)_{q}-CH₃, -(CH₂)_{q}C(O)NRⁿ(CH₂)_{q}-CH₃, - (CH₂CH₂O)_{q}-CH₃, -(CH₂CH₂O)_{q}-CH₂-CH₃, -(CH₂)_{q}C(O)NRⁿ(CH₂CH₂O)_{q}-CH₃, -(CH₂)_{q}C(O)NRⁿ(CH₂CH₂O)_{q}-CH₂-CH₃, -(CH₂CH₂O)_{q}C(O)NRⁿ(CH₂CH₂O)_{q}-CH₃, -(CH₂CH₂O)_{q}C(O)NRⁿ(CH₂CH₂O)_{q}-CH₂-CH₃, or - (CH₂CH₂O)_{q}C(O)NR⁷(CH₂)_{q}-CH₃, wherein each Rⁿ is independently H, C1-C6 alkyl, C3-C8 carbocyclyl, phenyl, or benzyl, and each q is independently 1, 2, 3, 4, 5, 6, 7, 8, 9, or 10; or X⁴ is H or C1-C6 alkyl;
** links to other moieties of the antibody-drug conjugate;
y is 0, 1, or 2;
Y is O, S, or CR¹R², wherein R¹ and R² are each independently H or C1-C6 alkyl;
s and t are each independently 0, 1, or 2, but not both 0;
or the drug is 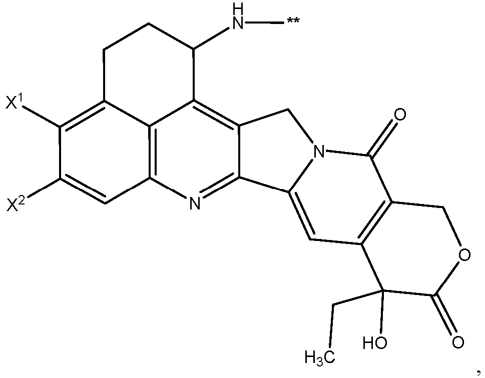
wherein X¹ and X² are each independently C1-C6 alkyl, halogen, or -OH; or the C1-C6 alkyl is -CH₃; or the halogen is F; ** links to other moieties of the antibody-drug conjugate;
or the drug is 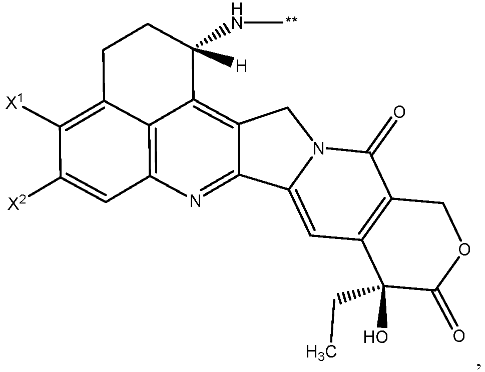
wherein X¹ and X² are each independently C1-C6 alkyl, halogen, or -OH; or the C1-C6 alkyl is -CH₃; or the halogen is F; ** links to other moieties of the antibody-drug conjugate.

32. The antibody-drug conjugate according to any one of claims 16-28, wherein n is 4-12; or n is 4-8; or n is 4 or 8.

33. An antibody-drug conjugate having a structure of Formula I-12, I-13, I-14, I-15, I-16, I-17, I-18, I-19, I-20, I-21, I-22, I-23, I-24 or I-25 or a stereoisomer thereof, or a pharmaceutically acceptable salt or solvate thereof, wherein the Formula I-12, I-13, I-14, I-15, I-16, I-17, I-18, I-19, I-20, I-21, I-22, I-23, I-24, or I-25 is: 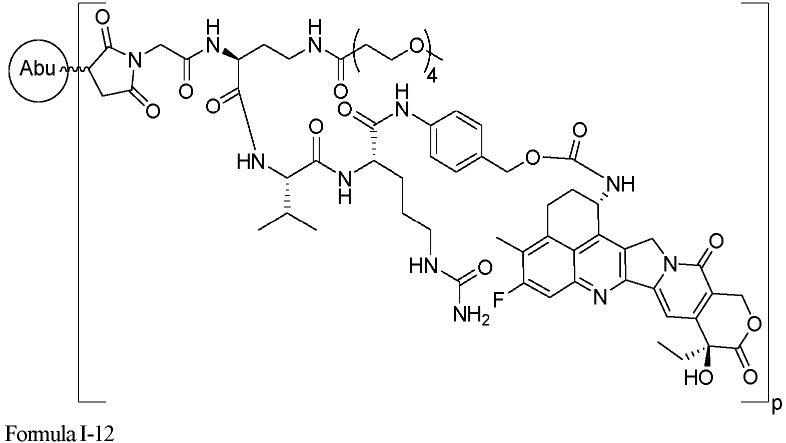 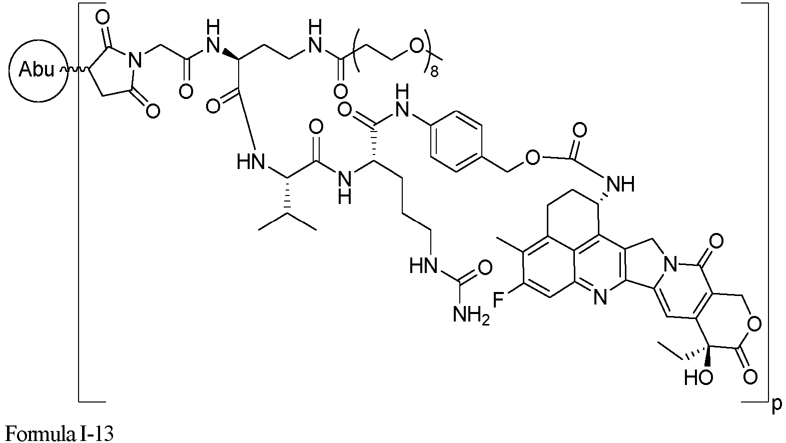 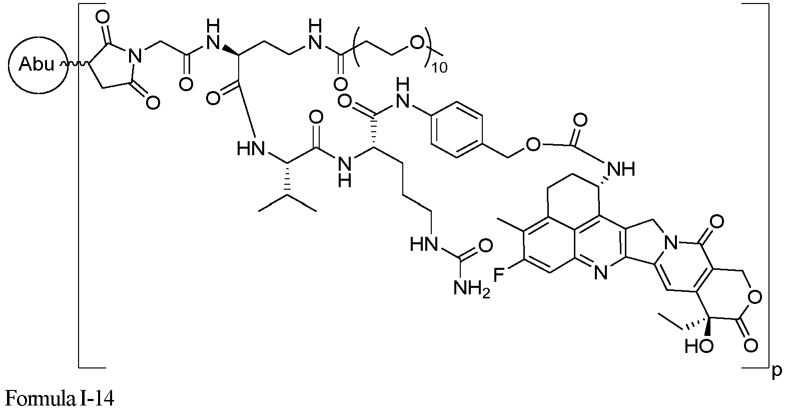 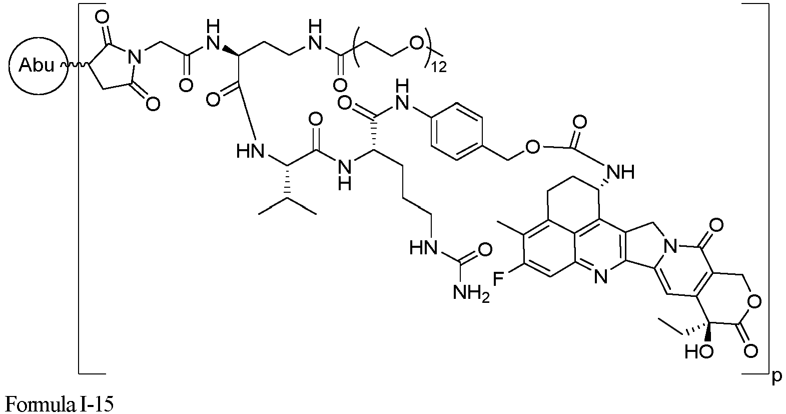 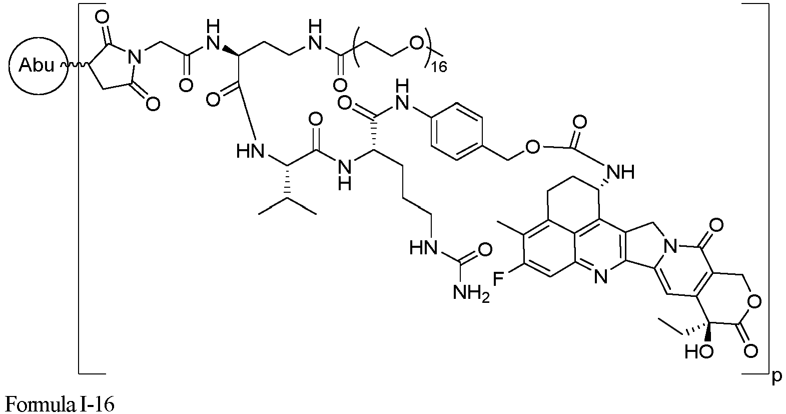 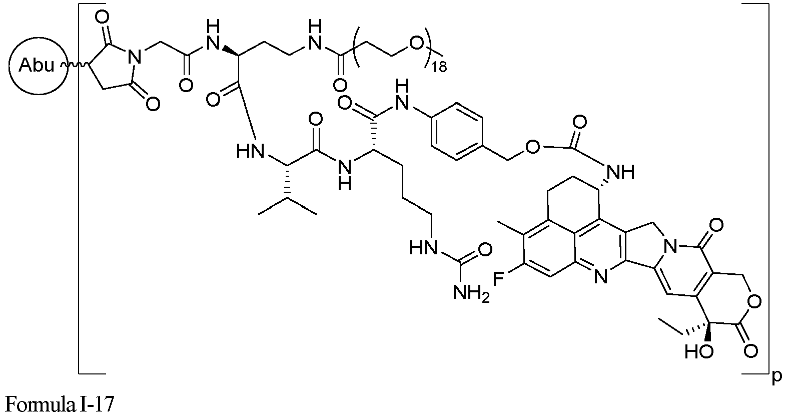 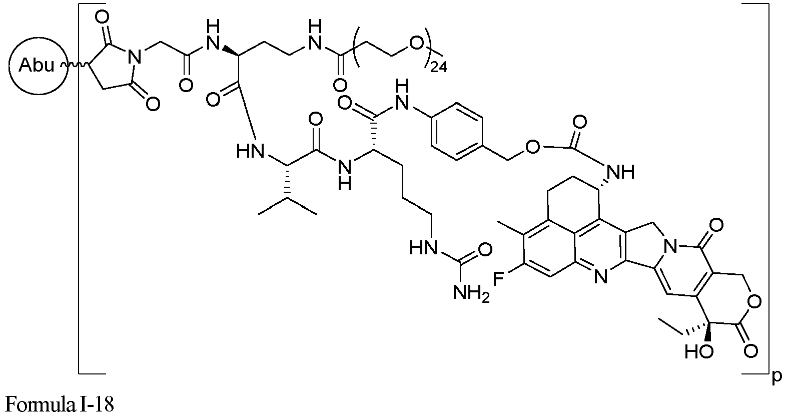 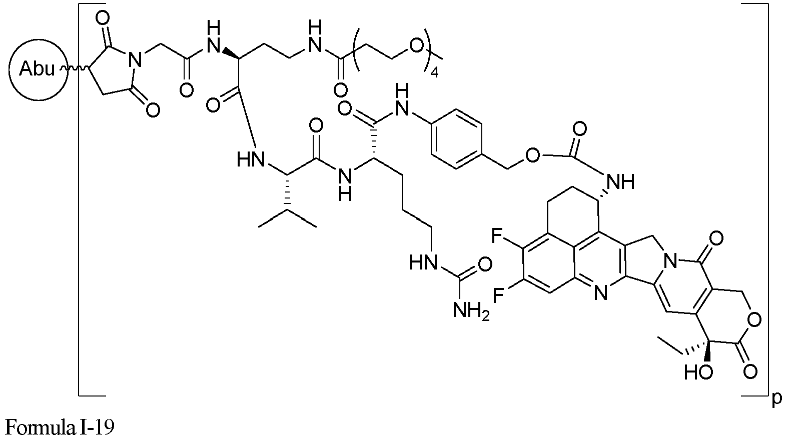 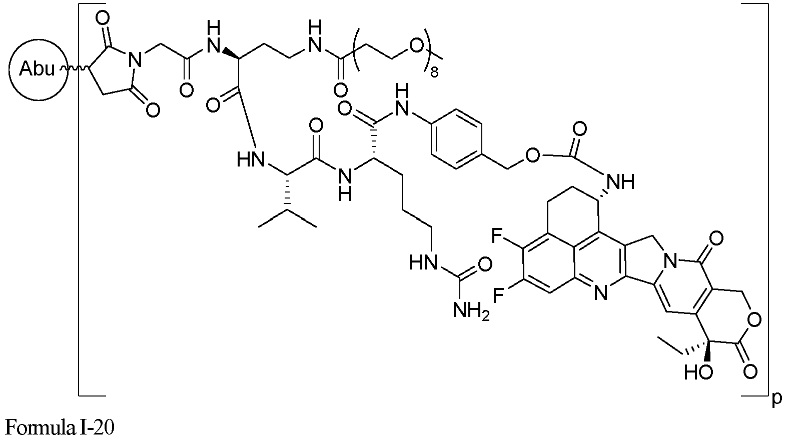 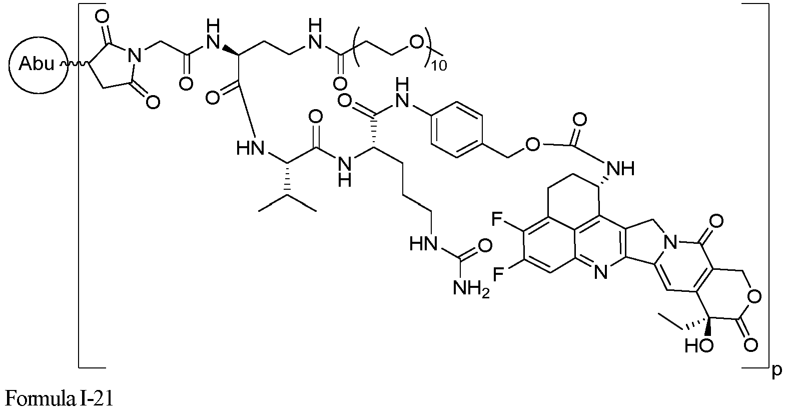 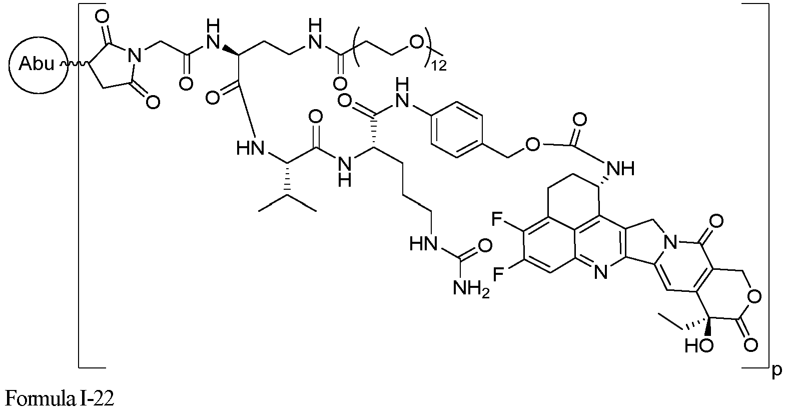 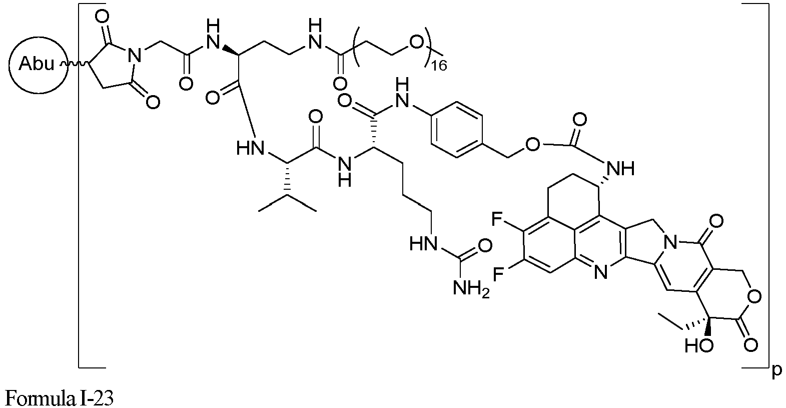 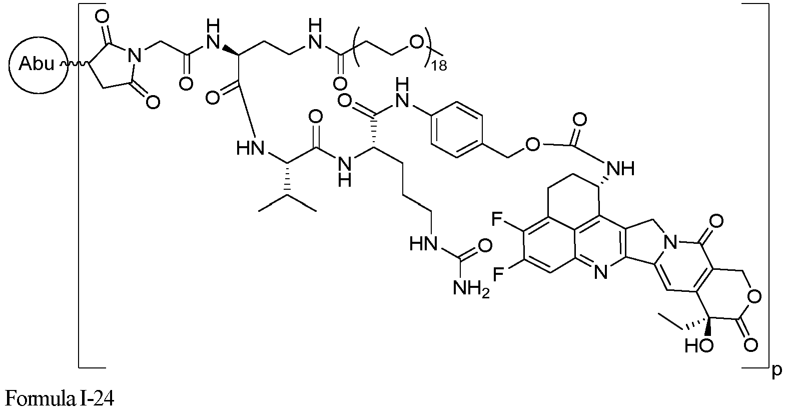 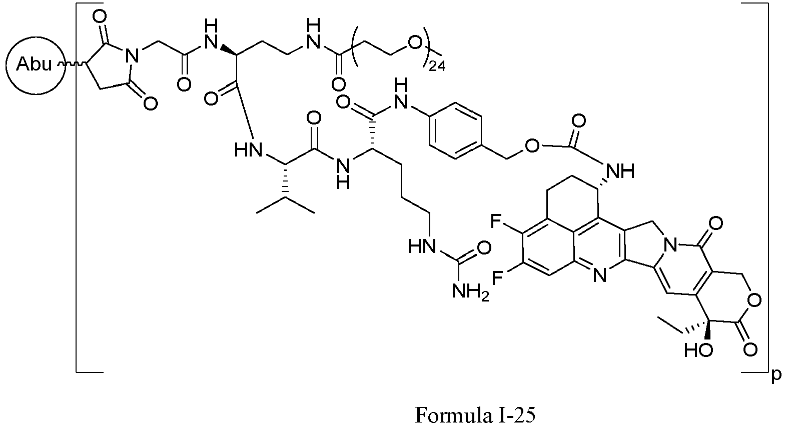 wherein
Abu is the antibody or antigen-binding unit according to any one of claims 1-13;
p is 1-10.

34. The antibody-drug conjugate according to any one of claims 16-33, wherein p is 2-8; or p is 4-8; or p is 6-8; or p is 7-8.

35. A pharmaceutical composition, comprising the antibody or antigen-binding unit according to any one of claims 1-13 or the antibody-drug conjugate according to any one of claims 15-34, and a pharmaceutically acceptable carrier, excipient and/or adjuvant material.

36. Use of the antibody or antigen-binding unit according to any one of claims 1-13, the antibody-drug conjugate according to any one of claims 15-34, or the pharmaceutical composition according to claim 35 in treating and/or preventing a disease or in preparing a medicament for treating and/or preventing the disease, wherein optionally, the disease is a disease associated with the expression of CLDN18.2; or the disease is a disease associated with the overexpression of CLDN18.2; or the disease is a cancer or tumor; or the cancer or tumor is a cancer or tumor expressing CLDN18.2; or the cancer or tumor is selected from bladder cancer, ovarian cancer, lung cancer, adenocarcinoma, gastric cancer, breast cancer, liver cancer, pancreatic cancer, skin cancer, malignant melanoma, head and neck cancer, sarcoma, bile duct cancer, renal cancer, colon cancer, small intestine cancer, testicular embryonal carcinoma, placental choriocarcinoma, cervical cancer, testicular cancer, uterine cancer, esophageal cancer, and gallbladder cancer cells.
